# EUROPÄISCHE PATENTSCHRIFT

(11) **EP 1 523 561 B1**
(45) Veröffentlichungstag und Bekanntmachung des Hinweises auf die Patenterteilung: **01.03.2006**
(21) Anmeldenummer: 03765074.4
(22) Anmeldetag: 21.07.2003
(51) Int. Cl.: C12N 15/82, A01H 5/00

(54) **VERFAHREN ZUR HERSTELLUNG VON TRANSGENEN PFLANZEN MIT ERHÖHTER VIRUSRESISTENZ DURCH "SILENCING" PFLANZLICHER DNAJ-ÄHNLICHER PROTEINE**
METHOD FOR THE PRODUCTION OF TRANSGENIC PLANTS WITH INCREASED VIRUS RESISTANCE BY SILENCING VEGETABLE DNAJ-LIKE PROTEINS
PROCEDE POUR PRODUIRE DES PLANTES TRANSGENIQUES PRESENTANT UNE RESISTANCE ACCRUE AUX VIRUS PAR EXTINCTION DE PROTEINES VEGETALES DU TYPE DNAJ

(30) Priorität: 19.07.2002 DE 10232978
(43) Veröffentlichungstag der Anmeldung: 20.04.2005
(73) Patentinhaber: IPK Institut für Pflanzengenetik und Kulturpflanzenforschung, 06466 Gatersleben (DE)
(72) Erfinder: HOFIUS, Daniel, 06484 Quedlinburg (DE); BÖRNKE, Frederik, 06484 Quedlinburg (DE); SONNEWALD, Uwe, 06484 Quedlinburg (DE)
(74) Vertreter: Neuefeind, Regina
(86) Internationale Anmeldenummer: PCT/EP2003/007945
(87) Internationale Veröffentlichungsnummer: WO 2004/009821

(56) Entgegenhaltungen:
- VON BARGEN SUSANNE ET AL: "Interactions between the tomato spotted wilt virus movement protein and plant proteins showing homologies to myosin, kinesin and DnaJ-like chaperones" PLANT PHYSIOLOGY AND BIOCHEMISTRY (PARIS), Bd. 39, Nr. 12, Dezember 2001 (2001-12), Seiten 1083-1093, XP002257960 ISSN: 0981-9428
- SOELLICK T -R ET AL: "The movement protein NSm of tomato spotted wilt tospovirus (TSWV): RNA binding, interaction with the TSWV N protein, and identification of interacting plant proteins" PROCEEDINGS OF THE NATIONAL ACADEMY OF SCIENCES OF THE UNITED STATES, Bd. 97, Nr. 5, 29. Februar 2000 (2000-02-29), Seiten 2373-2378, XP002257961 Feb. 29, 2000 ISSN: 0027-8424
- MALPICA C A ET AL: "ENGINEERING RESISTANCE AGAINST VIRAL DISEASES IN PLANTS" SUBCELLULAR BIOCHEMISTRY, XX, XX, Bd. 29, 1998, Seiten 287-320, XP001013173
- BEFFA ROLAND S ET AL: "Decreased susceptibility to viral disease of beta-1,3-glucanase-deficient plants generated by antisense transformation" PLANT CELL, Bd. 8, Nr. 6, 1996, Seiten 1001-1011, XP002257962 ISSN: 1040-4651
- IGLESIAS VICTOR ALEJANDRO ET AL: "Movement of plant viruses is delayed in a beta-1,3-glucanase-deficient mutant showing a reduced plasmodesmatal size exclusion limit and enhanced callose deposition" PLANT JOURNAL, Bd. 21, Nr. 2, Januar 2000 (2000-01), Seiten 157-166, XP002257963 ISSN: 0960-7412
- MIERNYK JAN A: "The J-domain proteins of Arabidopsis thaliana: An unexpectedly large and diverse family of chaperones" CELL STRESS AND CHAPERONES, Bd. 6, Nr. 3, Juli 2001 (2001-07), Seiten 209-218, XP009018876 ISSN: 1355-8145
- TOMITA YURIKO ET AL: "Mutation of host DnaJ homolog inhibits brome mosaic virus negative-strand RNA synthesis." JOURNAL OF VIROLOGY, Bd. 77, Nr. 5, März 2003 (2003-03), Seiten 2990-2997, XP002257964 ISSN: 0022-538X (ISSN print)

## Beschreibung

Gegenstand der Erfindung sind generell Pflanzen und Pflanzenzellen, die aufgrund einer Modulation der Genexpression und/oder des Bindungsverhalten von pflanzlichen DnaJ-ähnlichen Proteinen eine transiente oder permanente Virusresistenz aufweisen.

Die vorliegende Erfindung betrifft ebenfalls Verfahren zur Herstellung solcher Pflanzen.

Die Erfindung betrifft somit auch ein Verfahren zur Herstellung von Pflanzen und Pflanzenzellen mit erhöhter Virusresistenz, das dadurch gekennzeichnet ist, dass die Interaktion von viralen Komponenten mit pflanzlichen DnaJ-ähnlichen Protein im Wesentlichen unterbunden wird, indem die Expression der pflanzlichen DnaJ-ähnlichen Proteine reduziert wird. Darüber hinaus betrifft die Erfindung ein Verfahren zum Herstellen von Pflanzen und Pflanzenzellen mit erhöhter Virusresistenz, das dadurch gekennzeichnet ist, dass die Interaktion von viralen Komponenten mit pflanzlichen DnaJ-ähnlichen Proteinen durch dominant-negative Mutanten der DnaJ-ähnlichen Proteine, durch Antikörper gegen DnaJ-ähnliche, Proteine oder durch spezifische Inhibitoren im Wesentlichen blockiert wird.

Weiterhin betrifft die vorliegende Erfindung ein Verfahren zur Herstellung von transgenen Pflanzen und Pflanzenzellen mit erhöhter Virusresistenz, das dadurch gekennzeichnet ist, dass die Interaktion von viralen Komponenten mit pflanzlichen DnaJ-ähnlichen Protein im Wesentlichen unterbunden wird, indem die pflanzlichen DnaJ-ähnlichen Proteine gesilenct werden. Darüber hinaus betrifft die Erfindung ein Verfahren zum Herstellen von transgenen Pflanzen und Pflanzenzellen mit erhöhter Virusresistenz, das dadurch gekennzeichnet ist, dass die Interaktion von viralen Komponenten mit pflanzlichen DnaJ-ähnlichen Proteinen durch dominant-negative Mutanten der DnaJ-ähnlichen Proteine, durch Antikörper gegen DnaJ-ähnliche Proteine oder durch spezifische Inhibitoren im Wesentlichen blockiert wird.

Es gibt eine Vielzahl von pflanzlichen Viren, die wichtige Nutzpflanzen befallen und zu signifikanten jährlichen Ernteausfällen führen. Entsprechend gibt es seit jeher einen großen Bedarf an Verfahren, mit denen es möglich ist, Pflanzen herzustellen, die eine erhöhte Virusresistenz aufweisen.

Zur Herstellung von transgenen Pflanzen mit einer erhöhten Virusresistenz können unterschiedliche Strategien gewählt werden, die sich in der Regel nach der Art des Virus und damit nach dem vom Virus benutzten Infektionsmechanismus richten.

Die am weitesten verbreitete Strategie stellt die so genannte "Pathogen-vermittelte Resistenz"-Strategie dar (zusammengefasst in Baulcombe 1996, Plant Cell, 8:1833-1844). Bei dieser Strategie werden Nukleinsäuresequenzen, die entweder viralen Sequenzen entsprechen oder zu diesen komplementär sind, auf Pflanzenzellen übertragen. Je nachdem, ob diese übertragenen Nukleinsäuresequenzen transkribiert und translatiert oder nur transkribiert werden, unterscheidet man zwischen der Protein-basierten oder RNA-basierten Pathogen-vermittelten Resistenz.

Bei der Protein-basierten Pathogen-vermittelten Resistenz werden in der Regel virale Capsid-Proteine oder so genannte Movement-Proteine, die für den Transport des Virus zwischen und innerhalb der Pflanzenzellen und damit für die systemische Infektion der Pflanzen verantwortlich sind, in den transgenen Pflanzenzellen exprimiert. Die beobachtete Virusresistenz beruht vermutlich darauf, dass durch die Expression der viralen Komponenten im Falle von viralen Capsid-Proteinen die Assemblierung von funktionellen Viruspartikeln und im Falle der viralen Movement-Proteine die Propagation des Virus innerhalb der Pflanze auskompetitiert werden.

Die RNA-basierte Pathogen-vermittelte Resistenz beruht auf der Transkription von viralen Sequenzen. Diese viralen Sequenzen können für virale Produkte kodieren, müssen es aber nicht. Die Grundlage für die RNA-basierte Pathogen-vermittelte Resistenz ist in einem Prozess zu sehen, der als "post-transcriptional gene silencing" (PTGS) bezeichnet wird und auf dem Auftreten von doppelsträngigen RNA-Molekülen in der Pflanzenzelle beruht. Die Möglichkeiten zur Etablierung einer RNA-basierten Pathogen-vermittelten Resistenz sind entsprechend vielfältig. Zum Beispiel können virale Sequenzen verwendet werden, die sich in einer Sense- oder Antisense-Orientierung befinden. Bei der Transkription von viralen Sequenzen mit Antisense-Orientierung treten doppelsträngige RNA-Moleküle bei viralen Infektionen dann zwangsläufig auf. Aber auch bei der Transkription von viralen Sequenzen in Sense-Orientierung, die innerhalb eines Transkripts über ausgeprägte Komplementarität verfügen, können doppelsträngige RNA-Bereiche auftreten. Darüber hinaus treten doppelsträngige RNA-Moleküle bei vielen viralen Infektionen zwangsläufig auf, da viele Pflanzenviren, wie z. B. Potyviren ein RNA-Genom mit (+) -Polarität aufweisen und über ein doppelsträngiges RNA-Intermediat repliziert werden.

Das Auftreten von doppelsträngigen RNA-Molekülen innerhalb einer Pflanzenzelle induziert das PTGS-System, was die spezifische Degradation der entsprechenden viralen Sequenzen bewirkt. Dieser auch als "Silencing" bezeichnete und aus *C.elegans* als "RNA interference" (RNAi) bekannte Mechanismus tritt natürlicherweise bei Infektionen von Pflanzen mit Pflanzenviren auf. Er wird aber häufig nicht stark genug induziert, um die Virusinfektion zurückzudrängen. Transgene Pflanzen, die über eine RNA mit Homologie z. B. zu einem viralen Protein verfügen, transkribieren diese RNA in der Regel in ausreichender Weise, um das PTGS-System und damit eine Virusresistenz zu induzieren. Das PTGS-System von Pflanzen kann deswegen auch gezielt durch Einbringen von viralen doppelsträngigen RNA-Molekülen induziert werden. Auf diese Weise können stabile transgene Pflanzen mit erwünschter Virusresistenz hergestellt werden (Waterhouse et al., 2001, Nature, 411, 834-842).

Die Verwendung von Pathogen-vermittelten Resistenzmechanismen zur Herstellung von transgenen Pflanzen mit einer erhöhten Virusresistenz hat den Nachteil, dass aufgrund der notwendigen Komplementarität der RNA des Transgens zu der viralen RNA, die Pflanzen jeweils nur resistent gegenüber einem ganz spezifischen Virus sind. Sie verfügen damit nur über ein eingeschränktes Resistenzspektrum. Zusätzlich haben Viren Mechanismen entwickelt, durch die das von viralen Sequenzen induzierte PTGS-System unter Umständen reprimiert werden kann. Bei Potyviren ist dazu z. B. die "helper component-proteinase" (Hc-Pro) in der Lage (Kaschau et al., 1998, Cell: 461-470).

Andere Strategien zur Herstellung von Pflanzen mit erhöhter Virusresistenz beruhen darauf, dass Proteasen, die Viren im Laufe ihres Replikationszyklus benötigen, selektiv gehemmt werden. So konnte durch Überexpression eines Cystein-Proteinase-Inhibitors aus Reis die Aktivität von Cystein-Proteinasen inhibiert werden und Pflanzen mit einer erhöhten Resistenz gegen potyvirale Infektionen hergestellt werden (Gutierrez-Campos et al., 1999, Nat. Biotechn. 17: 1223-1226).

Andere Strategien zur Herstellung von transgenen Pflanzen mit erhöhter Virusresistenz beruhen auf der Übertragung von generell antiviral wirkenden Systemen auf Pflanzenzellen. Dazu zählt die Expression von Proteinen, die verschiedene Komponenten des viralen Replikationszyklus angreifen. Ein Beispiel hierfür sind transgene Pflanzen, die das so genannte "pokeweed antiviral protein" (PAP), ein Ribosomeninaktivierendes Protein, exprimieren und aus nicht verstandenen Gründen Resistenz gegenüber einem breiten Virusspektrum zeigen. Allerdings zeigen transgene Pflanzen, die das PAP-Protein exprimieren, ein vom Expressionslevel abhängiges signifikant reduziertes Wachstum (Lodge et al., 1993, PNAS, 90: 7089-7093, Tumer et al., 1997, PNAS, 94: 3866-3871).

Ein weiteres Beispiel für Pflanzen, die über ein generell antiviral wirkendes System verfügen, sind Pflanzen, in die das Interferon-induzierbare antivirale 2,5-Oligoadenylat-System aus tierischen Organismen überführt wurde. Die Pflanzen exprimieren konstitutiv eine 2,5-Oligoadenylat-Synthetase sowie eine 2,5-Oligoadenylat-abhängige Ribonuklease L, die durch doppelsträngige RNA aktivierbar ist (Mitra et al., 1996, PNAS, 93: 6780-6785). Darüber hinaus gibt es weitere Ansätze zur Erhöhung der pflanzlichen Virusresistenz, die andere mechanistische, häufig im Detail noch nicht verstandene Grundlagen haben. Eine Möglichkeit zur Erhöhung der pflanzlichen Virusresistenz ist die Induktion unspezifischer pflanzlicher Abwehrsysteme, wie sie bei der so genannten "systemic acquired resistence" z. B. durch die konstitutive Überexpression von Salicylsäure erreicht wird (Verberne et al., 2000, Nat. Biotechn., 18: 779-783).

Zudem ist zur Herstellung von Pflanzen mit erhöhter Virusresistenz die Übertragung von spezifischen Virus-Resistenzgenen auf die jeweiligen Pflanzen geeignet (Bendahmane et al., 1999, Plant Cell, 11: 781-791). Derartige transgene Pflanzen haben allerding den Nachteil, dass sie nur gegen ein geringes Spektrum von verschiedenen Virengruppen oder-stämmen resistent sind.

Aufgrund der großen Bedeutung, die virusresistente Pflanzen für die Landwirtschaft haben, besteht generell ein großer Bedarf an weiteren Methoden, die die Herstellung von transgenen Pflanzen mit erhöhter Virusresistenz erlauben. Insbesondere besteht ein Bedarf an Methoden, die es erlauben, transgene Pflanzen mit einer erhöhten Virusresistenz herzustellen, die gegenüber einem breiten Spektrum an Virengruppen und -stämmen resistent sind.

Aufgabe der vorliegenden Erfindung ist es, ein zu den aus dem Stand der Technik bekannten Methoden alternatives Verfahren zur Verfügung zu stellen, mit dem es möglich ist, Pflanzen mit einer erhöhten Virusresistenz herzustellen. Insbesondere ist es eine Aufgabe der vorliegenden Erfindung, ein Verfahren zur Verfügung zu stellen, mit dem Pflanzen hergestellt werden können, die eine Resistenz gegenüber einem breiten Spektrum an Virengruppen und -stämmen aufweisen. Der Erfindung liegt ebenfalls die Aufgabe zu Grunde, ein Verfahren zur Herstellung von Pflanzen mit erhöhter Virusresistenz zur Verfügung zu stellen, das generell anwendbar und einfach durchführbar ist.

Zur Lösung dieser und weiterer Aufgaben, wie sie sich aus der Beschreibung ergeben, dienen die Merkmale der unabhängigen Ansprüche.

Bevorzugte Ausführungsformen der Erfindung sind durch die Merkmale der Unteransprüche definiert.

Die Aufgabe wird durch die vorliegende Erfindung dadurch gelöst, dass ein Verfahren zur Herstellung von transgenen Pflanzen mit erhöhter Virusresistenz zur Verfügung gestellt wird, das dadurch gekennzeichnet ist, dass die Expression von pflanzlichen DnaJ-ähnlichen Proteinen, die mit viralen Komponenten interagieren, im Wesentlichen unterbunden wird. Die Aufgabe wird ebenfalls durch ein Verfahren zur Herstellung von transgenen Pflanzen gelöst, wobei die Interaktion von viralen Komponenten mit pflanzlichen DnaJ-ähnlichen Proteinen, die mit viralen Komponenten interagieren, durch Expression von dominant-negativen Mutanten von DnaJ-ähnlichen Proteinen oder Antikörpern gegen DnaJ-ähnliche Proteine im Wesentlichen unterbunden wird.

Während bei der Pathogen-vermittelten Resistenz die Funktion einer viralen Komponente spezifisch unterbunden wird und bei anderen Strategien zur Erzeugung von virusresistenten Pflanzen, wie der Expression des PAP-Proteins oder der Überführung des 2,5-Oligoadenylat-Systems von tierischen auf pflanzliche Zellen, generell antiviral wirkende Systeme in die Pflanzen eingebracht werden, wird bei einem Verfahren gemäß der vorliegenden Erfindung eine grundlegend andere Strategie gewählt.

Es ist nämlich überraschenderweise festgestellt worden, dass durch die Suppression bzw. das Silencing von pflanzlichen Wirtsgenen, deren Proteinprodukte durch Interaktion mit Viruskomponenten eine essentielle Bedeutung für die Replikation, Assemblierung sowie die Ausbreitung des Virus haben, Pflanzen hergestellt werden können, die über eine erhöhte Virusresistenz verfügen, ohne dass diese Pflanzen einen in signifikanter Weise beeinträchtigten Phänotyp aufweisen. Dieser Befund war insbesondere angesichts der überragend großen Bedeutung von DnaJ-ähnlichen Proteinen für die funktionelle Integrität von zellulären Systemen unerwartet.

Bei dem DnaJ-Protein oder DnaJ-ähnlichen Proteinen (auch HSP40- bzw. HSP40-ähnliche Proteine genannt) handelt es sich um wichtige Vertreter des Chaperon-Systems von Zellen. Chaperone sind Proteine oder Proteinkomplexe, die in vielfältige zelluläre Prozesse verwickelt sind. Dazu gehören u.a. die aktive Faltung von Proteinen, die Verhinderung der Aggregation von Proteinen, die Auflösung von Proteinaggregaten, die korrekte Faltung von fehlgefalteten Proteinen, die Ausbildung und Auflösung von Cytoskelett-Strukturen und das Protein-Targeting in verschiedene zelluläre Kompartimente (Hartl et al., 2002, Science, 295, 1852-8 und 1996, Nature, 381, 571-9).

DnaJ-ähnliche Proteine sind durch das Vorhandensein einer so genannten J-Domäne gekennzeichnet. Sie interagieren mit anderen Chaperonen, wie z. B. DnaK-ähnlichen Proteinen (auch Hsp70- oder Hsc70-Proteine genannt), deren ATPase-Aktivität sie stimulieren können. DnaK-Proteine verfügen über eine ATP- und eine Substrat-Bindungsdomäne. Im ATP gebundenen Zustand besitzt DnaK nur eine geringe Affinität für seine Substrate.

DnaJ-Proteine binden über die J-Domäne an DnaK-Proteine und stimulieren deren ATPase-Aktivität, was zur Folge hat, dass die DnaK-Proteine wegen der Aktivierung durch die DnaJ-Proteine ein größeres Substratspektrum binden können (Kelley, 1999, Current Biology, 9: 305-308 und Kelley, 1998, TIBS, 23: 222-227). Damit fungieren DnaJ-Proteine u.a. als Adaptoren für Substrate von DnaK-Proteinen und erweitern deren Substratspezifität. Sowohl DnaJ- als auch DnaK-Proteine verfügen über zahlreiche Homologe in verschiedenen Spezies. Dabei gibt es zunehmend Hinweise dafür, dass spezifische Mitglieder der DnaK-Familie nur mit speziellen Mitgliedern der DnaJ-Familie interagieren (Kelley, 1999, Current Biology, 9: 305-308). Entscheidend für die Funktion der DnaJ-Proteine ist dabei das Vorhandensein einer J-Domäne, über die diese Proteine an ihre DnaK-Partner binden.

Die Fähigkeit von J-Domänen, andere Chaperone zu rekrutieren, machen sich auch Viren, die pflanzliche oder tierische Zellen befallen, zu Nutzen. Einige virale Proteine binden z. B. direkt an Hsp70-Proteine. Wieder andere virale Proteine, wie z. B. das "Large T Antigen" verfügen über J-Domänen. Es wird vermutet, dass diese viralen Proteine das zelluläre Chaperon-System nützen, um entweder virale Proteinkomplexe zu deassemblieren, zu assemblieren oder zelluläre Komplexe, die der Ausbreitung von Viren innerhalb der Zelle entgegenstehen, aufzulösen (Sullivan et al., 2001, Virology, 287: 1-8, Kelley, 1998, TIBS, 23: 222-227).

Für pflanzliche Closteroviren ist z. B. gezeigt worden, dass sie ein eigenes Hsp70-ähnliches Protein enthalten, das für die Assemblierung und Ausbreitung der Viren essentiell ist (Alzhanova et al., 2001, EMBO J., 20: 6997-7007). Darüber hinaus konnte gezeigt werden, dass das Movement-Protein NSm des tomato spotted wilt virus (TSWV) mit DnaJ-ähnlichen Proteinen aus *Arabidopsis thaliana* und *Lycopersicon esculentum* interagiert (Von Bargen et al., 2001, Plant Physiol. Biochem., 39: 1083-1093 und Soellick et al., 2000, PNAS, 97: 2373-2378). Im Rahmen der vorliegenden Erfindung wird zudem gezeigt, dass das Capsid-Protein CP der Potyviren potato virus Y (PVY) und tobacco etch virus (TEV) ebenfalls mit einem DnaJ-ähnlichen Protein interagiert (siehe Beispiele).

Es kann also davon ausgegangen werden, dass Pflanzenviren während der Infektion sich der Funktionen des zellulären Chaperon-Systems bedienen und dazu u.a. DnaJ-ähnliche Proteine verwenden. Damit stellen die DnaJ-ähnlichen Proteine theoretisch einen Angriffspunkt für antivirale Strategien dar. Wegen ihrer überragenden Bedeutung als Chaperone für vielfältige zelluläre Funktionen im nicht-infizierten Wirt konnte jedoch nicht davon ausgegangen werden, dass die Suppression von DnaJ-ähnlichen Proteinen zu lebensfähigen Pflanzen mit einem im Wesentlichen nicht veränderten Phänotyp führen würde.

Durch die vorliegende Erfindung konnte jetzt erstmals gezeigt werden, dass die Suppression der Expression von DnaJ-ähnlichen Proteinen durch Silencing zu Pflanzen führt, die über eine erhöhte Virusresistenz verfügen und deren Phänotyp darüber hinaus im Wesentlichen unverändert zu Wildtyp-Pflanzen ist. Durch die vorliegende Erfindung kann ebenfalls erstmals gezeigt werden, dass die Expression von dominant-negativen Mutanten von DnaJ-ähnlichen Proteinen, die nicht mehr in der Lage sind, mit viralen Faktoren oder ihren zellulären Bindungspartnern zu interagieren, die Herstellung von Pflanzen erlaubt, die über eine erhöhte Virusresistenz verfügen. Das erfindungsgemäße Verfahren, nach dem Pflanzen mit einer erhöhten Virusresistenz dadurch hergestellt werden, dass die Expression oder das Bindungsverhalten von DnaJ-ähnlichen Proteinen verändert werden, stellt gegenüber den anderen Verfahren des Stands der Technik nicht nur eine nützliche Alternative dar, es weist darüber hinaus auch Vorteile auf.

Da DnaJ-ähnliche Proteine und das Chaperon-System von pflanzlichen Wirtszellen in vielfältiger Weise in den Infektionsprozess von Pflanzenviren involviert sind, bietet das erfindungsgemäße Verfahren die Möglichkeit, z.B. transgene Pflanzen herzustellen, die resistent gegenüber einem breiten Virus-Spektrum sind, d.h. resistent sowohl gegen unterschiedliche Virusgruppen als auch -stämme. Das erfindungsgemäße Verfahren kann, wie im Folgenden noch ausgeführt wird, sehr effizient durchgeführt werden und es ist generell anwendbar.

Je nachdem ob mehrere Isoformen von DnaJ-ähnlichen Proteinen oder nur spezifische DnaJ-ähnliche Proteine gesilenct werden und abhängig von der Bindungsspezifität der viralen Komponenten für die DnaJ-ähnlichen Proteine, können Pflanzen hergestellt werden, die eine Resistenz gegenüber einem breiten Virusspektrum oder gegenüber spezifischen Viren zeigen. Zum Beispiel zeigen die im Rahmen der Erfindung näher charakterisierten DnaJ-ähnlichen Proteine NtCPIP1, NtCPIP2a und 2b im Hefe - Two - Hybrid System eine Spezifität für Komponenten der Potyviren PVY und TEV, wohingegen das DnaJ-ähnliche Protein NtDnaJ_M541 bevorzugt mit Komponenten des TSWV interagiert.

Dem Fachmann ist klar, dass in manchen Fällen die Dauer der Resistenz auch von der Art und Anzahl der gesilencten DnaJ-ähnlichen Proteine abhängen kann (transiente Resistenz), da z. B. bei der Supression eines einzelnen DnaJ-ähnlichen Proteins die viralen Komponenten unter Umständen noch mit anderen Isoformen des DnaJ-ähnlichen Proteins interagieren können. In diesem Fall wird sich der Virus trotz einer transienten Resistenz langfristig in der Pflanze ausbreiten können. Allerdings besteht auch an Pflanzen, die eine nur transienten Virusresistenz aufweisen, Interesse, wie weiter unter ausgeführt wird.

Erfindungsgemäß werden unter pflanzlichen DnaJ-ähnlichen Proteinen solche Proteine verstanden, die über eine J-Domäne verfügen und in der Lage sind, mit viralen Komponenten, d.h. viralen (Glyko-)Proteinen, viralen Nukleinsäuren und/oder viralen Lipiden zu interagieren. Erfindungsgemäß verfügen pflanzliche DnaJ-ähnliche Proteine dann über eine J-Domäne, wenn ein Aminosäurebereich des pflanzlichen Proteins signifikante Homologie zu der Konsensus-Sequenz der J-Domäne zeigt. Eine solche Homologie wird üblicherweise über verschiedene Alignment-Programme, wie z. B. CLUSTAL festgestellt. Allgemein stehen dem Fachmann zur Bestimmung der Sequenzidentität/-ähnlichkeit geeignete Algorithmen zur Verfügung, z.B. auch das Programm, das unter http://www.ncbi.nlm.nih.gov/BLAST (z.B. der Link "Standard nucleotide-nucleotide BLAST [blastn]") zugänglich ist.

Ein weiteres Programm, das bevorzugt zur Bestimmung von Sequenzhomologien verwendet werden kann, ist ClustalW des MegAlign-Programms von DNASTAR Inc. (Madison, NY).

DnaJ-Proteine sind strukturell unterschiedlich und werden je nach der Kombination dreier (zur J-Domäne) zusätzlicher unterschiedlicher Domänen, wie sie ursprünglich im *E. coli*-Ortholog identifiziert wurden, gruppiert: eine Gly/Phe-reiche Domäne, eine Cys-reiche Zinkfingerdomäne und eine weniger gut konservierte C-terminale Domäne, die möglicherweise an der Substratbindung beteiligt ist.

Abhängig von der Kombination dieser Domänen sind DnaJ-ähnliche Proteine in z.B. drei Gruppen I bis III eingeteilt worden (Cheetham et al. (1998) Cell Stress Chaperones, 3, 28-36).

Eine umfassende Analyse von DnaJ-ähnlichen Proteinen in *Arabidopsis thaliana* hat zudem zu einer weiteren Klassifizierung anhand der Ähnlichkeit der J-Domäne geführt (Miernyk (2001) Cell Stress Chaperones, 6, 209-218).

Die erfindungsgemäßen pflanzlichen DnaJ-ähnlichen Proteine, wie sie im Rahmen der vorliegenden Erfindung identifiziert wurden, bilden eine eigene Unterklasse, die durch das alleinige Vorhandensein einer J-Domäne charakterisiert ist. Diese pflanzlichen DnaJ-ähnlichen Proteine sind bei der Herstellung der erfindungsgemäßen Pflanzen und Pflanzenzellen bevorzugt. Andere pflanzliche DnaJ-ähnlichen Proteine bzw. deren kodierende Sequenzen sind aber auch verwendbar. Ebenso sind nichtpflanzliche DnaJ-ähnliche Proteine bzw. deren kodierenden Sequenzen verwendar, z.B. wenn die Herstellung virusresistenter Pflanzen durch die Expression dominant-negativer Mutanten erfolgt.

Als pflanzliche DnaJ-ähnliche Proteine werden neben den im Rahmen der vorliegenden Erfindung identifizierten DnaJ-ähnlichen Proteinen und deren Homologen bevorzugt solche Proteine bezeichnet, die dem phylogenetischen Stamm 5 der DnaJ-ähnlichen Proteine aus *Arabidopsis thaliana* angehören, oder deren Homologe (Miernyk et al., vide supra, Seite 213). Insgesamt konnten in *Arabidopsis thaliana* 89 DnaJ-ähnliche Proteine identifiziert werden, von denen der phylogenetische Stamm 5 sieben Mitglieder umfasst, die alle eine vorhergesage cytosolische Lokalisation aufweisen. Die Mitglieder dieser Klasse, sowie die Homologen hierzu, werden im Rahmen der vorliegenden Erfindung ebenfalls bevorzugt als pflanzliche DnaJ-ähnliche Proteine bezeichnet.

Der erfindungsgemäß bevorzugten Gruppe der pflanzlichen DnaJ-ähnlichen Proteine aus Tabak (NtDnaJ-M541), Tomate (Le19/8) und *Arabidopsis* (AtA39), die kürzlich als TSWV MP (NSm)-bindende Proteine identifiziert wurden, fehlen mit Ausnahme der gemeinsamen J-Domäne andere DnaJ-typische Domänenstrukturen (Soellick et al. (2001) *vide supra,* von Bargen et al. (2001) *vide supra*). Vielmehr konnte eine Lys-reiche Domäne (4x K-X-X-X-K-E/K) identifiziert werden, die teilweise auch in den im Rahmen der Erfindung identifizierten NtCPIP-Proteinen (Aminosäuren 172 - 177 und 269 - 274 für NtCPIP1 und Aminosäuren 171 - 176 und 168 - 273 für NtCPIP2a und 2b) identifiziert werden (K = Lysin, E = Arginin, X = beliebige Aminosäure).

Erfindungsgemäß werden als DnaJ-ähnliche Proteine somit alle pflanzlichen Proteine bezeichnet, die über eine J-Domäne verfügen. Erfindungsgemäß werden als pflanzliche DnaJ-ähnliche Proteine insbesondere solche Proteine bezeichnet, die nur über eine J-Domäne und eventuell die oben genannte Lys-reiche Domäne aufweisen.

Erfindungsgemäß verfügen pflanzliche DnaJ-ähnliche Proteine insbesondere dann über eine J-Domäne, wenn sie über Aminosäuresequenzen verfügen, die homolog zu den drei gut konservierten Clustern von J-Domänen sind, die den Sekundärstrukturelementen Helix I, Helix II und Helix III entsprechen. Insbesondere das HPD-Tripeptid der Helix II, wie charakteristische Aminosäuren der Helix III sollten vorhanden sein (Kelley, 1998, TIBS, 23: 222-227).

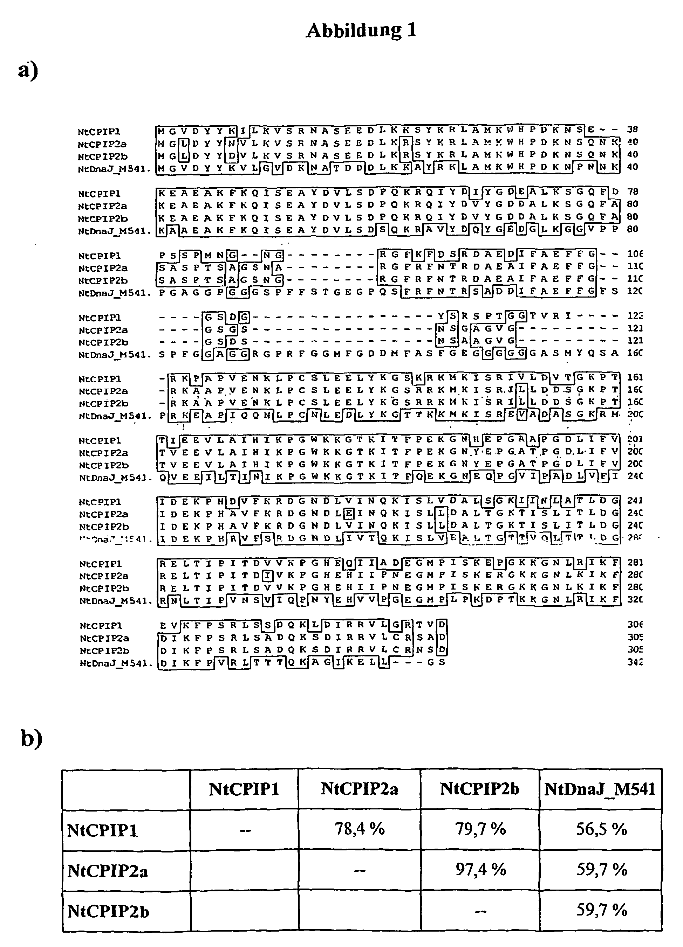

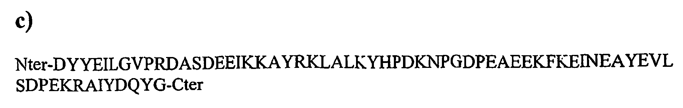

Ein Alignment, das mehrere erfindungsgemäße pflanzliche DnaJ-ähnliche Proteine, die nachgewiesenermaßen mit viralen Komponenten interagieren, ist in Abbildung 1 gezeigt. Es handelt sich dabei um die im Rahmen dieser Erfindung neu identifizierten Proteine NtCPIP1, NtCPIP2a und NtCPIP2b aus Tabak sowie das NtDnaJ_M541 (Soellick et al., vide supra). Die für die J-Domäne charakteristischen Sequenzelemente finden sich im N-Terminus der vorgenannten Proteine, d.h. innerhalb der ersten 70 Aminosäuren.

Eine für J-Domänen charakteristische Sequenz ist in Abbildung 1c gezeigt. Es handelt sich um SEQ ID NO:7. Bereiche eines Proteins zeigen dann eine signifikante Homologie zu der in SEQ ID NO:7 angegebenen Sequenz, wenn die Sequenz dieses Bereichs zu mindestens 40%, bevorzugt zu mindestens 50%, besonders bevorzugt zu mindestens 60%, ebenfalls besonders bevorzugt zu mindestens 70% und/oder 75%, insbesondere bevorzugt zu mindestens 80%, 82%, 84%, 86% und/oder 88% und am meisten bevorzugt zu mindestens 90%, 92%, 94%, 96%, 98% und/oder 99% identisch zu der Sequenz mit SEQ ID NO:7 (Abbildung 1c) ist. Dabei handelt es sich um die DnaJ-Konsensus Sequenz, wie sie in der Conserved Domain Database des NCBI unter der Accesion No: pfam00226.4 zu finden ist.

Als ein Beispiel für die Identitätsgrade von verschiedenen J-Domänen sind in der Abbildung 1a das Alignement von verschiedenen J-Domänen verschiedener DnaJ-ähnlicher Proteine gezeigt. In der Abbildung 1b ist die Identität (als "percent identity") angegeben) bzw. Abstand (als "divergence" angegeben) gezeigt. Es handelt sich um die im Rahmen der Erfindung charakterisierten Proteine NtCPIP1, NtCPIP2a, NtCPIP2b sowie um NtM541, Le19-8 (NCBI Accesion No: AJ295232), AtA39, (NCBI Accesion No: AL021749, St_DnaJ (NCBI No: T07371), Nt_DnaJ (NCBI Accesion No: CAC12824.1), At_DnaJ (NCBI Accesion No: T49127), R1_DnaJ (NCBI Accesion No: Y14649), Sc_DnaJ (NCBI Accesion No: NP_014335) und Ca_DnaJ (NCBI Accesion No: P30725).

Die höchste Ähnlichkeit zu den im Rahmen der vorliegenden Erfindung identifizierten pflanzlichen DnaJ-ähnlichen Proteine NtCPIP1, 2a und 2e zeigen verschiedene Proteine aus *Arabidopsis thaliana.* Die GenBank Zugangsnummem für die Gene dieser DnaJ-ähnlichen Proteine lauten AAD39315, AAS32885, AAF07844, AAD25656 und T48181. Diese Proteine sowie die Homologen hierzu werden im Rahmen der vorliegenden Erfindung ebenfalls bevorzugt als pflanzliche DnaJ-ähnliche Proteine bezeichnet.

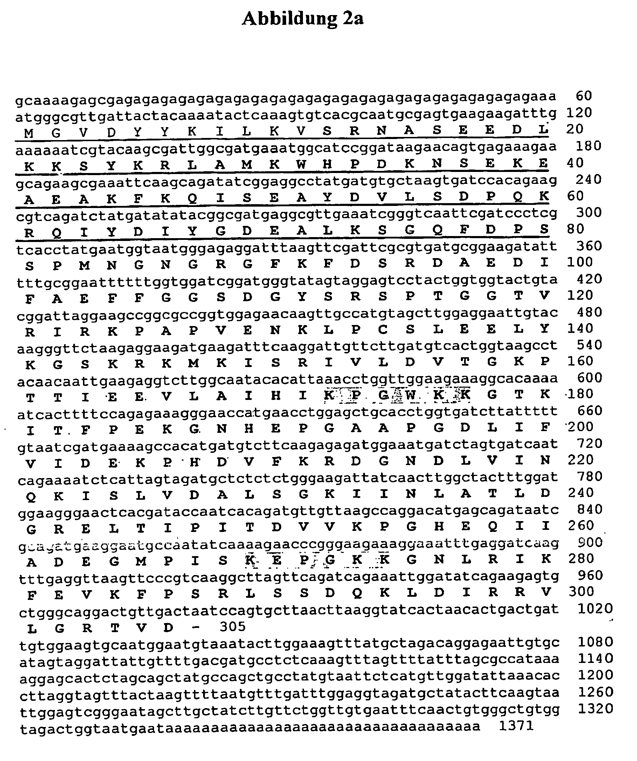

Insbesondere sind mit pflanzlichen DnaJ-ähnlichen Proteinen, deren Suppression durch Silencing erfindungsgemäß für die Herstellung von Pflanzen mit einer erhöhten Virusresistenz verwendet werden kann, solche Proteine gemeint, die eine Aminosäuresequenz der Sequenz ID Nos. 4-6 aufweisen oder zu diesen homolog sind (siehe auch Abbildung 2a bis 2c). Unter Homologie wird im Rahmen der vorliegenden Erfindung verstanden, dass die Proteine eine Identität von mindestens 30%, bevorzugt von mindestens 40%, besonders bevorzugt von mindestens 50%, ebenfalls besonders bevorzugt von mindestens 60%, insbesondere bevorzugt von mindestens 70% und/oder 75% und am meisten bevorzugt von mindestens 80%, 82%, 84%, 86% und/oder 88% oder von mindestens 90%, 92% 94%, 96%, 98% und/oder 99% auf Aminosäureebene aufweisen. Für den Fachmann ist es offensichtlich, dass bei Sequenzidentität von über 80% es sich um homologe Proteine handelt.

Gegenstand der Erfindung sind generell Pflanzen und Pflanzenzellen, die aufgrund einer Modulation der Genexpression und/oder des Bindungsverhalten von pflanzlichen DnaJ-ähnlichen Proteinen eine transiente oder permanente Virusresistenz aufweisen. Unter Modulation der Expression von pflanzlichen DnaJ-ähnlichen Proteinen wird dabei verstanden, dass die Expression dieser Proteine im Vergleich zu entsprechenden Wildtyp-Kontrollpflanzen herauf- oder herunterreguliert ist. Bei einer erfmdungsgemäß bevorzugten Ausführungsform Erfindungsgemäß findet eine Suppression der Expression von pflanzlichen DnaJ-ähnlichen Proteinen zur Herstellung von Pflanzen mit einer erhöhten Virusresistenz statt. In anderen Fällen kann auch die Überexpression von pflanzlichen DnaJ-ähnlichen Proteinen zur Herstellung von Pflanzen mit einer erhöhten Virusresistenz bevorzugt sein, insbesondere dann, wenn es sich dabei um dominant-negative Mutanten der DnaJ-ähnlichen Proteine handelt.

Unter Modulation des Bindungsverhaltens von pflanzlichen DnaJ-ähnlichen Proteinen wird verstanden, dass die pflanzlichen DnaJ-ähnlichen Proteine Mutationen aufweisen, die dazu führen, dass die DnaJ-ähnlichen Proteine nicht oder nur noch sehr beschränkt in der Lage sind, entweder mit viralen Faktoren, wie z.B. Proteinen, oder ihren physiologischen zellulären Bindungspartnern zu interagieren. Solche Pflanzen können z.B. durch Überexpression dominant-negativer Mutanten von DnaJ-ähnlichen Proteinen hergestellt werden, wenn es sich um transgene Pflanzen handelt. Alternativ können dominant-negative Mutationen in den endogenen DnaJ-ähnlichen Proteinen eingeführt werden, um virusresistente Pflanzen zu erhalten.

Die Herstellung solcher Pflanzen bzw. Pflanzenzellen durch das so genannte "TILLING"-Verfahren ist weiter unten detailliert dargestellt.

Das erfindungsgemäße Verfahren kann auch verwendet werden, um transgene Pflanzen mit einer erhöhten Virusresistenz herzustellen. Solche Pflanzen können gegen unterschiedliche Klassen, Gruppen und Stämme von Viren resistent sein. Bevorzugt sind transgene Pflanzen, die durch ein erfindungsgemäßes Verfahren hergestellt werden, gegen Poty-, Carla-, Hordei-, Potex-, Bunyai-, Clostero-, Cucumo-, Diantho-, Tobamo-, Gemini-, Lutero-, Rymo-, Tobra-, Furo-, Como-, Nepo-, Bromo- und Tospoviren resistent.

Besonders bevorzugt sind transgene Pflanzen, die mit dem erfindungsgemäßen Verfahren hergestellt werden, gegen Vertreter von Potyviren resistent, wie z.B. turnip mosaic virus (TuMV), tobacco vein mottling virus (TVMV), plum pox virus (PPV), bean common mosaic virus (BCMV), lettuce mosaic virus (LMV), zucchini yellow mosaic virus (ZYMV), potato virus A (PVA), potato virus Y (PVY) und tobacco etch virus (TEV).

Ebenfalls bevorzugt sind transgene Pflanzen, die nach einem erfindungsgemäßen Verfahren hergestellt wurden, und resistent gegenüber Vertretern der Tospoviren, wie z. B. Bromo-, Chomo- und Caulimoviren sind. Besonders bevorzugt sind transgene Pflanzen, die mit einem erfindungsgemäßen Verfahren hergestellt wurden und gegen den cowpea mosaic virus (CPMV), den tomato spotted wilt virus (TSWV) sowie den cauliflower mosaic virus (CaMV) resistent sind.

Insbesondere sind die transgenen Pflanzen der vorliegenden Erfindung resistent gegen die Poytviren-Virusstämme PVY^{N}, PVY⁰, PVY^{T} und PVY^{C} sowie gegen die potato tuber necrotic isolates (PVY^{NTN}).

Erfindungsgemäß sind transgene Pflanzen, in denen ein spezifisches pflanzliches DnaJ-ähnliches Protein gesilenct wurde, nicht zwangsläufig gegen alle genannten Virenklassen-, gruppen und -stämme resistent. Vielmehr werden solche transgenen Pflanzen insbesondere resistent gegen solche Viren sein, deren Komponenten während des Infektionszyklusses mit dem pflanzlichen DnaJ-ähnlichen Protein interagieren. Da häufig die gleiche Viruskomponente unterschiedlicher Virusgruppen mit dem gleichen DnaJ-ähnlichen Proteinen interagiert, können mit dem erfindungsgemäßen Verfahren transgene Pflanzen hergestellt werden, die gegenüber einem verhältnismäßig breiten Viren-Spektrum resistent sind.

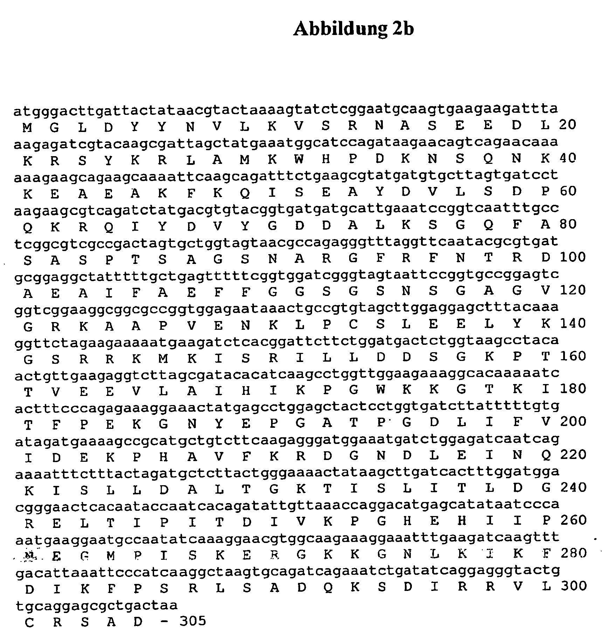

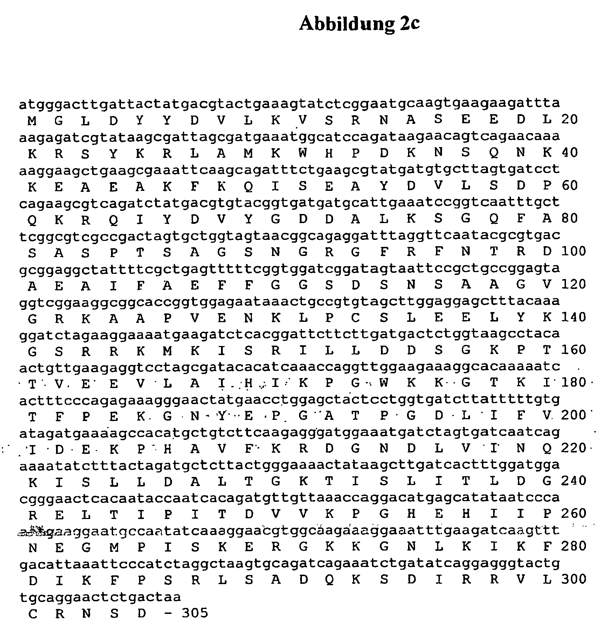

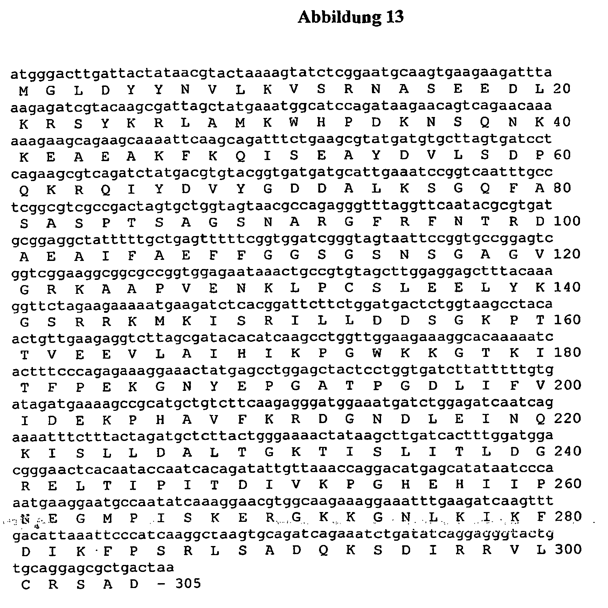

Auf diese Weise können z. B. durch Silencing des DnaJ-ähnlichen Proteins, das durch SEQ ID No. 1 (NtCPIP1, siehe Abbildung 2a) kodiert wird, durch das erfindungsgemäße Verfahren transgene Pflanzen hergestellt werden, die gegenüber dem PVY-Virus und dem TEV-Virus resistent sind. Dies ist darin begründet, dass die Capsid-Proteine dieser Viren mit dem DnaJ-ähnlichen Protein der SEQ ID NO. 4 (NtCPIP1, siehe Abbildung 2a) interagieren. Das Gleiche gilt entsprechend für Proteine, die durch SEQ ID NO:2 (NtCPIP2a, siehe Abbildung 2b), SEQ ID NO:3 (NtCPIP2b, siehe Abbildung 2c) und SEQ ID NO:10 (Abbildung 13) kodiert werden und die Aminosäuresequenzen von SEQ ID NO:5 (NtCPIP2a, siehe Abbildung 2b) und SEQ ID NO:6 (NtCPIP2b, siehe Abbildung 2c) aufweisen.

Pflanzliche DnaJ-ähnliche Proteine mit den Aminosäuresequenzen von NtCPIP1, 2a und 2b (SEQ ID Nos. 4, 5 und 6) und Nukleinsäuresequenzen, die für diese Proteine kodieren, und deren Homologe sowie deren Verwendung für die Herstellung transgener Pflanzen sind offenbart.

Da für den Infektionszyklus wichtige Viruskomponenten unter Umständen auch in der Lage sind, mit verschiedenen zellulären DnaJ-ähnlichen Proteinen (z. B. mit verschiedenen Isoforaren) zu interagieren, kann es in diesen Fällen notwendig sein, mehrere DnaJ-ähnliche Proteine (z. B. die verschieden Isoformen) zu silencen, um eine nachhaltige Virusresistenz der Pflanzen zur erreichen. In den genannten Fällen kann das Silencen nur eines spezifischen DnaJ-ähnlichen Proteins zu einer transienten Resistenz führen. Wie mehrere DnaJ-ähnliche Proteine gesilenct werden können, wird noch beschrieben.

Auch das transiente Silencing bzw. die transiente Resistenz kann von Vorteil sein, da es eine wertvolle Ergänzung zu anderen Verfahren ist, die die Herstellung von Pflanzen mit erhöhter Virusresistenz, aber beeinträchtigtem Phänotyp erlauben. Da Pflanzen, die durch ein erfindungsgemäßes Verfahren hergestellt werden und eine transiente Resistenz während der Etablierung der Virusinfektion zeigen, keinen im Wesentlichen veränderten Phänotyp aufweisen, können erfindungsgemäße Verfahren, die eine transiente Resistenz bewirken, zusammen mit anderen Verfahren helfen, Pflanzen herzustellen, die sich durch eine nachhaltigere und stabilere Virusresistenz auszeichnen, ohne dass der durch die Verfahren bedingte eingeschränkte Phänotyp verschlimmert wird.

Aufgrund des erfindungsgemäß durchgeführten Verfahrens können auch transgene Pflanzenzellen hergestellt werden, die eine erhöhte Resistenz gegenüber Viren aufweisen.

Gegenstand der Erfindung sind neben den transgenen Pflanzenzellen dabei auch immer die durch Regeneration der erfindungsgemäßen Pflanzenzellen erhältlichen transgenen Pflanzen. Bei der transgenen Pflanze bzw. den transgenen Pflanzenzellen kann es sich um jede beliebige monokotyle oder dikotyle Pflanze bzw. Pflanzenzelle handeln, vorzugsweise handelt es sich um Nutzpflanzen bzw. Zellen von Nutzpflanzen. Besonders bevorzugt handelt es sich um Pflanzen mit Speicherorganen, wie z. B. Kartoffel. Ebenfalls bevorzugt sind Zuckerrübe, Tomate, Banane, Karotte, Zuckerrohr, Mais, Erdbeere, Ananas, Papaya.

Ernteprodukte und Vermehrungsmaterial transgener Pflanzen, deren Zellen eine erhöhte Virusresistenz aufweisen sind offenbart. Bei den Ernteprodukten und dem Vermehrungsmaterial handelt es sich insbesondere um Früchte, Samen, Knollen, Wurzelstöcke, Sämlinge, Stecklinge, etc. Es kann sich auch um Teile dieser Pflanzen wie Pflanzenzellen, Protoplasten und Kalli handeln.

Gegenstand der Erfindung sind somit auch transgene Pflanzen, die durch das erfindungsgemäße Verfahren hergestellt wurden und die im Folgenden beschriebenen Nukleinsäuremoleküle in das pflanzliche Genom integriert haben oder diese als autonom replizierende Moleküle enthalten. Z. B. können autonom replizierende Virusvektoren wie PVX (Ruiz et al., 1998, Plant Cell, 10:937-946) oder TRV (Ratcliff et al., 2001, Plant J., 25, 237-245), die für pflanzliche DnaJ-ähnliche Proteine spezifische Sequenzen tragen, verwendet werden, um über das Prinzip des "Virus-induced gene silencing" (VIGS) entsprechende DnaJ-ähnliche Proteine zu silencen.

Bei diesen Pflanzen kann es sich im Prinzip um jede beliebige Pflanze handeln. Vorzugsweise ist es eine monokotyle oder dikotyle Nutz-, Nahrungs- oder Futterpflanze.

Beispiele für monokotyle Pflanzen sind Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören. Dikotyle Nutzpflanzen umfassen unter anderem Baumwolle, Leguminosen wie Hülsenfrüchte und insbesondere Alfalfa, Sojabohne, Raps, Tomate, Zuckerrübe, Kartoffel, Zierpflanzen sowie Bäume. Weitere Nutzpflanzen können Obst (insbesondere Äpfel, Birnen, Kirschen, Weintrauben, Citrus, Ananas und Bananen), Ölpalmen, Tee-, Kakao- und Kaffeesträucher, Tabak, Sisal sowie bei Heilpflanzen Rauwolfia und Digitales umfassen. Besonders bevorzugt sind die Getreide Weizen, Roggen, Hafer, Gerste, Reis, Mais und Hirse, Zuckerrübe, Raps, Soja, Tomate, Kartoffel und Tabak. Weitere Nutzpflanzen können dem US-Patent US 6,137,030 entnommen weden.

Die erfindungsgemäßen Pflanzenzellen umfassen differenzierte und undifferenzierte Pflanzenzellen einschließlich Protoplasten, die durch das erfindungsgemäße Verfahren hergestellt wurden und die die im Folgenden beschriebenen Nukleinsäuremoleküle in das pflanzliche Genom integriert haben oder diese als autonom replizierende Moleküle enthalten.

Erfindungsgemäß können transgene Pflanzen, die über eine erhöhte Virusresistenz verfügen, dadurch hergestellt werden, dass die Expression von pflanzlichen DnaJ-ähnlichen Proteinen im Wesentlichen unterbunden wird. "Im Wesentlichen unterbunden" bedeutet im Rahmen der vorliegenden Erfindung, dass der Expressionslevel von DnaJ-ähnlichen Proteinen in transgenen Pflanzen auf ein Niveau reduziert ist, bei dem die Pflanzen gegen virale Infektionen resistent sind bzw. nach anfänglichen Symptomen einer viralen Infektion Anzeichen für eine Erholung aufweisen. Unter "virusresistenten transgenen Pflanzen" werden im Rahmen der vorliegenden Erfindung auch solche Pflanzen verstanden, die geringe Symptome einer viralen Infektion zeigen, die jedoch so schwach ausgeprägt sind, dass die Verwendbarkeit und Nutzbarkeit der befallenen Pflanzen nicht in Frage gestellt ist. Unter "transient virusresistenten transgenen Pflanzen" werden im Rahmen der vorliegenden Erfindung auch solche Pflanzen verstanden, die für einen vorübergehenden Zeitraum, typischerweise zu Beginn einer viralen Infektion, deutliche Symptome einer Virusresistenz zeigen. Erfindungsgemäße Pflanzen weisen einen im Wesentlichen unveränderten Phänotyp auf, d.h. die Verwendung als Nutz-, Nahrungs- oder Futterpflanze etc. ist nicht in Frage gestellt.

Bei dem erfindungsgemäßen Verfahren zur Herstellung von transgenen Pflanzen mit erhöhter Virusresistenz kann die Suppression der Expression von pflanzlichen DnaJ-ähnlichen Proteinen durch unterschiedliche Strategien erreicht werden. Die Expression von pflanzlichen DnaJ-ähnlichen Proteinen kann in transgenen Pflanzen z. B. durch "silencing" im Wesentlichen unterdrückt werden. Beim silencing wird eine Nukleinsäure, die für pflanzliche DnaJ-ähnliche Proteine oder Teile davon kodiert und/oder dazu komplementär ist, auf die Pflanze übertragen. Um sicherzustellen, dass die Pflanzen für die übertragenen Nukleinsäuren transgen sind, wird die zu übertragende Nukleinsäure in der Regel durch einen Vektor, wie z. B. ein Plasmid auf die Pflanze übertragen, der in der Lage ist, sich innerhalb der Pflanzenzelle stabil zu replizieren oder die übertragene Nukleinsäure in das pflanzliche Genom zu integrieren.

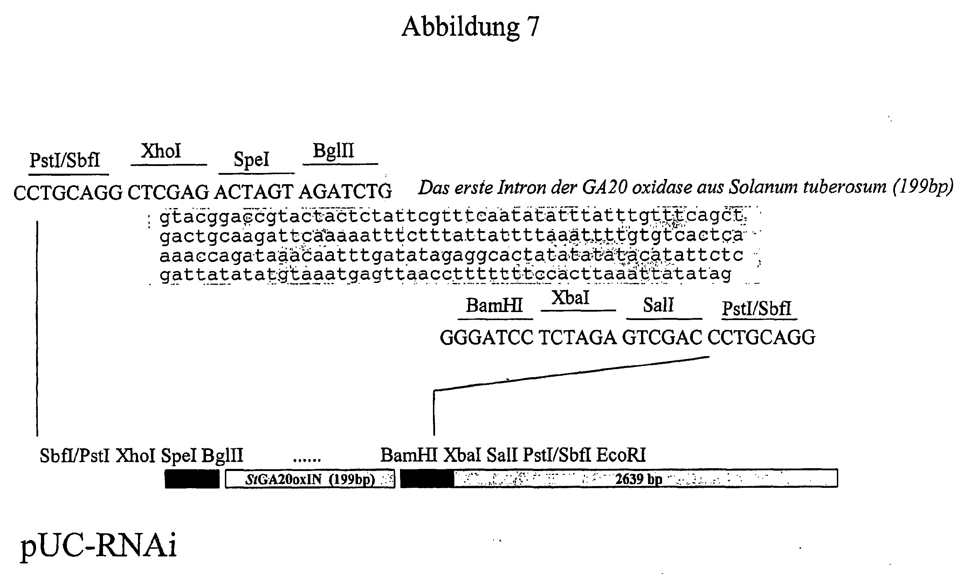

Besonders bevorzugt kann für das silencing von pflanzlichen DnaJ-ähnlichen Proteinen das RNAi-Verfahren verwendet werden. Dabei wird ein Vektor auf die Pflanzenzelle übertragen, der in 5'-3'-Richtung die folgenden Elemente umfasst: Einen in Pflanzen funktionsfähigen Promotor; operativ daran gebunden eine DNA-Sequenz, die die Antisense-Sequenz der für das pflanzliche DnaJ-ähnliche Protein oder Teile davon kodierenden Sequenz umfasst und an ihrem 3'-Ende vom Spleißosom erkennbare 3'-Exonsequenzen aufweist; ein Intron; eine DNA-Sequenz, die die Sense-Sequenz, der für das pflanzliche DnaJ-ähnliche Protein oder Teile davon kodierenden DNA-Sequenz umfasst und an ihrem 5'-Ende vom Spleißosom erkennbare 5'-Exon-Sequenzen aufweist; sowie eine Terminationssequenz. Ein solcher Vektor ist in Abbildung 7 dargestellt. Die Position der Antisense und Sense-Sequenzen kann natürlich vertauscht werden. Dem Fachmann ist dabei klar, dass die jeweiligen 5' und 3'-Splice sites entsprechend angepasst werden müssen.

Werden solche Vektoren stabil auf Pflanzenzellen übertragen, entsteht bei der Transkription dieser Vektoren zunächst eine prä-mRNA, die aus einem ersten Exon, das die Antisense-Sequenz der für pflanzliche DnaJ-ähnliche Proteine oder Teile davon kodierenden Sequenz umfasst, einem Intron und einem zweiten Exon, das die Sense-Sequenz, der für pflanzliche DnaJ-ähnliche Proteine oder Teile davon kodierenden DNA-Sequenz umfasst, besteht. Da durch den Spleiß-Vorgang das Intron entfernt wird, entsteht ein kontinuierliches RNA-Molekül mit Bereichen, die zueinander komplementär sind. Ein solches RNA-Molekül wird eine doppelsträngige Struktur ausbilden (Smith et al., 2000, Nature, 407:319-320).

Solche doppelsträngigen RNA-Moleküle sind in der Lage, durch Induktion des PTGS-Systems spezifisch die mRNA von pflanzlichen DnaJ-ähnlichen Proteinen zu silencen, so dass als Folge pflanzliche DnaJ-ähnliche Proteine nicht mehr exprimiert werden. Welche pflanzlichen DnaJ-ähnlichen Proteine nicht mehr exprimiert werden, kann dabei durch die entsprechende Wahl der Antisense- und Sense-Sequenzen bestimmt werden. Je nachdem, ob diese Antisense- und Sense-Sequenzen nur die für die J-Domäne kodierenden Sequenzen oder Teile davon umfassen, oder Sequenzen umfassen, die für das jeweilige DnaJ-ähnliche Protein charakteristisch sind, ist es daher möglich, entweder eine Vielzahl von DnaJ-ähnlichen Proteinen oder nur ganz spezifische DnaJ-ähnliche Proteine zu silencen. Die für ein Protein charakteristischen Sequenzen können ebenso wie die für die J-Domäne charakteristischen Aminosäurebereiche durch Sequenzhomologieprogramme bestimmt werden. Das Auffinden von Protein-charakteristischen Sequenzen gehört dabei zum üblichen Wissen eines Fachmanns. Der Fachmann weiß, dass eine Vielzahl von pflanzlichen DnaJ-ähnlichen Proteinen auch durch die mehrfache Verwendung von jeweils charakteristischen Sequenzen gesilenct werden können.

Der Fachmann weiß ebenfalls, dass neben den genannten Vektoren auch andere Vektoren für das RNAi-Verfahren bzw. PTGS eingesetzt werden können. Solche Vektoren können z. B. so gebaut sein, dass die Sense- und Antisense-Sequenzen jeweils von einem U6-Promotor ausgehend transkribiert werden, in der Zelle hybridisieren und das PTGS-System induzieren (Tuschl, 2002, Nat. Biotechnol. 20,446-448; Miyagishi et al., 2002, Nat. Biotechnol., 20, 497-500; Lee et al., 2002, Nat. Biotechnol., 20, 500-505). Bei anderen Vektoren sind die Sense- und Antisense-Sequenzen durch eine "loop"-Sequenz verbunden und werden von einem humanen RNAse P RNA H1-Promotor transkribiert. Durch Rückfaltung des "loop" können die Sense- und Antisense-Sequenzen hybridisieren, doppelsträngige RNA ausbilden und das PTGS-System induzieren (Tuschl, 2002, vide supra; Paul et al., 2002, Nat. Biotechnol., 20, 505-508; Paddison et al., 2002, Genes Dev., 16, in press, Brummelkamp et al., 2002, Science, 296, 550-553).

Bei einer anderen Ausführungsform der Erfindung umfassen die zur Übertragung der Nukleinsäuren verwendeten Vektoren in 5'-3'-Orientierung einen Promotor, operativ daran gebunden eine DNA-Sequenz, die die Antisense-Sequenz der für pflanzliche DnaJ-ähnliche Proteine oder Teile davon kodierenden Sequenz umfasst, sowie eine Terminationssequenz. Bei der Transkription solcher Vektoren in Pflanzenzellen, entsteht ein RNA-Molekül, dessen Sequenz komplementär zu der für pflanzliche DnaJ-ähnliche Protein oder Teile davon kodierenden Sequenz ist. Durch Hybridisierung der Antisense-Sequenz mit endogenen mRNA-Sequenzen von pflanzlichen DnaJ-ähnlichen Proteinen in vivo kann daher die Expression von pflanzlichen DnaJ-ähnlichen Proteinen in Pflanzenzellen unterdrückt werden.

In einer weiteren Ausführungsform umfassen die Vektoren, die zur Übertragung der Nukleinsäuren verwendet werden, in 5'-3'-Orientierung einen Promotor, operativ daran gebunden eine DNA-Sequenz, die die für pflanzliche DnaJ-ähnliche Proteine oder Teile davon kodierende Sequenz umfasst und zu sich selbst komplementäre Abschnitte enthält, sowie eine Terminationssequenz. Bei der Transkription dieser Vektoren in der Pflanzenzelle entstehen RNA-Moleküle, die über Sequenzbereiche verfügen, die mit sich selbst hybridisieren können. Dadurch können in der Zelle doppelsträngige RNA-Moleküle auftreten, die das PTGS-Systems induzieren, was dann dazu führt, dass die mRNA der entsprechenden DnaJ-ähnlichen Proteine spezifisch abgebaut wird. Dieses auch als Co-Suppression bezeichnete Verfahren zum Silencing von pflanzlichen Proteinen setzt voraus, dass die mRNA des zu supprimierenden pflanzlichen DnaJ-ähnlichen Proteins über Abschnitte verfügt, die zueinander komplementär sind. Solche Abschnitte können vom Fachmann durch einfache visuelle Inspektion der für das jeweilige Protein kodierenden DNA-Sequenz oder durch entsprechende Sequenzprogramme wie z. B. DNAStar der DNASTAR Inc., Madison, USA, identifiziert werden.

Bei einer anderen Ausführungsform des erfindungsgemäßen Verfahrens werden zur Übertragung der Nukleinsäuren auf die Pflanzenzellen Vektoren verwendet, die in 5'-3'-Orientierung einen in Pflanzen funktionsfähigen Promotor, operativ daran gebunden eine DNA-Sequenz, die für ein Ribozym kodiert, das spezifisch die mRNA von pflanzlichen DnaJ-ähnlichen Proteinen erkennt, sowie eine Terminationssequenz umfassen. Dem Fachmann ist bekannt, wie Ribozyme, die eine gegen eine bestimmte mRNA gerichtete Endonuklease-Aktivität besitzen, hergestellt werden können. Im Detail ist dies z. B. in Steinecke et al., 1992, EMBO J., 11: 1525 beschrieben. Im Rahmen dieser Erfindung sind mit dem Begriff "Ribozymen" auch solche RNA-Sequenzen gemeint, die neben dem eigentlichen Ribozym noch Führungssequenzen umfassen, die zu der mRNA der DnaJ-ähnlichen Proteine oder Teilen davon komplementär sind und so das mRNA-spezifische Ribozym noch gezielter zum mRNA-Substrat des Ribozyms führen.

Eine weitere Alternative zur Herstellung von transgenen Pflanzen mit erhöhter Virusresistenz, bietet die Übertragung von Nukleinsäuren durch Vektoren, die in 5'-3'-Orientierung einen in Pflanzen funktionsfähigen Promotor, operativ daran gebunden eine DNA-Sequenz, die Antisense-Sequenzen der für die pflanzlichen DnaJ-ähnlichen Proteine oder Teile davon kodierenden Sequenzen sowie die für RNAse P kodierende Sequenz umfasst, sowie eine Terminationssequenz umfassen. Bei der Transkription solcher Vektoren entstehen in der Zelle RNA-Moleküle, die über eine Führungssequenz (die Antisense-Sequenz) verfügen, welche die RNAse P zur mRNA des DnaJ-ähnlichen Proteins führt, wodurch die Spaltung der mRNA durch RNAse P bewirkt wird (US-Patent Nr. 5,168,053). Vorzugsweise umfasst die Führungssequenz 10 bis 15 Nukleotide; die zur DNA-Sequenz des DnaJ-ähnlichen Proteins komplementär sind, und eine 3'-NCCA Nukleotidsequenz, wobei N vorzugsweise ein Purin ist. Die Transkripte der externen Führungssequenz binden an die ZielmRNA über die Ausbildung von Basenpaaren, was die Spaltung der mRNA durch die RNAase P am Nukleotid 5' von der gepaarten Region ermöglicht. Eine solche gespaltene mRNA kann nicht in ein funktionsfähiges Protein translatiert werden.

Darüber hinaus können zur erfindungsgemäßen Herstellung von trans genen Pflanzen mit einer erhöhten Virusresistenz auch Vektoren verwendet werden, die eine DNA-Sequenz enthalten, die in 5'-3'-Orientierung die folgenden Bestandteile aufweist: Eine DNA-Sequenz, die dem 5'-Bereich der für DnaJ-ähnliche Proteine kodierenden DNA-Sequenz entspricht, eine DNA-Sequenz für Resistenzgene, sowie eine DNA-Sequenz, die dem 3'-Bereich der für pflanzliche DnaJ-ähnliche Proteine kodierenden Sequenz entspricht. Solche Vektoren können verwendet werden, um über homologe Rekombination einen spezifischen Gen-knock-out der interessierenden pflanzlichen DnaJ-ähnlichen Proteine herbeizuführen. Bei Pflanzenzellen, in denen die homologe Rekombination stattgefunden hat, wird in die DNA, die für das pflanzliche DnaJ-ähnliche Protein kodiert, die Sequenz für das Resistenzgen inseriert, so dass keine funktionelle mRNA des pflanzlichen DnaJ-ähnlichen Proteins mehr in der Zelle hergestellt werden kann. Durch Selektion gegen das Resistenzgen können die Pflanzenzellen, in denen die Rekombination stattgefunden hat, identifiziert werden. Wie solche Vektoren zum Gen-Knock-out durch homologe Rekombination im Einzelnen zu konstruieren sind, welche Elemente sie umfassen müssen (Promotoren, Enhancer, flankierende Sequenzen) und wie die Pflanzenzellen des Knock-outs identifiziert werden ist dem Fachmann bekannt. Als Resistenzgene werden typischerweise Antibiotika-Resistenzgene verwendet. Andere Resistenzgene, die eine Selektion der Zellen, in denen die Rekombination stattgefunden hat, erlauben, können natürlich auch verwendet werden.

Wenn im Rahmen der vorliegenden Erfindung von Nukleinsäure-Sequenzen gesprochen wird, die für pflanzliche DnaJ-ähnliche Proteine oder Teile davon kodieren, dann sind damit sowohl die komplette kodierende DNA-Sequenz des jeweiligen pflanzlichen DnaJ-ähnlichen Proteins als auch die komplette mRNA-Sequenz bzw. die jeweiligen Teilbereiche gemeint. Da einige der oben erwähnten Verfahren zur Herstellung von transgenen Pflanzen, in denen die Expression von pflanzlichen DnaJ-ähnlichen Proteinen signifikant reduziert ist, darauf beruhen, dass eine spezifische Hybridisierung zwischen der endogenen mRNA der pflanzlichen DnaJ-ähnlichen Proteine und den Sequenzen, die bei der Transkription der oben erwähnten Vektoren entstehen, stattfindet (wie z. B. der Antisense-Strategie), weiß der Fachmann, dass die übertragenen Nukleinsäuren nicht immer die gesamte für DnaJ-ähnliche Proteine kodierende Sequenz, unabhängig ob es sich um die Sense- oder Antisense-Sequenz handelt, enthalten müssen. Vielmehr können für eine spezifische Hybridisierung bereits relativ kurze Bereiche der für DnaJ-ähnliche Proteine kodierenden Sequenzen ausreichend für ein effizientes Silencing sein.

Bei Vektoren, deren Transkription zu doppelsträngigen RNA-Molekülen führt, reicht es aus, wenn die Sequenzen, die den Sequenzbereichen der mRNA von pflanzlichen DnaJ-ähnlichem Protein entsprechen, um die 25 Nukleotide umfassen. Die übertragenen Sequenzen umfassen in der Regel zwischen 25-1000 Nukleotide, bevorzugt zwischen 25-750 Nukleotide, besonders bevorzugt um die 400-800 und 500-750 Nukleotide. Es können aber auch Sequenzen verwendet werden, die zwischen 25-500 Nukleotide, zwischen 25-300 Nukleotide, zwischen 25-150 Nukleotide und zwischen 25-100 Nukleotide umfassen.

Der Fachmann weiß, dass beim RNAi bzw. PTGS die zur Ausbildung von doppelsträngigen RNA-Molekülen verwendeten Sense- und Antisense RNAs auch um die 21-23 Nukleotide mit einem charakteristischen 3'-Überhang umfassen können (Tuschl, 2002, Nat. Biotechnol. 20, 446-448).

Wenn Nukleinsäuren auf die Pflanzenzellen übertragen werden, deren Transkription in der Zelle zu Sequenzen führen, die zu der mRNA der pflanzlichen DnaJ-ähnlichen Proteine komplementär sind (wie z. B. bei der Antisense-Strategie), dann müssen diese Sequenzen nicht hundertprozentig komplementär zu der mRNA sein. Vielmehr reicht es aus, wenn diese Sequenzen zu mindestens 50%, bevorzugt zu mindestens 60%, besonders bevorzugt zu mindestens 70%, weiterhin besonders bevorzugt zu mindestens 80%, insbesondere bevorzugt zu mindestens 90% und am meisten bevorzugt zu mindestens 95% komplementär sind. Die Abweichungen können dabei durch Deletion, Addition, Substitution und/oder Insertion entstanden sein. Dem Fachmann ist natürlich klar, dass mit abnehmender Komplementarität die Wahrscheinlichkeit steigt, dass mehrere DnaJ-ähnliche Proteine gesilenct werden.

Generell gilt, dass nur solche komplementären Sequenzen erfindungsgemäß verwendet werden können, die in der Lage sind, spezifisch mit mRNA-Bereichen der pflanzlichen DnaJ-ähnlichen Proteine zu hybridisieren. Sequenzen, die in vivo mit in RNA-Bereichen von anderen Proteinen als pflanzlichen DnaJ-ähnlichen Proteinen hybridisieren und deren Silencing bewirken, sind für die erfindungsgemäßen Verfahren nicht geeignet. Je nach der gewählten Sequenz, d.h. ob die Sequenz der J-Domäne entspricht oder den für das jeweilige DnaJ-ähnliche Protein charakteristischen Sequenzbereichen entspricht, und je nach dem Komplementaritätsgrad, wird eine Vielzahl oder einige wenige pflanzliche DnaJ-ähnliche Proteine gesilenct werden. Unter Umständen wird nur die Expression eines ganz spezifischen DnaJ-ähnlichen Proteins unterbunden. Die Länge von komplementären Sequenzen beträgt bevorzugt zwischen 20-1000 Nukeotide, ebenfalls bevorzugt zwischen 20-750 Nukleotide, besonders bevorzugt zwischen 20-500 Nukleotide, ebenfalls besonders bevorzugt zwischen 20-300 Nukleotide, insbesondere bevorzugt zwischen 20-150 Nukleotide, ebenfalls insbesondere bevorzugt zwischen 20-75 Nukleotide und am meisten bevorzugt um die 20-50 Nukleotide. Unter Umständen können die Sequenzen auch nur um die 20 oder 25 Nukleotide umfassen.

Einige der oben erwähnten Verfahren können auch mit Sequenzen durchgeführt werden, die nicht Bestandteil des kodierenden Teils der mRNA von pflanzlichen DnaJ-ähnlichen Proteinen sind oder dazu komplementär sind. Es kann z. B. ausreichend sein, wenn es sich um Sequenzen aus dem 5'- oder 3'- untranslatierten Bereich handelt, sofern diese regulatorischen Sequenzen charakteristisch für die mRNA des DnaJ-ähnlichen Proteins oder der DnaJ-ähnlichen Proteine sind.

Solche Sequenzen können insbesondere dann eingesetzt werden, wenn das Silencing durch doppelsträngige RNA-Konstrukte induziert wird oder die Translation einer mRNA durch Antisense-Konstrukte inhibiert wird. Daher umfasst der Begriff mRNA erfindungsgemäß nicht nur die kodierenden Bestandteile der mRNA von pflanzlichen DnaJ-ähnlichen Proteinen, sondern auch alle regulatorischen Sequenzen, die in der prä-mRNA oder reifen mRNA auftreten und die für die mRNA von DnaJ-ähnlichen Proteinen charakteristisch sind. Dies gilt entsprechend auch für die DNA-Sequenz. Dabei kann es sich z. B. um 5'- und 3'- untranslatierte Bereiche handeln, um Promotorsequenzen, um upstream activating sequences, um Introns etc.

Wenn Vektoren verwendet werden, deren Transkription zu RNA-Molekülen führt, die aus einer Führungssequenz und RNAse P bestehen, dann muss die Führungssequenz ausreichend komplementär sein, um spezifisch pflanzliche DnaJ-ähnliche Proteine zu erkennen. Welcher Bereich der mRNA der pflanzlichen DnaJ-ähnlichen Proteine durch die Führungssequenz erkannt wird, kann gemäß den jeweiligen Erfordernissen gewählt werden. Bevorzugt umfassen solche Führungssequenzen um die 20 Nukleotide, sie sollten jedoch nicht wesentlich kürzer als 15 Nukleotide sein. Bei einer 100%-igen Komplementarität der Führungssequenz sollten auch 12 Nukleotide ausreichend sein. Selbstverständlich können die Führungssequenzen bis zu 100 Nukleotide oder mehr umfassen, da dadurch lediglich ihre Spezifität für die jeweilige mRNA erhöht wird.

Wenn im Rahmen der vorliegenden Erfindung von Sense-Sequenzen gesprochen wird, dann sind damit diejenigen Sequenzen gemeint, die dem kodierenden Strang der Gene für pflanzliche DnaJ-ähnliche Proteine entsprechen oder Teile davon umfassen. Solche Sequenzen müssen jedoch nicht zu 100% identisch mit den für die interessierenden pflanzlichen DnaJ-ähnliche Proteine kodierenden Sequenzen sein. Es genügt, wenn die Sequenzen zu den für DnaJ-ähnliche Proteine kodierenden Sequenzen derart ausreichend ähnlich sind, dass ihre Expression in pflanzlichen Zellen zu einem effizienten und spezifischen Silencing von pflanzlichen DnaJ-ähnlichen Proteinen in der Zelle z. B. durch RNA interference oder Co-Suppression führt. Es sollte ausreichen, wenn diese Sequenzen zu mindestens 50% identisch sind, bevorzugt zu mindestens 60%, besonders bevorzugt zu mindestens 70%, weiterhin besonders bevorzugt zu mindestens 80%, insbesondere bevorzugt zu mindestens 90% und am meisten bevorzugt zu mindestens 95% identisch sind. Bei solchen Identitätsgraden wird erfindungsgemäß davon gesprochen, dass die Sequenzen zueinander homolog sind bzw. eine Homologie aufweisen. Die Abweichungen zu der für DnaJ-ähnliche Proteine oder Teilen davon kodierenden Sequenz können dabei durch Deletion, Addition, Substitution und/oder Insertion entstanden sein. Dem Fachmann ist natürlich klar, dass mit abnehmender Identität die Wahrscheinlichkeit steigt, dass mehrere DnaJ-ähnliche Proteine gesilenct werden. Sequenzen, deren Identitäts- bzw. Homologiegrad so gering ist, dass andere Proteine als pflanzliche DnaJ-ähnliche Proteine gesilenct werden, sind für die erfindungsgemäßen Verfahren nicht ausreichend spezifisch und nicht geeignet.

Die Begriffe Komplementarität, Homologie und Identität sind dem Fachmann bekannt.

Unter Sequenzhomologie bzw. Homologie wird dabei erfindungsgemäß allgemein verstanden, dass die Nukleinsäure- bzw. Aminosäuresequenz eines DNA-Moleküls bzw. eines Proteins zu mindestens 40%, bevorzugt zu mindestens 50%, weiter bevorzugt zu mindestens 60%, ebenfalls bevorzugt zu mindestens 70% und/oder 75%, besonders bevorzugt zu mindestens 80%, 82%, 84%, 86% und/oder 88%, insbesondere bevorzugt zu mindestens90%, 92% und/oder 94% und am meisten bevorzugt zu mindestens 95%, 96%, 97%, 98% und/oder 99% zu den Nukleinsäure- bzw. Aminosäuresequenzen eines bekannten DNA- oder RNA-Moleküls bzw. Proteins identisch sind.

Mit dem Begriff Komplementarität wird die Fähigkeit eines Nukleinsäuremoleküls beschrieben, mit einem anderen Nukleinsäuremolekül aufgrund von Wasserstoffbrücken zwischen komplementären Basen zu hybridisieren. Der Fachmann weiß, dass zwei Nukleinsäuremoleküle nicht über eine 100-prozentige Komplementarität verfügen müssen, um miteinander hybridisieren zu müssen. Bevorzugt ist eine Nukleinsäuresequenz, die mit einer anderen Nukleinsäuresequenz hybridisieren soll, zu dieser zu mindestens 40%, zu mindestens 50%, zu mindestens 60%, bevorzugt zu mindestens 70%, besonders bevorzugt zu mindestens 80%, ebenfalls besonders bevorzugt zu mindestens 90%, insbesondere bevorzugt zu mindestens 95% und am meisten bevorzugt zu mindestens 98 bzw. 100% komplementär.

Nukleinsäuremoleküle sind identisch, wenn sie gleiche Nukleotide in gleicher 5'-3'-Reihenfolge aufweisen.

Wenn entsprechend von Antisense-Sequenzen gesprochen wird, dann sind erfindungsgemäß diejenigen Sequenzen gemeint, die dem nicht kodierenden DNA-Strang der Gene der interessierenden pflanzlichen DnaJ-ähnlichen Proteine entsprechen. Auch diese Sequenzen müssen natürlich nicht hundertprozentig identisch mit der Sequenz des nicht-kodierenden DNA-Strangs der Genen der jeweiligen interessierenden DnaJ-ähnlichen Proteine sein, sondern können die oben genannten Homologiegrade aufweisen. Dieser Sachverhalt kommt auch in dem Umstand zum Ausdruck, dass Antisense-Sequenzen, die definitionsgemäß zu der mRNA eines Gens komplementär sind, nicht zu 100% komplementär zu dieser mRNA sein müssen. Sie können z. B. auch zu mindestens 50% komplementär, bevorzugt zu mindestens 60% komplementär, besonders bevorzugt zu mindestens 70% komplementär, weiterhin besonders bevorzugt zu mindestens 80% komplementär, insbesondere bevorzugt zu mindestens 90% komplementär und am meisten bevorzugt zu mindestens 95%, 98% und/oder 100% komplementär sein. Wie oben ausgeführt, ist es ausreichend, wenn die Antisense-Sequenzen in der Lage sind, spezifisch mit der jeweiligen interessierenden mRNA der pflanzlichen DnaJ-ähnlichen Proteine zu hybridisieren. Die Hybridisierung kann entweder in vivo unter zellulären Bedingungen oder in vitro stattfinden.

Die Hybridisierung einer Antisense-Sequenz mit einer endogenen mRNA-Sequenz findet typischerweise *in vivo* unter zellulären Bedingungen oder *in vitro* statt.

Erfindungsgemäß wird eine Hybridisierung *in vitro* immer unter Bedingungen durchgeführt werden, die stringent genug sind, um eine spezifische Hybridisierung zu gewährleisten. Solche stringenten Hybridisierungsbedingungen sind dem Fachmann bekannt und können der Literatur entnommen werden (Sambrook et al., 2001, Molecular cloning: A laboratory manual, 3rd edition, Cold Spring Harbor Laboratory Press).

Allgemein bedeutet "spezifisch hybridisieren", dass ein Molekül unter stringenten Bedingungen präferenziell an eine bestimmte Nukleotidsequenz bindet, wenn diese Sequenz in einem komplexen Gemisch von (z.B. Gesamt-) DNA oder RNA vorliegt. Der Begriff "stringente Bedingungen" steht allgemein für Bedingungen, unter denen eine Nukleinsäuresequenz präferenziell an ihre Zielsequenz hybridisieren wird, und zu einem deutlich geringeren Ausmaß oder überhaupt nicht an andere Sequenzen. Stringente Bedingungen sind z.T. Sequenz-abhängig und werden unter verschiedenen Umständen unterschiedlich sein. Längere Sequenzen hybridisieren spezifisch bei höheren Temperaturen.

Im Allgemeinen werden stringente Bedingungen so ausgewählt, dass die Temperatur etwa 5 °C unter dem thermischen Schmelzpunkt (Tₘ) für die spezifische Sequenz bei einer definierten Ionenstärke und einem definierten pH liegt. Die Tₘ ist die Temperatur (unter definierter Ionenstärke, pH-und Nukleinsäurekonzentration), bei der 50% der zu der Zielsequenz komplementären Moleküle zu der Zielsequenz im Gleichgewichtszustand hybridisieren. Typischerweise sind stringente Bedingungen solche, bei denen die Salzkonzentration mindestens ungefähr 0,01 bis 1,0 M Natriumionen-Konzentration (oder ein anderes Salz) bei einem pH zwischen 7,0 und 8,3 beträgt und die Temperature mindestens ungefähr 30 °C für kurze Moleküle (also z.B. 10-50 Nukleotide) beträgt. Zusätzlich können stringente Bedingungen, wie oben bereits erwähnt, durch Zugabe destabilisierender Agenzien, wie beispielsweise Formamid, erreicht werden.

Typische Hybridisierungs- und Waschpuffer haben z.B. folgende Zusammensetzung:

| | |
|---|---|
| *Prähybridisierungslösung*: | |
| | 0,5 % SDS |
| | 5x SSC |
| | 50 mM NaPO₄, pH 6,8 |
| | 0, 1 % Na-Pyrophosphat |
| | 5x Denhardt's Lösung |
| | 100 µg/ml Lachssperm |
| | |
| *Hybridisierungslösung:* | Prähybridisierungslsg. |
| | 1x10⁶ cpm/ml Sonde (5-10 min 95°C) |
| | |
| *20x SSC:* | 3 M NaCl |
| | 0,3 M Natriumcitrat |
| | ad pH 7 mit HCl |
| *50x Denhardt's Reagenz:* | 5 g Ficoll |
| | 5 g Polyvinylpyrrolidon |
| | 5 g Bovine Serum Albumine |
| | ad 500 ml A. dest. |

Eine typische Verfahrensweise für die Hybridisierung sieht folgendermaßen aus:

| | | | |
|---|---|---|---|
| *Optional:* | Blot 30 min in 1x SSC/0,1% SDS bei 65°C waschen | | |
| *Prähybridisierung:* | mindestens 2 h bei 50-55°C | | |
| *Hybridisierung:* | über Nacht bei 55-60°C | | |
| *Waschen:* | 05 min | 2x SSC/ 0,1% SDS | Hybridisierungstemp. |
| | 30 min | 2x SSC/ 0,1% SDS | Hybridisierungstemp. |
| | 30 min | 1x SSC/ 0,1% SDS | Hybridisierungstemp. |
| | 45 min | 0,2x SSC/ 0,1% SDS | 65°C |
| | 5 min | 0,1x SSC | Raumtemperatur |

Der Fachmann weiß, dass die genannten Lösungen und das dargestellte Protokoll je nach Anwendung modifiziert werden kann oder muss.

Die Begriffe "Sense" und "Antisense" sind darüber hinaus dem Fachmann bekannt. Der mit dem Silencing von Genen in Pflanzen vertraute Fachmann weiß entsprechend aus dem Stand der Technik auch, wie groß die zum Silencing verwendeten Nukleinsäuremoleküle sein müssen und welche Homologie bzw. Komplementarität sie zu den jeweils interessierenden Sequenzen aufweisen müssen. Erfindungsgemäß können Antisense-Sequenzen, die z. B. in vivo und/oder in vitro nicht spezifisch mit kodierenden Sense-Sequenzen von pflanzlichen DnaJ-ähnlichen Proteinen hybridisieren können, d.h. auch mit den kodierenden Sense-Sequenzen von anderen Protein-Klassen hybridisieren, nicht verwendet werden.

Die Vektoren, die zum Silencing von pflanzlichen DnaJ-ähnlichen Proteinen verwendet werden, umfassen neben der zu übertragenden Nukleinsäuresequenz weitere regulatorische Elemente. Welche konkreten regulatorischen Elemente diese Vektoren enthalten müssen, hängt dabei jeweils von der Methode ab, die mit diesen Vektoren durchgeführt werden sollen. Über welche regulatorischen Elemente und auch anderen Elemente diese Vektoren verfügen müssen, ist dem Fachmann, der mit den verschiedenen oben erwähnten Verfahren zum Herstellen von transgenen Pflanzen, in denen die Expression eines Proteins unterbunden wird, vertraut ist, bekannt.

Typischerweise handelt es sich bei den regulatorischen Elementen, die die Vektoren enthalten, um solche, die die Transkription und, falls erwünscht, Translation in der Pflanzenzelle gewährleisten.

So können die zu übertragenden Nukleinsäuresequenzen beispielsweise unter Kontrolle von in Pflanzen funktionellen Promotoren stehen. Bei diesen Promotoren kann es sich um konstitutive, aber auch induzierbare oder gewebe- bzw. entwicklungsspezifische Promotoren handeln. Darüber hinaus kann es sich auch um virusspezifische Promotoren handeln. Auf diese Weise können z. B. transgene Pflanzen hergestellt werden, die unter normalen Umständen die pflanzlichen DnaJ-ähnlichen Proteine exprimieren, bei Virusbefall jedoch in den zuerst befallenen Zellen aufgrund des virusspezifischen Promotors die Gene für DnaJ-ähnliche Proteine silencen.

Typischerweise wird man als Promotor für Vektoren den konstitutiven 35S-Promotor verwenden. Darüber hinaus können natürlich auch weitere Promotoren verwendet werden, die aus unterschiedlichen Quellen wie z. B. aus Pflanzen oder Pflanzenviren erhalten werden und für die Expression von Genen in Pflanzen geeignet sind. Die Auswahl des Promotors wie auch anderer regulatorischer Sequenzen bestimmen dabei das räumliche und zeitliche Expressionsmuster und damit auch das Silencing der pflanzlichen DnaJ-ähnlichen Proteine in transgenen Pflanzen.

Neben weiteren konstitutiven Promotoren wie beispielsweise dem Actin Promotor (McElroy et al., 1990, Plant Cell, 2:163) und dem Ubiquitin Promotor (Binet et al., 1991, Plant Science, 79:87) kommen als gewebespezifische Promotoren die Promotoren der Phosphoenolpyruvat-Carboxylase aus Mais (Hudspeth et al., 1989, Plant Mol. Biol., 12:579) oder der Fructose-1,6-bisphosphatase aus Kartoffel (WO 98/18940), die blattspezifische Expression vermitteln, in Frage. Es können auch Verwundungs-, Licht- oder Pathogen-induzierte sowie andere entwicklungsabhängige Promotoren oder Kontrollsequenzen verwendet werden (Xu et al., 1993, Plant Mol. Biol. 22:573; Logemann et al., 1989, Plant Cell, 1:151; Stockhaus et al., 1989, Plant Cell, 1:805; Puente et al., 1996, EMBO J., 15:3732; Gough et al., 1995, Mol. Gen. Genet., 247:323). Eine Zusammenfassung von verwendbaren Kontrollsequenzen findet sich z. B. in Zuo et al., 2000, Curr. Opin. Biotech., 11:146.

Geeignete Promotoren umfassen auch Promotoren, die eine Expression lediglich in photosynthetisch aktiven Geweben garantieren, wie z. B. der ST-LS1-Promotor (Stockhaus *et al.* (1987) Proc. Natl. Acad. Sci. USA 84:7943-7947; Stockhaus *et al.* (1989) EMBO J. 8:2445-2451). Ebenfalls verwendet werden können Promotoren, die während der Pflanzentransformation, der Pflanzenregeneration oder bestimmten Stadien dieser Prozesse aktiv sind, wie z. B. zellteilungsspezifische Promotoren wie der Histon H3-Promotor (Kapros *et al.* (1993) In Vitro Cell Cev. Biol. Plant 29:27-32) oder das chemisch induzierbare Tet-Repressor-System (Gatz *et al.* (1991) Mol. Gen. Genet. 227:229-237). Weitere geeignete Promotoren können der Literatur, z. B. Ward (1993, Plant Mol. Biol. 22:361-366), entnommen werden. Gleiches gilt für induzierbare und zell- bzw. gewebespezifische Promotoren, wie Meristemspezifische Promotoren, die ebenfalls in der Literatur beschrieben worden sind und im Rahmen der Erfindung ebenfalls geeignet sind.

Weitere induzierbare Promotoren umfassen virusinduzierbare Promotoren wie den ACMV virion-sense Promotor (Hong et al., 1996, Virology, 220:119-227), der durch das Genprodukt AC2 induziert wird. Darüber hinaus sind alle Promotoren solcher Proteine geeignet, die in Virus-befallenem Gewebe induziert werden, wie z. B. Phenylalänin Ammonium Lyase, Chalcone Synthase, Hydroxyprolin-reiches Glykoprotein, Extensin, Pathogenese-verwandte Proteine (z. B. PR-1a) und Wund-induzierbare Protease-Inhibitoren (US 6,013,864).

Darüber hinaus ist der Durchschnittsfachmann in der Lage, mittels Routinemethoden weitere geeignete Promotoren zu isolieren. So kann der Fachmann mit Hilfe gängiger molekularbiologischer Methoden, z. B. Hybridisierungsexperimenten oder DNA-Protein-Bindungsstudien, Speicherorgan-spezifische regulatorische Nukleinsäurelemente identifizieren. Dabei wird z. B. in einem ersten Schritt aus Speicherorgangewebe des gewünschten Organismus, aus dem die regulatorischen Sequenzen isoliert werden sollen, die gesamte poly(A)⁺-RNA isoliert und eine cDNA-Bank angelegt. In einem zweiten Schritt werden mit Hilfe von cDNA-Klonen, die auf pcly(A)⁺-RNA-Molekülen aus einem Nicht-Speicherorgangewebe basieren, aus der ersten Bank mittels Hybridisierung diejenigen Klone identifiziert, deren korrespondierende poly(A)⁺-RNA-Moleküle lediglich im Gewebe des Speicherorgans akkumulieren. Anschließend werden mit Hilfe dieser so identifizierten cDNAs Promotoren isoliert, die über Speicherorgan-spezifische regulatorische Elemente verfügen. Dem Fachmann stehen darüber hinaus weitere auf PCR basierende Methoden für die Isolierung geeigneter Speicherorgan-spezifischer Promotoren zur Verfügung.

In einer weiteren Ausführungsform handelt es sich um den Promotor des Klasse I Patatin-Gens B33 aus Kartoffel. Weiterhin bevorzugte Promotoren sind solche, die insbesondere in Früchten aktiv sind. Hierzu zählen beispielsweise der Promotor eines Polygalacturonase-Gens, z. B. aus Tomate, der während der Reife von Tomatenfrüchten Expression vermittelt (Nicholass *et al.* (1995) Plant Mol. Biol. 28:423-435; dieser Stand der Technik beschreibt die Analyse von Promotor/GUS-Fusionskonstrukten), der Promotor einer ACC-Oxidase, z. B. aus Apfel, der in transgenen Tomaten Reife- und Fruchtspezifität vermittelt (Atkinson *et al.* (1998) Plant Mol. Biol. 38:449-460; dieser Stand der Technik offenbart ebenfalls Promotor/GUS-Expressionsanalysen), oder der 2A11-Promotor aus Tomate (van Haaren *et al.* (1991) Plant Mol. Biol. 17:615-630, beschreibt ebenfalls Promotor/GUS-Fusionen).

Auch im Fall von Frucht-spezifischen Promotoren kann der Fachmann weitere geeignete Promotoren der Literatur entnehmen oder, wie oben für Speicherorgan-spezifische Promotoren beschrieben, mittels Routineverfahren isolieren.

Der Fachmann weiß, dass die Verwendung von induzierbaren Promotoren die Herstellung von Pflanzen und Pflanzenzellen erlaubt, die die erfindungsgemäßen Sequenzen nur transient exprimieren und damit transient silencen. Eine solche transiente Epxression erlaubt die Herstellung von Pflanzen, die nur eine transiente Virusresistenz zeigen. Solch eine transiente Resistenz kann z. B. dann wünschenswert sein, wenn die Gefahr einer Viruskontamination droht und die Pflanzen deswegen nur für einen bestimmten Zeitraum resistent gegen den Virus sein müssen. Weitere Situationen, in denen eine transiente Resistenz wünschenswert ist, sind dem Fachmann bekannt. Dem Fachmann ist darüber hinaus auch bekannt, dass er durch die Verwendung nicht stabil in Pflanzenzellen replizierender Vektoren, die die entsprechenden Sequenzen zum Silencing pflanzlicher DnaJ-ähnlicher Proteine tragen, eine transiente Epxression und damit ein transientes Silencing und eine transiente Resistenz erzielen kann.

Die erfindungsgemäßen Vektoren können als regulatorische Elemente auch noch z. B. Enhancer-Elemente umfassen, ferner können sie Resistenzgene, Replikationssignale und weitere DNA-Bereiche enthalten, die eine Propagation der Vektoren in Bakterien wie z. B. *E.coli* ermöglichen. Die regulatorischen Elemente umfassen auch Sequenzen, die eine Stabilisierung der Vektoren in den Wirtszellen bewirken. Insbesondere umfassen solche regulatorischen Elemente Sequenzen, die eine stabile Integration des Vektors in das Wirtsgenom der Pflanze oder eine autonome Replikation des Vektors in den Pflanzenzellen ermöglichen. Solche regulatorischen Elemente sind dem Fachmann bekannt.

Bei den so genannten Terminationssequenzen handelt es sich um Sequenzen, die sicherstellen, dass die Transkription bzw. die Translation ordnungsgemäß beendet wird. Sollen die übertragenen Nukleinsäuren translatiert werden, handelt es sich bei ihnen typischerweise um Stopcodons und entsprechende regulatorische Sequenzen; sollen die übertragenen Nukleinsäuren nur transkribiert werden, handelt es sich in der Regel um poly-A-Sequenzen.

Erfindungsgemäß sind mit Vektoren Plasmide, Cosmide, Viren und andere in der Gentechnik gängige Vektoren gemeint, mit denen Nukleinsäuremoleküle auf Pflanzen bzw. Pflanzenzellen übertragen werden können.

Zur Vorbereitung der Einführung fremder Gene in höhere Pflanzen bzw. deren Zellen stehen eine große Anzahl von Klonierungsvektoren zur Verfügung, die ein Replikationssignal für *E. coli* und ein Markergen zur Selektion transformierter Bakterienzellen enthalten. Beispiele für derartige Vektoren sind pBR322, pUC-Serien, M13mp-Serien, pACYC184 usw. Die gewünschte Sequenz kann an einer passenden Restriktionsschnittstelle in den Vektor eingeführt werden. Das erhaltende Plasmid wird für die Transformation von *E. coli*-Zellen verwendet. Transformierte *E. coli-*Zellen werden in einem geeigneten Medium gezüchtet und anschließend geerntet und lysiert. Das Plasmid wird wiedergewonnen. Als Analysemethode zur Charakterisierung der gewonnenen Plasmid-DNA werden im Allgemeinen Restriktionsanalysen, Gelelektrophoresen und weitere biochemisch-molekularbiologische Methoden eingesetzt. Nach jeder Manipulation können die Plasmid-DNA gespalten und gewonnene DNA-Fragmente mit anderen DNA-Sequenzen verknüpft werden. Jede Plasmid-DNA-Sequenz kann in den gleichen oder anderen Plasmiden kloniert werden. Klonierungsstandard-verfahren können Sambrook et al., 2001 (Molecular cloning: A laboratory manual, 3rd edition, Cold Spring Harbor Laboratory Press) entnommen werden.

Für die Einführung von DNA in eine pflanzliche Wirtszelle stehen eine Vielzahl bekannter Techniken zur Verfügung, wobei der Fachmann die jeweils geeignete Methode ohne Schwierigkeiten ermitteln kann. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, den direkten Gentransfer isolierter DNA in Protoplasten, die Elektroporation von DNA, die Einbringung von DNA mittels der biolistischen Methode sowie weitere Möglichkeiten. Dabei können sowohl stabile als auch transiente Transformanten erzeugt werden.

Bei der Injektion und Elektroporation von DNA in Pflanzenzellen werden per se keine speziellen Anforderungen an die verwendeten Plasmide gestellt. Ähnliches gilt für den direkten Gentransfer. Es können einfache Plasmide wie beispielsweise pUC-Derivate verwendet werden. Sollen aber aus derartig transformierten Zellen ganze Pflanzen regeneriert werden, ist die Anwesenheit eines selektierbaren Markergens notwendig. Dem Fachmann sind die gängigen Selektionsmarker bekannt und es stellt für ihn kein Problem dar, einen geeigneten Marker auszuwählen. Gebräuchliche Seklektionsmarker sind solche, die den transformierten Pflanzenzellen Resistenz gegenüber einem Biozid oder einem Antibiotikum wie Kanamycin, G418, Bleomycin, Hygromycin, Methotrexat, Glyphosat, Streptomycin, Sulfonyl-Harnstoff, Gentamycin oder Phosphinotricin und dergleichen vermitteln.

Je nach Einführungsmethode gewünschter Gene in die Pflanzenzelle können weitere DNA-Sequenzen erforderlich sein. Werden beispielsweise für die Transformation der Pflanzenzelle das Ti- oder Ri-Plasmid verwendet, so muss mindestens die rechte Begrenzung, häufig jedoch die rechte und linke Begrenzung der im Ti- und Ri-Plasmid enthaltenen T-DNA als Flankenbereich mit den einzuführenden Genen verbunden werden.

Werden für die Transformationen Agrobakterien verwendet, muss die einzuführende DNA in spezielle Plasmide kloniert werden, und zwar entweder in einen intermediären oder in einem binären Vektor. Die intermediären Vektoren können aufgrund von Sequenzen, die homolog zu Sequenzen in der T-DNA sind, durch homologe Rekombination in das Ti- oder Ri- Plasmid der Agrobakterien integriert werden. Dieses enthält außerdem die für den Transfer der T-DNA notwendige vir-Region. Intermediäre Vektoren können nicht in Agrobakterien replizieren. Mittels eines Helferplasmids kann der intermediäre Vektor auf *Agrobacterium tumefaciens* übertragen werden (Konjugation). Binäre Vektoren können sowohl in *E. coli* als auch in Agrobakterien replizieren. Sie enthalten ein Selektionsmarker-Gen und einen Linker oder Polylinker, welche von der rechten und linken T-DNA-Grenzregion eingerahmt werden. Sie können direkt in die Agrobakterien transformiert werden (Holsters et al. (1978), Molecular and General Genetics 163,181-187). Das als Wirtszelle dienende Agrobakterium soll ein Plasmid, das eine vir-Region trägt, enthalten. Die vir-Region ist für den Transfer der T-DNA in die Pflanzenzelle notwendig. Zusätzlich kann T-DNA vorhanden sein. Das derartig transformierte Agrobakterium wird zur Transformation von Pflanzenzellen verwendet.

Die Verwendung von T-DNA für die Transformation von Pflanzenzelle ist intensiv untersucht und ausreichend in EP 120 515 beschrieben worden.

Für den Transfer der DNA in die Pflanzenzelle können Pflanzen-Explantate zweckmäßigerweise mit *Agrobacterium tumefaciens* oder *Agrobacterium rhizogenes* kultiviert werden. Aus dem infizierten Pflanzenmaterial (beispielsweise Blattstücke, Stengelsegmente, Wurzeln, aber auch Protoplasten oder Suspensions-kultivierte Pflanzenzellen) können dann in einem geeignetem Medium, welches Antibiotika oder Biozide zur Selektion transformierter Zellen enthalten kann, wieder ganze Pflanzen regeneriert werden. Die Regeneration der Pflanzen erfolgt nach üblichen Regenerationsmethoden unter Verwendung bekannter Nährmedien. Die so erhaltenen Pflanzen bzw. Pflanzenzellen können dann auf Anwesenheit der eingeführten DNA untersucht werden.

Andere Möglichkeiten der Einführung fremder DNA unter Verwendung des biolistischen Verfahrens oder durch Protoplasten-Transformation sind dem Fachmann bekannt (vgl. L. Willmitzer (1993) Transgenic Plants in: Biotechnology, A Multi-Volume Comprehensive Treatise (Herausgeber: H.J. Rehm et al.), Band 2, 627-659, VCH Weinheim, Deutschland).

Während die Transformation dikotyler Pflanzen bzw. derer Zellen über Ti-Plasmid-Vektorsysteme mit Hilfe von *Agrobacterium tumefaciens* wohl etabliert ist, weisen neuere Arbeiten darauf hin, dass auch monokotyle Pflanzen bzw. deren Zellen der Transformation mittels auf Agrobakterien basierender Vektoren sehr wohl zugänglich sind (siehe u.a. Chan et al. (1993), Plant Mol. Biol. 22, 491-506).

Alternative Systeme zur Transformation von monokotylen Pflanzen bzw. deren Zellen sind die Transformation mittels des biolistischen Ansatzes (Wan und Lemaux (1994) Plant Physiol. 104, 37-48; Vasil et al. (1993) Bio/Technology 11, 1553-1558; Ritala et al. (1994) Plant Mol. Biol. 24, 317-325; Spencer et al. (1990), Theor. Appl. Genet. 79, 625-631), die Protoplasten-Transformation, die Elektroporation von partiell permeabilisierten Zellen sowie die Einbringung von DNA mittels Glasfasern.

Die transformierten Zellen wachsen innerhalb der Pflanze in der üblichen Weise (siehe auch McCormick et al. (1986), Plant Cell Reports 5, 81-84). Die resultierenden Pflanzen können normal angezogen werden und mit Pflanzen, die die gleiche transformierte Erbanlage oder andere Erbanlagen besitzen, gekreuzt werden. Die daraus entstehenden hybriden Individuen besitzen die entsprechenden phänotypischen Eigenschaften.

Es sollten zwei oder mehrere Generationen angezogen werden, um sicherzustellen, dass das phänotypische Merkmal stabil beibehalten und vererbt wird. Auch sollten Samen geerntet werden, um sicherzustellen, dass der entsprechende Phänotyp oder andere Eigenarten erhalten geblieben sind.

Ebenso können nach üblichen Methoden transgene Linien bestimmt werden, die für die neuen Nukleinsäuremoleküle homozygot sind und ihr phänotypisches Verhalten hinsichtlich einer vorhandenen bzw. nicht vorhandenen Pathogen-Responsivität untersucht und mit dem von hemizygoten Linien verglichen werden.

Selbstverständlich können Pflanzenzellen, die die erfindungsgemäßen Nukleinsäuremoleküle enthalten, auch als Pflanzenzellen (einschließlich Protoplasten, Kalli, Suspensionskulturen und dergleichen) weiterkultiviert werden.

Die oben dargestellten Vektoren können auf Pflanzenzellen auf verschiedene Weise übertragen werden. Ob die Vektoren in linearer oder zirkulärer Form vorliegen müssen, hängt von der jeweiligen Anwendung ab. Dem Fachmann ist bekannt, ob und wann er entsprechende linearisierte Vektoren verwenden kann oder nicht. Zum Beispiel weiß der Fachmann, dass zur Herstellung von spezifischen Knock-outs von Genen für DnaJ-ähnliche Proteine durch homologe Rekombination es ausreichen kann, die entsprechenden Vektoren zu linearisieren und in transgene Pflanzen zu injizieren.

Erfindungsgemäß umfasst der Begriff transgene Pflanze sowohl die Pflanze in ihrer Gesamtheit, als auch alle Pflanzenteile, in denen erfindungsgemäß die Expression von pflanzlichen DnaJ-ähnlichen Proteinen, die mit viralen Komponenten interagieren, supprimiert ist. Bei solchen Pflanzenteilen kann es sich um Pflanzenzellen handeln, um Pflanzensamen, um Blätter, um Blüten und um Pollen. Mit "transgener Pflanze" sind erfindungsgemäß auch das Vermehrungsmaterial von erfindungsgemäßen transgenen Pflanzen gemeint wie z. B. Samen, Früchte, Stecklinge, Knollen, Wurzelstücke etc., wobei dieses Vermehrungsmaterial ggf. oben beschriebene transgene Pflanzenzellen enthält, sowie Teile dieser Pflanzen wie Protoplasten, Pflanzenzellen und Kalli.

Bei der Herstellung von transgenen Pflanzen bieten sich verschiedene Methoden und Möglichkeiten an, wie bereits oben ausgeführt worden ist. Generell können Pflanzen bzw. Pflanzenzellen mit Hilfe herkömmlicher gentechnologischer Transformationsmethoden derart modifiziert werden, dass die neuen Nukleinsäuremoleküle in das pflanzliche Genom integriert werden, d.h. dass stabile Transformanten erzeugt werden und die überführten Nukleinsäuremoleküle mit dem pflanzlichen Genom repliziert werden. Je nach dem verwendeten Vektorsystem können erfindungsgemäß auch transgene Pflanzen hergestellt werden, bei denen die zu übertragenden Nukleinsäuren in der Pflanzenzelle bzw. der Pflanze als selbstständig replizierendes System enthalten sind. Die zur Übertragung der Pflanzen verwendeten Vektoren müssen dann entsprechend über DNA-Sequenzen verfügen, die die Replikation von zur Übertragung verwendeten Plasmiden innerhalb der Zelle ermöglichen.

Ein weiteres erfindungsgemäßes Verfahren zur Herstellung von transgenen Pflanzen mit erhöhter Virusresistenz sieht vor, dass in der Pflanze bzw. in den Pflanzenzellen dominant-negative Mutanten von pflanzlichen DnaJ-ähnlichen Proteine exprimiert werden. Durch Einführung von dominant-negativen Mutanten in die endogenen pflanzlichen DnaJ-ähnlichen Proteine können erfindungsgemäß auch nicht-transgene Pflanzen und Pflanzenzellen hergestellt werden, die über eine erhöhte Virusresistenz verfügen.

Unter "dominant-negativen Mutanten" werden erfindungsgemäß pflanzliche DnaJ-ähnliche Proteine verstanden, die über Mutationen und/oder Deletionen verfügen, die es verhindern, dass die pflanzlichen DnaJ-ähnlichen Proteine entweder mit den viralen Komponenten und/oder mit den anderen Komponenten des Chaperonssystems interagieren.

Wenn solche dominant-negativen Mutanten in der transgenen Zelle bzw. Pflanze exrpimiert bzw. überexprimiert werden, sind sie in der Lage, die Interaktion der viralen Komponenten mit den Wildtyp-DnaJ-ähnlichen Proteinen bzw. die Interaktion der Wildtyp-DnaJ-ähnlichen Proteine mit den anderen Faktoren des Chaperon-systems auszukompetitieren, so dass der Virus keine Möglichkeit zur Propagation hat. Überraschend bei diesem Verfahren ist, dass dadurch transgene Pflanzen hergestellt werden können, die über eine erhöhte Virusresistenz verfügen und gleichzeitig einen im Wesentlichen normalen Phänotyp aufweisen, obwohl solche dominant-negativen Mutanten das endogene Chaperonsystem der Pflanzenzelle negativ beeinflussen sollten.

Der Fachmann ist sich darüber bewusst, dass erfindungsgemäße virusresistente transgene Pflanzen und Pflanzenzellen nicht nur durch Expression bzw. Überexpression dominant-negativer Mutanten von pflanzlichen DnaJ-ähnlichen Proteinen hergestellt werden können, sondern selbstverständlich auch durch Expression bzw. Überexpression von dominant-negativen Mutanten von DnaJ-ähnlichen Proteinen aus anderen Organismen. Dabei kommen DnaJ-Proteine aus Prokaryoten wie *E. coli* oder deren Homologe und Orthologe aus Eukaryoten wie Hefen (z.B. *S. cerevisiae*), *C. elegans* oder höheren Säugetieren wie Maus, Ratte und Mensch in Frage. Voraussetzung ist, dass die Expression dieser dominant-negativen Mutanten eine Kompetition der endogenen pflanzlichen DnaJ-ähnlichen Proteine mit viralen Komponenten und/oder deren zellulären Interaktionspartnern bewirkt. Die DnaJ-ähnlichen Proteine aus anderen Organismen können durch die oben beschriebenen Datenbankanalysen und Homologie-Vergleiche identifiziert werden. Voraussetzung ist, dass sie über eine J-Domäne verfügen.

Solche transgenen Pflanzen, die dominant-negative Mutanten von DnaJ-ähnlichen Proteinen und bevorzugt pflanzlichen DnaJ-ähnlichen Proteinen exprimieren, können hergestellt werden, indem ein Vektor auf Pflanzenzellen übertragen wird, der folgende DNA-Sequenzen in 5'-3'-Orientierung enthält: Einen in Pflanzen funktionellen Promotor, operativ daran gebunden eine DNA-Sequenz, die die für DnaJ-ähnliche Proteine und bevorzugt für pflanzliche DnaJ-ähnliche Proteine oder Teile davon kodierende Sequenz umfasst, wobei diese Sequenz Mutationen oder Deletionen aufweisen, die die Interaktion von DnaJ-ähnlichen Proteinen mit viralen Komponenten oder anderen Faktoren des Chaperonsystems negativ beeinflussen, sowie ein Terminationssignal.

Bei den Promotoren und den Terminationssignalen kann es sich um die gleichen Promotoren und Signale handeln, die bereits oben erwähnt wurden. Dasselbe gilt selbstverständlich auch für alle weiteren regulatorischen Elemente sowie die Art der zu verwendenden Vektoren. Der Fachmann wird die entsprechenden regulatorischen Fragmente und den jeweiligen Vektor entsprechend den jeweiligen Bedürfnissen wählen.

Dominant-negative Mutationen und/oder Deletionen kann der Fachmann durch einfaches routiniertes Experimentieren identifizieren. Zum einen sind eine Reihe von Mutationen in der J-Domäne bekannt, die z. B. die Interaktion mit den DnaK-ähnlichen Proteinen und deren Homologen inhibieren. Dazu gehören insbesondere Mutationen innerhalb der drei Bereiche, die für die Helix I bis III kodieren, insbesondere das HPD Tripeptid (Kelley, 1999, Current Biology, 9,305-308).

Mutationen, die spezifisch die Interaktion der DnaJ-ähnlichen Proteine und bevorzugt der pflanzlichen DnaJ-ähnlichen Proteine mit den viralen Komponenten unterbinden, können durch Herstellen von rekombinanten DnaJ-ähnlichen Proteinen und bevorzugt pflanzlichen DnaJ-ähnlichen Proteinen, die unterschiedliche Mutationen und/oder Deletionen tragen, und Testen dieser rekombinanten Proteine in Bindungsassays mit viralen Komponenten in einfacher Weise ermittelt werden. In gleicher Weise können dominant-negative Mutanten von pflanzlichen DnaJ-ähnlichen Proteinen ermittelt werden, die nicht mehr in der Lage sind, mit den zellulären Bindungspartnern zu interagieren.

Auf gleiche Weise kann z.B. in *in vitro* Bindungstests getestet werden, ob dominant-negative Mutanten von DnaJ-ähnlichen Proteinen und bevorzugt von pflanzlichen DnaJ-ähnlichen Proteinen in der Lage sind, die Interaktion der pflanzlichen DnaJ-ähnlichen Wildtyp-Proteine mit den viralen Komponenten und/oder den zellulären Bindungspartnern zu kompetitieren.

Unter dominant-negativen Mutationen werden alle Mutationsarten verstanden, d.h. Insertionen, Deletionen und Punktmutationen, die in der Lage sind, die Interaktion von pflanzlichen DnaJ-ähnlichen Proteinen mit viralen Faktoren und/oder zellulären Bindungspartnern zu unterbinden.

Bei dem erfindungsgemäßen Verfahren zum Herstellen von transgenen Pflanzen mit erhöhter Virusresistenz durch Expression von dominant-negativen Mutanten von DnaJ-ähnlichen Proteinen findet eine Modulation des Levels von mit Viruskomponenten interagierenden DnaJ-ähnlichen Proteinen und kein Silencing der Wirtsfaktoren statt, was den zusätzlichen Vorteil bringt, dass dieser Mechanismus keinen direkten Angriffspunkt für Virus-kodierte Suppressoren, wie sie beim RNAi vermittelten Gene silencing auftreten können, bietet.

Zur Herstellung von transgenen Pflanzen mit erhöhter Virusresistenz gegen Potyviren können z. B. dominant-negative Mutanten der DnaJ-ähnlichen Proteine NtCPIP1 und NtCPIP2a, denen die ersten 65 bzw. 66 Aminosäuren fehlen (NtCPIP1Δ65N und NtCPIP2aΔ66N), stabil in Pflanzenzellen exprimiert werden. Diese dominant-negativen Mutanten von NtCPIP1 und NtCPIP2a verfügen nicht über die J-Domäne, können aber noch mit dem Capsid-Protein (CP) von PVY interagieren. Diese dominant-negativen Mutanten kompetitieren bei ihrer Expression mit den endogenen WT-DnaJ-ähnlichen Proteinen um die Bindung an das PVY-CP und hemmen so die Ausbreitung des Virus. Die Expression von rekombinanten Proteinen in Pflanzenzellen und die dazu verwendbaren Vektoren und Plasmide sind dem Fachmann bekannt. In analoger Weise können andere DnaJ-ähnliche Proteine wie NtCPIP2b und NtDnaJ_M541, deren N-Terminus deletiert ist, zur Herstellung von transgenen Pflanzen mit erhöhter Virusresistenz verwendet werden. Ebenso können als dominant-negative Mutanten z.B. die bereits oben erwähnten DnaJ-ähnlichen Proteine aus *A. thaliana* verwendet werden, bei denen die J-Domäne deletiert ist.

Dem Fachmann ist bekannt, wie Punktmutationen, Insertionsmutationen oder Deletionsmutationen in den für DnaJ-ähnliche Proteine kodierenden Nukleinsäuresequenzen eingeführt werden können. PCR-Techniken können z.B. zur Einführung von Punktmutationen bevorzugt sein ("PCR technology: Principle and Applications for DNA Amplification", H. Ehrlich, id., Stockton Press). Beispiele zur Einführung von Punktmutationen oder Deletionsmutationen in für Proteine kodierende DNA-Sequenzen finden sich in den Ausführungsbeispielen.

Transgene Pflanzen, die eine erhöhte Virusresistenz aufweisen, können erfindungsgemäß auch derart hergestellt werden, dass z. B. ein rekombinanter Antikörper, der spezifisch die Interaktion der pflanzlichen DnaJ-ähnlichen Proteine mit den viralen Komponenten oder den Faktoren des Chaperonsystems blockiert bzw. kompetitiert, in den Pflanzen exprimiert wird. Solche Antikörper können insbesondere gegen die J-Domäne gerichtet sein. Wie solche rekombinanten Antikörper gegen eine spezifische Domäne von pflanzlichen DnaJ-ähnlichen Proteinen isoliert und identifiziert werden können, ist dem Fachmann bekannt und kann der Literatur entnommen werden (Harlow et al., 1999, Using antibodies: a laboratory manual, Cold Spring Harbor Laboratory Press).

Unter rekombinanten Antikörpern werden erfindungsgemäß die bekannten unterschiedlichen Formen von rekombinanten Antikörpern verstanden, wie sie z.B. in Skerra et al. (Curr. Opin. Immunol. (1993) 2, 250 - 262) beschrieben wurden. Die erfindungsgemäßen rekombinanten Antikörper umfassen dabei die so genannten Fab-Fragmente, Fv-Fragmente, scFv-Antikörper, scFv-Homodimere, die über Disulfit-Brücken miteinander verknüpft sind und so genannte VH-Ketten. Die Fab-Fragmente bestehen aus assemblierten vollständigen leichten und verkürzten schweren Ketten, wohingegen Fv-Fragmente aus nicht-kovalent miteinander verknüpften VH- und VL-Ketten bestehen. Ein Überblick über die genannten Fragmente und rekombinanten-Antikörper findet sich in Conrad et al. (Plant Mol. Biol. (1998) 38, 101 - 109). Die genannten Fab- und Fv-Fragmente können *in vivo* miteinander assoziieren.

Da dieser Prozess unter Umständen jedoch nicht sehr effizient abläuft, werden erfindungsgemäß bevorzugt scFv-Antikörper eingesetzt. Diese bestehen aus dem variablen Anteil der leichten Kette und dem variablen Anteil der schweren Kette, die über ein flexibles Linkerpeptid miteinander fusioniert sind. Die Herstellung solcher scFv-Antikörper ist im Stand der Technik intensiv beschrieben worden (siehe u.a. Conrad et al., *vide supra*; Breitling et al. (1999) Recombinant Antibodies, John Wiley.& Sons, New York). Die scFv-Antikörper weisen die gleiche Antigen-Spezifität und Aktivität wie normale Antikörper auf, müssen aber nicht wie andere natürliche oder rekombinante Antikörper *in vivo* aus Einzelketten assembliert werden. Sie eignen sich deshalb insbesondere für die erfindungsgemäßen Verfahren.

In den oben angegebenen Referenzen wird ausführlich dargestellt, wie Nukleinsäuresequenzen, die für die erfindungsgemäß bevorzugten scFv-Antikörper kodieren, durch den Fachmann isoliert und hergestellt werden können.

Üblicherweise wird dabei von bestehenden Hybridoma-Zelllinien ausgegangen, die monoklonale Antikörper produzieren. Danach werden die für die leichte und schwere Ketten des Antikörpers kodierenden cDNAs isoliert und in einem zweiten Schritt die kodierenden Regionen für die variable Region der leichten und der schweren Kette in einem Molekül miteinander fusioniert.

Ein weiterer, dem Fachmann bekannter Weg zur Erzeugung rekombinanter Antikörper ist das screening von Bibliotheken rekombinanter Antikörper (sogenannte "phage display libraries", siehe auch Hoogenboom et al. (2000) Immunology Today 21, 371-378; Winter et al. (1994) Annu. Rev. Immunol. 12, 433-455; De Wildt et al. (2000) Nat. Biotechnol. 18, 989-994). Durch dem Fachmann bekannte Vorgehens-weisen können bei diesem Verfahren rekombinante Antikörper gegen ein gegebenes Antigen angereichert, selektiert und isoliert werden.

Darüber hinaus können erfindungsgemäß durch Expression von Inhibitoren, die spezifisch die Interaktion von DnaJ-ähnlichen Proteinen mit viralen Komponenten oder anderen Faktoren des zellulären Chaperonsystems inhibieren, transgene Pflanzen hergestellt werden, die eine erhöhte Virusresistenz aufweisen. Bei solchen Inhibitoren kann es sich z. B. um Peptide handeln, die in die jeweiligen Bindungstaschen der DnaJ-ähnlichen Proteine für die Interaktion mit den viralen Komponenten oder anderen Faktoren des Chaperonsystems binden.

Eine weitere Möglichkeit zur Herstellung von transgenen Pflanzen mit erhöhter Virusresistenz besteht darin, nur die J-Domäne zu überexprimieren. Dadurch kann die Interaktion von viralen Komponenten mit pflanzlichen DnaJ-ähnlichen Proteinen auskompetiert werden. Typischerweise hat die überexprimierte J-Domäne die SEQ ID NO:7 oder ist dazu homolog. Durch die Überexpression der J-Domäne wird die Interaktion der endogenen pflanzlichen DnaJ-ähnlichen Proteine, die mit viralen Komponenten interagieren, mit deren zellulären Bindungspartnern kompetitiert, so dass die Propagation des Virus gehemmt wird.

Im Folgenden sind Beispiele aufgeführt, wie pflanzliche DnaJ-ähnliche Proteine, die mit viralen Komponenten interagieren, identifiziert werden können, wie die Bindungsspezifität dieser pflanzlichen DnaJ-ähnlichen Proteine bestimmt werden kann und wie durch Silencing der pflanzlichen DnaJ-ähnlichen Proteine transgene Pflanzen mit einer erhöhten Virusresistenz hergestellt werden können. Diese Beispiele sind nicht ausschließend zu deuten.

Ein weiterer Gegenstand der vorliegenden Erfindung betrifft Pflanzenzellen und Pflanzen, in denen die endogenen Gene von pflanzlichen DnaJ-ähnlichen Proteinen Mutationen, d.h. Substitutionen, Insertionen und/oder Deletionen aufweisen, die dazu führen dass die exprimierten endogenen DnaJ-ähnlichen Proteine nicht mehr oder nur bedingt in der Lage sind, mit viralen Faktoren und/oder ihren endogenen zellulären Bindungspartnern zu interagieren. Pflanzen bzw. Pflanzenzellen, die solche Mutationen-aufweisenden endogenen Genkopien für DnaJ-ähnliche Proteine besitzen, werden wie die oben beschriebenen transgenen Pflanzen und Pflanzenzellen sich durch eine erhöhte transiente oder permanente Virusresistenz gegenüber den oben genannten Virusgruppen und Stämmen auszeichnen. Solche Pflanzen und Pflanzenzellen, die im Gegensatz zu den oben genannten Pflanzen und Pflanzenzellen nicht transgen sind, können durch klassische Mutagenese hergestellt werden.

Erfindungsgemäß müssen solche nicht-transgenen Pflanzen oder Pflanzenzellen aber in den für pflanzliche DnaJ-ähnliche Proteine kodierenden Genen die oben genannten Mutationsarten aufweisen, die zu einer Modulation der Expression der pflanzlichen DnaJ-ähnlichen Proteine und/oder des Bindungsverhaltens der pflanzlichen DnaJ-ähnlichen Proteine führen. Modulation der Expression der endogenen pflanzlichen DnaJ-ähnlichen Proteine kann z.B. bedeuten, dass durch Mutationen in regulatorischen DNA-Elementen der Gene der pflanzlichen DnaJ-ähnlichen Proteine, wie z.B. Promotor, Enhancern oder allgemein so genannten "upstream activating sequences", die Expression der endogenen pflanzlichen DnaJ-ähnlichen Proteine herunterreguliert wird.

Die Modulation des Bindungsverhaltens der pflanzlichen DnaJ-ähnlichen Proteine bedeutet im Rahmen der vorliegenden Erfindung, dass die oben genannten Mutationsarten zu einer Veränderung des Bindungsverhaltens der endogenen pflanzlichen DnaJ-ähnlichen Proteine gegenüber den viralen Faktoren und/oder den normalen zellulären Bindungspartnern führen. Bevorzugt ist eine Modulation des Bindungsverhaltens der DnaJ-ähnlichen Proteine, die dazu führt, dass diese nicht mehr oder nur bedingt mit viralen Faktoren und/oder ihren zellulären Partnern interagieren. Auch eine Kombination der Modulation der Expression und des Bindungsverhalten der pflanzlichen DnaJ-ähnlichen Proteine ist denkbar.

Zum Beispiel können Pflanzen oder Pflanzenzellen Mutationen in den Gensequenzen für DnaJ-ähnliche Proteine aufweisen, die zur Reduktion der Expression dieser Proteine führen. Andere Pflanzen oder Pflanzenzellen weisen Mutationen auf, die zu den oben beschriebenen dominant-negativen Mutanten führen. In beiden Fällen werden Pflanzen mit einer erhöhten Virusresistenz erhalten.

Der Fachmann ist sich bewusst, dass durch Mutagenese z.B. auch Pflanzen bzw. Pflanzenzellen hergestellt werden können, die aufgrund von Mutationen in Enhancer- und/oder Promotor-Sequenzen der Gene für pflanzliche DnaJ-ähnliche Proteine eine Reduktion der Expression dieser Proteine aufweisen und gleichzeitig Mutationen in den kodierenden Bereichen der für pflanzliche DnaJ-ähnliche Proteine kodierenden Gene aufweisen, die bewirken, dass die verbleibenden exprimierten pflanzlichen DnaJ-ähnlichen Proteine nicht mehr oder nur begrenzt mit den viralen und/oder anderen zellulären Bindungspartnern interagieren können. Umgekehrt können entsprechende Mutationen in Enhancer- und/oder Promotor-Sequenzen und in den kodierenden Sequenzen bewirken, dass eine oben dargestellte dominant-negative Mutante von pflanzlichen DnaJ-ähnlichen Proteinen, die nicht mehr oder nur sehr begrenzt in der Lage ist, mit viralen und/oder normalen zellulären Interaktionspartnem wechselzuwirken, überexprimiert wird und es somit zur oben beschriebenen Kompetitionsreaktion kommt.

Diese Pflanzen zeichnen sich durch eine erhöhte transiente oder permanente Virusresistenz gegenüber den oben genannten Virusklassen, -gruppen und -stämmen aus.

Bevorzugt können die erfindungsgemäßen nicht-transgenen Pflanzen und Pflanzenzellen, die sich durch eine Modulation der Expression und/oder des Bindungsverhaltens der endogenen pflanzlichen DnaJ-ähnlichen Proteine auszeichnen und eine permanente oder transiente Virusresistenz besitzen, durch den so genannten "TILLING"-Ansatz (Targeting Induced Local Lesion in Genoms) hergestellt werden. Dieses Verfahren ist im Detail in Colbert et al. (2001, Plant Physiology, 126, 480 - 484), McCallum et al. (2000, Nat. Biotechnol., 18, 455 - 457) und McCallum et al. (2000, Plant Physiology, 123, 439 - 442) beschrieben worden. Die vorgenannten Referenzen werden hier explizit als Offenbarung hinsichtlich der "TILLING"-Methode eingeführt.

Das TILLING-Verfahren ist eine Strategie der so genannten reversen Genetik, das die Produktion hoher Dichten von Punktmutationen in mutagenisierten Pflanzenkollektionen, z.B. durch chemische Mutagenese mit Ethylmethansulphonat (EMS), mit der schnellen systematischen Identifizierung von Mutationen in Zielsequenzen kombiniert. Zunächst wird die Zielsequenz über PCR in DNA-Pools mutagenisierter M2-Populationen amplifiziert. Denaturierungs- und Annealingsreaktionen der heteroallelischen PCR-Produkte erlauben die Ausbildung von Heteroduplexen, bei denen ein DNA-Strang von dem mutierten und der andere vom "Wildtyp" PCR-Produkt stammt. An der Stelle der Punktmutation erfolgt ein sogenannter Mismatch, der entweder über denaturierende HPLC (DHPLC, McCallum et al., 2000, Plant Physiol., 123, 439-442) oder mit dem *Cel*I Mismatch-Detektionssystem (Oleykowsky *et al.,* 1998, Nucl. Acids Res. 26, 4597-4602) identifiziert werden kann. *Cel*I ist eine Endonuklease, die Mismatche in Heteroduplex-DNA erkennt und spezifisch an diesen Stellen die DNA spaltet. Die Spaltungsprodukte können dann über automatisierte Sequenzierungs-Gelelektrophorese aufgetrennt und detektiert werden (Colbert et al., 2001, vide supra). Nach Identifizierung von Zielgen-spezifischen Mutationen in einem Pool werden individuelle DNA-Proben entsprechend analysiert, um die Pflanze mit der Mutation zu isolieren. Auf diese Weise wird bei den erfindungsgemäßen pflanzen und Pflanzenzellen nach der Herstellung der mutagenisierten Pfanzenpopulationen durch Verwendung gegen DnaJ-ähnliche Proteine gerichtete Primersequenzen die Identifizierung der mutagenisierten Pflanzenzellen bzw. Pflanzen durchgeführt. Das TILLING-Verfahren ist generell für alle Pflanzen anwendbar und daher sind die oben genannten Kultur- und Nutzpflanzen für das erfindungsgemäße Verfahren geeignet.

### Beispiel 1: Identifizierung von pflanzlichen DnaJ-ähnlichen Proteinen, die mit viralen Komponenten interagieren.

Im folgenden Beispiel ist dargestellt, wie pflanzliche DnaJ-ähnliche Proteine aus Nicotiana tabacum durch einen Two-Hybrid Screen identifiziert wurden, die mit dem Capsid-Protein (CP) von Potato virus Y (PVY) und Tobacco Etch Virus (TEV) interagieren. Der übersichtlicheren Darstellung wegen werden zunächst die im Rahmen dieses Experiments verwendeten Methoden und Materialien abgehandelt.

### Allgeineine Klonierungsverfahren

Klonierungsverfahren wie z. B.: Restriktionsspaltungen, DNA-Isolierung, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von *E. coli* Zellen, Anzucht von Bakterien, Sequenzanalyse rekombinanter DNA wurden nach Sambrook et al. (vide supra.) beschrieben durchgeführt. Die Transformation von *Agrobacterium tumefaciens* wurde entsprechend der Methode von Höfgen und Willmitzer (Nucl. Acid Res. (1988) 16, 9877) ausgeführt. Die Anzucht der Agrobakterien erfolgte in YEB Medium (Vervliet et al. Gen. Virol. (1975) 26, 33). Arbeiten mit Hefe erfolgten nach den Protokollen des Yeast Protocols Handbook (1999, Clontech Laboratories, Inc., Palo Alto, CA, USA).

### Bakterien-/Hefestämme und Plasmide

*E. coli* (XL-1 Blue, XL-MRF' und XLOLR) Bakterien wurden durch die Firma Stratagene und Clontech (KC8) bezogen. Der bei den Arbeiten mit dem Hefe-Zwei-Hybrid System verwendete Hefestamm Y190 wurde von Harper et al. (1993, Cell 75: 805) eingeführt. Der zur Pflanzentransformation eingesetzte Agrobakterium Stamm (C58C1 mit dem Plasmid pGV 3850kan) wurde von Deblaire et al. (1985, Nucl. Acid Res. 13: 4777) beschrieben.
Zur Klonierung wurden die Vektoren pGEM-T (Promega), pCR-Blunt (Invitrogen), pBinAR (Höfgen und Willmitzer 1990, Plant Sci, 66: 221), sowie der unten detailliert beschriebene Vektor pUC-RNAi benutzt. Für die Arbeiten mit dem Hefe-Zwei-Hybrid System wurden die Vektoren pGBT9, pGBKT7 (Clontech) und pAD-GAL4 (Stratagene) verwendet.

### Tabaktransformation

Zur Transformation von Tabakpflanzen (*Nicotiana tabacum* L. cv. Samsun NN) wurden 10 ml einer unter Selektion gewachsenen Übernachtkultur von *Agrobacterium tumefaciens* abzentrifugiert, der Überstand verworfen, und die Bakterien im gleichen Volumen Antibiotika-freien Mediums resuspendiert. In einer sterilen Petrischale wurden Blattscheiben steriler Pflanzen (Durchmesser ca. 1cm) in dieser Bakterienlösung gebadet. Anschließend wurden die Blattscheiben in Petrischalen auf MS-Medium (Murashige und Skoog, Physiol. Plant. (1962) 15, 473) mit 2% Saccharose und 0.8% Bacto-Agar ausgelegt. Nach 2-tägiger Inkubation im Dunkeln bei 25°C wurden sie auf MS-Medium mit 100mg/l Kanamycin, 500mg/l Claforan, 1mg/l Benzylaminopurin (BAP), 0.2mg/l Naphtylessigsäure (NAA), 1.6% Glukose und 0.8% Bacto-Agar übertragen und die Kultivierung (16 Stunden Licht / 8 Stunden Dunkelheit) fortgesetzt. Wachsende Sprosse wurden auf hormonfreies MS-Medium mit 2% Saccharose, 250mg/l Claforan und 0.8% Bacto-Agar überführt.

### Isolierung einer Potato virus Y (PVY) Hüllprotein (Capsid Protein, CP) kodierenden cDNA aus virusinfiziertem Blattmaterial

Die Klonierung einer PVY Capsid Protein kodierenden cDNA erfolgte mittels RT-PCR (Reverse Transcription Polymerase Chain Reaction) von RNA aus virusinfiziertem Blattmaterial. Dazu wurden Blätter von *Nicotiana tabacum* L. Varietät Samsun NN mit einem PVY (N-Stamm, PVY^{N}) Feldisolat (Bundesanstalt für Züchtungsforschung an Kulturpflanzen Aschersleben) nach der in Herbers et al. (1996, Plant Cell, 8, 793) beschriebenen Methode infiziert. Gesamt-RNA wurde aus dem Blattmaterial nach Logemann et al. (1987, Anal. Biochem. 163: 16) isoliert. Die Amplifizierung erfolgte unter Verwendung der spezifischen PCR-Primer D44 (5'-ATGAATTCGCAAATGACACAATTGATGC-3') und D45 (5'-ATGTCGACCATGTTCTTGACTCCAAGTAG-3'), die von einer veröffentlichten PVY^{N}-Sequenz (GenBank Akzession D00441) abgeleitet und dabei mit *Eco*RI-(D44) sowie *Sal*I-Schnittstellen (D45) versehen wurden (unterstrichen). 5'Primer D44 umfasst die Basen 8573-8592 und 3'Primer D45 die Basen 9353-9573.

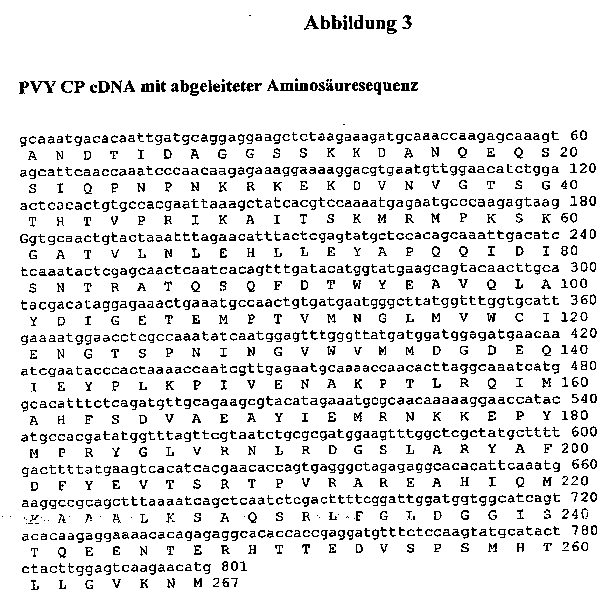

Die RT-PCR erfolgte in einem gekoppelten Reverse-Transkription/cDNA-Amplifikations-Assay nach dem Protokoll der Tth DNA Polymerase (Biomaster). Der Reaktionsansatz (20 µl) für die Reverse Transkription enthielt 0,1 µg Gesamt-RNA als Matrizenmaterial, 3,2 µl Primer D45 (5 µM), 2 µl Tth-Polymerase 10x Reaktionspuffer, 0,4 µl dNTPs (10 mM dATP, dCTP, dGTP, dTTP), 2 µl MnCl₂ (10 mM) sowie 5U Tth DNA Polymerase. Der Ansatz wurde 5 min bei 60°C, dann 10 min bei 70°C inkubiert, bevor zur cDNA-Amplifikation Chelating 5x Reaktionspuffer, 3,2 µl Primer D44 (5 µM), und 3 µl MgCl₂ (50 mM) zum Reaktionsansatz (Endvolumen 100 µl) zugegeben wurde. Die PCR-Amplifikation wurde in einem GeneAmp PCR-System (Perkin Elmer) mit folgendem Programm durchgeführt: 1 Zyklus 95°C 2 min, 60°C 1min; 35 Zyklen 95°C 1min, 60°C 1 min; 1 Zyklus 95°C 1min, 60°C 7 min. Das erhaltene cDNA-Fragment wurde in den Vektor pGEM-T (Promega) kloniert und die Identität der amplifizierten PVY CP cDNA mittels Sequenzanalyse verifiziert (Abb. 3).

### Identifizierung von PVY CP interagierenden Proteinen

PVY CP interagierende Proteine wurden mittels eines Hefe-Zwei-Hybrid Ansatzes nach konventionellen Techniken wie in MacDonald (Hrsg) et al. (2001, Two-Hybrid Systems: Methods and Protocols, Methods in Molecular Biology, Vol. 177, Totowa; N.J., USA) beschrieben identifiziert. Die Erstellung der Hefe-Zwei-Hybrid cDNA Bank erfolgte nach Protokoll und unter Verwendung der Materialien des "HybriZAP Two-Hybrid cDNA Gigapack Cloning Kit" (Stratagene) und umfasste die folgenden Schritte: Gesamt-RNA wurde aus "Source"-Blättern von *Nicotiana tabacum* L Varietät Samsun NN nach der Methode von Logemann et al. (1987, vide supra) isoliert. Aus 3,7 mg Blatt-RNA wurde unter Verwendung von Oligo dT Cellulose Poly(A)⁺ RNA nach der Methode von Sambrook et al. (vide supra) gewonnen.

5 µg Poly(A)⁺ RNA wurden mittels Reverser Transkriptase in Erststrang-cDNA umgeschrieben, die als Matrize für die Zweitstrang-Synthese durch die DNA-Polymerase diente. Nach Auffüllen überstehender DNA-Enden wurden *Eco*RI-Adapter angefügt und diese mittels einer T4-Polynukleotid-Kinase phosphoryliert. Nach *Xho*I-Verdau der Adapter wurde die geschnittene cDNA-Population mittels einer Sepharose CL-2b Säule größenfraktioniert und in einen *Eco*RI/*Xho*I geöffneten HybriZAP Vektor ligiert. Der Ligationsansatz wurde mit Hilfe des "Gigapack III Gold Packaging Extract" in Lambda-Phagen verpackt und nach einem Massen-*in vivo*-Excisions- und Amplifikationsschritt die primäre HybriZAP cDNA-Bank in eine pAD-GAL4 Phagemid cDNA-Bank konvertiert.

Die PVY CP kodierende cDNA wurde als *Eco*RI/*Sal*I-Fragment in offenem Leserahmen mit der GAL4-Bindungsdomäne in den Vektor pGBT9 (Clontech) kloniert. Dieses "Bait"-Plasmid Konstrukt wurde zunächst in den Hefe-Reporterstamm Y190 (vide supra) transferiert, und in diesen die cDNA-Bank Plasmide nach der in Schiestl und Gietz (1989, Curr. Genet. 156, 339) beschriebenen PEG/LiAc/ssDNA-Methode transformiert. Zur Selektion interagierender Plasmide wurden die Hefezellen auf Minimalmedium ohne die Aminosäuren Tryptophan, Leucin, Histidin (SD Trp⁻/Leu⁻/His⁻) und mit 25 mM 3-Amino-1,2,4-triazol (3-AT; Gietz et al. 1995, Yeast 11: 355; Bartel und Field 1995, Methods in Enzymology 254: 241) ausplattiert.

Die Transformationseffizienz wurde durch Ausplattieren von Aliquots auf SD Trp⁻/Leu⁻Platten mit 8×10⁷ durchmusterten Transformanden bestimmt. Nach 7-10 Tagen Inkubation bei 30°C wurden 33 Hefekolonien mit deutlichem Wachstum (mind. 2 mm im Durchmesser) isoliert und auf Expression des *lac*Z Reportergens nach der von Breeden und Naysmyth (1989, Cold Spring Harbor Symposium Quant. Biol. 50: 643) beschriebenen Beta-Galaktosidase Filterlift-Assay-Methode getestet. 4 Hefekolonien, die sowohl Wachstum in Abwesenheit von Histidin als auch *lacZ-*Aktivität zeigten, wurden zur weiteren Charakterisierung ausgewählt. Gesamt-DNA wurde aus den Hefezellen isoliert und nach Elektroporation in den *E. coli*-Stamm KC8 (Clontech) auf Anwesenheit der pAD-GAL4-cDNA Plasmide selektioniert.

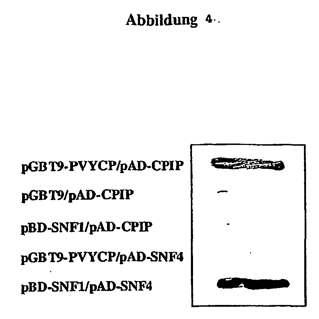

Die Spezifität der Interaktion wurde über Kotransformation der Aktivierungsdomänene Plasmide bzw. des Bait-Plasmids mit den unverwandten Kontrollplasmiden pBD-p53, pBD-SNF1 und pAD-SNF1 (Stratagene) verifziert (Abb. 4). Die isolierten Aktivierungsdomänen Plasmide zeigten spezifische Interaktion mit dem PVY CP-Konstrukt und nicht mit den Kontrollen pBD-p53 und pBD-SNF1, so dass die Aktivierungsdomänen fusionierten cDNAs sequenziert wurden. Die vier Klone (benannt als Capsid protein interacting protein, *CPIP7, 13, 19, 29*) waren identisch zueinander, wobei *CPIP29* für zusätzliche 25 Aminosäuren am N-Terminus kodierte. Die gesamte cDNA wurde nach Durchmusterung einer λ ZAP II cDNA Bank aus Tabak Blattmaterial (Herbers et al. 1995, Plant Mol. Biol. 29: 1027) mit *CPIP7* als cDNA-Sonde isoliert, wobei Standardprotokolle aus Sambrook et al. (vide supra) zur Anwendung kamen.

Der Vollängen cDNA Klon wurde als *NtCPIP1* bezeichnet und kodierte für 306 A-minoäuren, die innerhalb der ersten 70 Aminosäuren signifikante Homologie zu Mitgliedern der Familie der DnaJ-ähnlichen Proteine bzw. HSP40 Hitzeschockproteine aufwiesen und die namensgebende J-Domäne umfassten (Abb. 2a). Höchste Homologie (bestimmt nach Clustal-Methode im DNAStar Paket, DNASTAR Inc., Madison, Wisc.) zeigten *Arabidopsis thaliana* Klone mit 60,8 % (GenBank Accession AC007258), 60,1 % (GenBank Accession AC005489), sowie 58,8 % (GenBank Accession AC010871) Identität auf Proteinebene. Darüberhinaus waren 56,5 % Identität zu einem DnaJ-ähnlichen Protein aus *Nicotiana tabacum* (NtDnaJ_M541; GenBank Accession AAF05720) und 55,6 % zu dessen Ortholog AtA39 (GeneBank Accession AL021749) aus *Arabidopsis* nachweisbar, die als pflanzliche Interaktionspartner des TSWV NSm Zell-zu-Zell Transportproteins von Soellick et al (2000, Proc. Natl. A-cad. Sci. U.S.A. 97, 2373) identifiziert wurden.

Bei der nachfolgenden Durchmusterung einer λ ZAP II cDNA-Bank aus Tabak Blattmaterial (Herbers et al., 1995, Plant. Mol. Biol., 29: 1027) mit *CPIP7* als cDNA-Sonde nach unter Verwendung von Standardmethoden aus Sambrook et al. (vide supra) konnten zwei zusätzliche DnaJ-ähnliche Proteine identifiziert werden, deren DNA-Sequenzen samt der abgeleiteten Aminosäuresequenzen in Abbildungen 2b und 2c gegeben ist.

### Beispiel 2: Charakterisierung der Bindungsspezifität von NtCPIP 1

Zur Charakterisierung der Bindungspezifität von NtCPIP 1, NtCPIP2a und NtCPIP2b wurden weitere potyvirale Capsid Proteine auf Interaktion mit NtCPIP1, NtCPIP2a und NtCPIP2b im Hefe-zwei-Hybrid System getestet. Die für die Capsid Proteine des *Tobacco etch virus* (TEV, GenBank Accession M15239, Basen 8518-9309), *Tobacco vein mottling virus* (TVMV, GenBank Accession X04083, Basen 2759-3024), *Turnip mosaic virus* (TuMV, GenBank Accession D10601, Basen 4027-4890) und *Potato virus* A (PVA Stamm DAT, Genbank Accession AJ296311, Basen 8532-9338) kodierenden Sequenzen wurden in den pGBT9 oder pGBKT7 (Clontech) Vektor im offenen Leserahmen mit der GAL4-Bindungsdomäne ligiert, und die erhaltenen Plasmide mit pAD-CPIP29 (aus dem primären Hefe-Zwei-Hybrid Screen stammendes Plasmid) in den Reporterstamm Y190 kotransformiert.

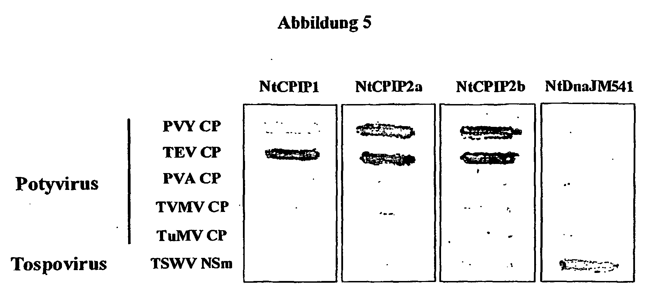

Die Hefen wurden für 3-4 Tage auf SD Trp⁻/Leu⁻ Medium selektioniert und nach Transfer auf SD Trp⁻/Leu⁻/His⁻ Medium auf Expression der HIS3 und lacZ Reportergene getestet. Es zeigte sich, dass NtCPIP1, NtCPIP2a und NtCPIP2b spezifisch mit den Capsid Proteinen von PVY und TEV, hingegen nicht mit denen von TVMV, TuMV und PVA interagiert (Tab. 1 und Abb. 5). Die Spezifität der Bindung wurde auch dadurch bestätigt, dass keine Bindung zwischen NtCPIP 1 und dem TSWV NSm Protein (Soellick et al. 2000, vide supra) zu detektieren war, wie auch dessen Interaktionspartner NtDnaJ_M541 keine Interaktion mit den PVY und TEV Capsid Proteinen im Hefe-Zwei-Hybrid-System zeigte (Tab. 1 und Abb. 5).

**Tabelle 1**

| **DNA-Bindungsdomäne** | *Aktivierungsdomäne* | | | |
|---|---|---|---|---|
| | *NtCPIP1* | *NtCPIP2a* | *NtCPIP2b* | *NtDnaJ_M541** |
| *PVY CP* | + | + | + | - |
| *TEV CP* | + | + | + | - |
| *PYA CP* | - | - | - | - |
| *TVMV CP* | - | - | - | - |
| *TuMV CP* | - | - | - | - |
| *NSm** | - | - | - | - |

| | | | | |
|---|---|---|---|---|
| *(Soellick et al. 2000, vide supra) | | | | |

### Beispiel 3: Detaillierte Charakterisierung der Interaktion von PVY CP mit NtCPIP1

Im folgenden Experiment wurde untersucht, welche Region von PVY CP für die Interaktion mit NtCPIP1 verantwortlich ist. Dazu wurde eine Reihe von N- oder C-terminal deletierten Fragmenten von CP und eine Serie von CP-Punktmutanten als GAL4-Bindungsdomänen-Fusionsproteine hergestellt.

### Herstellung von Punktmutanten und Deletionsmutanten für die Zwei-Hybrid-Analyse

Einzel- und Doppelaminosäuresubstitutionsmutationen wurden in der kodierenden Sequenz der PVY CP "core"-Region durch gerichtete Mutagenese (engl.: "site directed mutagenesis") eingeführt. Dazu wurde der "quick change site-directed mutagenesis" Kit (Stratagene) und entsprechende Oligonukleotide gemäß der Herstellerangaben verwendet. Zur Herstellung der Einzelaminosäuresubstitutionsmutanten S125W, R157D und D201R wurde das pGEM/T-PVY CP Plasmid als Vorlage benutzt. Doppel-Aminosäuresubstitutionsmutanten von PVY CP wurden dadurch erhalten, dass die S125W oder D201R-Mutation jeweils in das Plasmid eingeführt wurden, das die Einzel-Mutanten R157D enthielt oder indem eine D201R-Mutation in das Plasmid, das die S125W-Mutante enthielt, eingeführt wurde. Nachdem die Mutation durch Sequenzierung überprüft worden war, wurden die mutagenisierten CP-Sequenzen aus dem pGEM/T-Vektor ausgeschnitten und in den pGBT9 "bait" Vektor kloniert.

Die Δ29N und Δ18C-Deletionsmutanten wurden durch PCR-Amplifikation einer für CP kodierenden Region, der entweder die Aminosäuren 1 - 29 (Nukleotide (nt) 88 - 801 der PVY CP cDNA-Sequenz) oder 249 - 267 (nt 1- 747) fehlten, erhalten. Die PCR-Fragmente wurden in den pCR-Blunt (*in vitro-*Gen) subkloniert und in die EcoRI/SalI-Restriktionsschnittstellen des pGBT9 ligiert.

### Bindungsspezifität von NTCPIP1 und NtCPIP2a für Punktmutanten und Deletionsmutanten von PVY CP

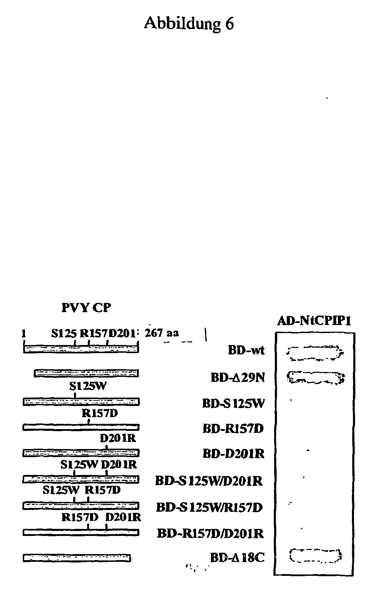

Zur Bestimmung der genauen Bindungsregionen in PVY CP bzw. in NtCPIP1 und NtCPIP2a wurden die oben genannten Punkt- und Deletionsmutanten im Hefe "Zwei-Hybrid"-Assay getestet, wie es oben bereits für die Interaktion verschiedener viraler CPs mit verschiedenen pflanzlichen DnaJ-ähnlichen Proteinen beschrieben wurde. Wie man Abb. 6 entnehmen kann, wird die Bindung von PVY CP an NtCPIP 1 nicht durch die N- und C-terminalen Deletionen von CP beeinflusst. Die Expression des *lac*Z Reportergens war durch die drei Punktmutanten und alle Doppelmutanten komplett unterdrückt, so dass der "core" Region des CP eine essentielle Rolle bei der Vermittlung mit NtCPIP1 zukommt.

### Beispiel 4: Herstellung von virusresistenten Tabakpflanzen durch Silencing von NtCPIP1

Um Tabakpflanzen für NtCPIP1 zu silencen und damit resistent z. B. gegen PVY zu machen, wurde ein Plasmid-Konstrukt hergestellt, dessen Transkription in der Zelle zu einem doppelsträngigen RNA-Molekül mit einer Sequenz, die Teile der für NtCPIP1 kodierenden Sequenz umfasst, führt. Ein solches doppelsträngiges RNA-Molekül soll dann das PTGS-System induzieren und die Expression von NtCPIP1 unterdrücken.

### Erzeugung des Plasmids pBinNtCPIP1-RNAi

Die *in vivo*-Bedeutung der Interaktion zwischen PVY CP und NtCPIP1 während des Infektionsverlaufes sollte durch Suppression der *NtCPIP1* Genexpression in transgenen Pflanzen analysiert werden. Dazu wurde die Strategie des so genannten RNAivermittelten "Silencing" verfolgt, die sich durch eine hohe Suppressionshäufigkeit und -effizienz (Smith et al. 2000, Nature 407: 319; Wesley et al. 2001, Plant J 27: 581) auszeichnet. Zur Erstellung eines RNAi-Konstruktes für *NtCPIP1* wurde zunächst das erste Intron der GA20-Oxidase aus *Solanum tuberosum* (StGA20oxIN, unveröffentlicht) unter Verwendung der Primer GAIN-1 (5'-CCT GCA GGC TCG AGA CTA GTA GAT CTG GTA CGG ACC GTA CTA CTC TA-3') und GAIN-2 (5'-CCT GCA GGG TCG ACT CTA GAG GAT CCC CTA TAT AAT TTA AGT GGA AAA-3') über PCR amplifiziert, so dass am 5'Ende die Schnittstellen PstI/Sbf1-XhoI-SpeI-BglII sowie am 3'Ende die Schnittstellen BamHI-XbaI-SalI-PstI/SbfI angefügt wurden. Dieses PCR-Fragment wurde in einen pCR-Blunt Vektor subkloniert und nach einem StuI-Verdau "Blunt-end" in einen pUC18 Vektor ligiert, der vorher durch einen EcoRI/HindIII-Verdau geöffnet und durch pfu-Polymerase aufgefüllt wurde.

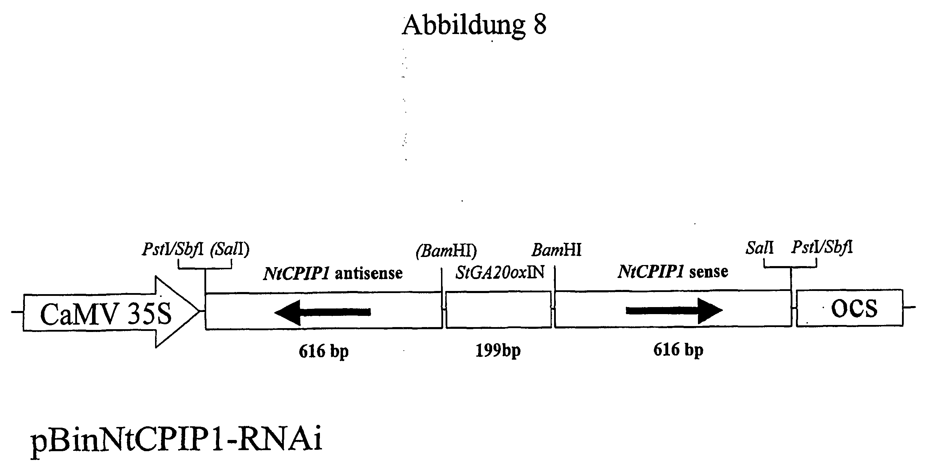

Der erhaltene Vektor wurde als pUC-RNAi bezeichnet (Abb. 7). Dann wurde über PCR unter Verwendung der Primer D153 (5'-GGA TCC CAG AAA ATC TCA TTA GTA GAT GC-3') und D 154 (5'-GTC GAC ATT CAT TAC CAG TCT ACC ACA GC-3') ein BamHI/SalI gelinkertes *NtCPIP1*-Fragment (Basen 721-1336) amplifiziert, und dieses nach Subklonierung in den pCR-Blunt Vektor sukzessive in Sense (s) und Antisense (α) Orientierung in den zunächst BamHI/SalI, danach BglII/XhoI geöffneten pUC-RNAi inseriert. Die gesamte RNAi-Kassette bestehend aus *αNt*C-PIP1-*St*GA20oxIN-*sNt*CPIP1 wurde schließlich über PstI aus dem pUC-RNAi Vektor in einen SbfI geschnittenen BinAR ligiert und so das Plasmid pBinNtCPIP1-RNAi erhalten (Abb. 8). Zur Pflanzentransformation wurde das Konstrukt schließlich in Agrobakterien transformiert.

### Analyse transgener Tabakpflanzen auf RNAi-vermitteltes "Silencing" "von NtCPIP1

Tabakpflanzen (*Nicotiana tabacum* L. Varietät Samsun NN) wurden mit pBinNtC-PIP1-RNAi tragenden Agrobakterien nach der beschriebenen Methode (vide supra) transformiert. 81 Kanamycin-resistente Primärtransformanden wurden auf Expression des Konstruktes zunächst über Northern-Analyse getestet, wobei das zur Herstellung des pBinNtCPIP1-RNAi Konstruktes verwendete *NtCPIP1*-Fragment (BamHI/SalI) als cDNA-Sonde bei der RNA-Hybridisierung diente.

### Analyse der Transkription durch "Northern Blot"-Analyse

Gesamt-RNA wurde aus Blattmaterial wie beschrieben isoliert (Logemann (1987), Anal. Biochem. 163, 16 - 20). Für die "Northern Blot"-Analyse wurden 30 µg rRNA auf einem 1,5% (w/v) Formaldehyd-Agarose-Gel aufgetrennt und auf einen GeneScreen (NEN, Boston, Ma) geblottet. Die radioaktive Markierung der cDNA wurde mit Hilfe des "High Prime" Kit (Boehringer Mannheim, Deutschland) und [α-]³²P]-dCTP durchgeführt. Die Hybridisierung wurde wie bekannt durchgeführt (Herbers et al., *vide supra*) und Signale durch Belichtung von Kodak X-ray-Filmen (Sigma) oder mit Hilfe eines "imaging analyzer" (Fuji Bas 2000, Fuji, Tokyo, Japan) detektiert.

24 Pflanzen zeigten eine eindeutiges Hybridisierungs-Signal, das allerdings einen charakteristischen "Schmier" über die RNA-Spur aufwies.

### Analyse transgener Pflanzen mit RT-PCR

Aus diesem Grund wurde das "Silencing" über semiquantitative RT-PCR analysiert. 20 µg Gesamt-RNA von ausgewählten Linien (NtCPIP1-RNAi-2, -3, -18, -69, -79) sowie von zwei nicht-transgenen Kontrollen (WT) wurde zunächst mit DNase (Böhringer Mannheim) bei 37°C für 45 min verdaut und diese anschließend für 10 min bei 65°C inhibiert. Nach Phenol/Choroform/Isoamylalkohol (25:24:1) Behandlung wurde die RNA mit Natriumacetat gefällt, mit 70 % Ethanol gewaschen und in 100 µl DEPC-behandeltem H₂O gelöst. Die cDNA Erststrangsynthese wurde in einem Ansatz mit 12,5 µl DNase behandelter RNA, 5 µl 5x Reaktions-Puffer, 2 µl dNTPs (2,5 mM), 1 µl Oligo dT Primer (50 mM, dT_{[30]}V[G/C/A]) und 2,5 µl DEPC-behandeltem H₂O nach Inkubation für 5 min bei 65°C, dann für 5 min bei 37°C schließlich nach Zugabe von 1 µl Reverser Transkriptase (Moloney Murine Leukemia Virus Reverse Transkriptase, Rnase H Minus, M-MLV [H-], Promega) und 1 µl RNAse-Inhibitor bei 37°C (60 min) durchgeführt. Nach Hitzeinaktivierung für 5 min bei 95°C wurde die cDNA als Matrize für die anschließende PCR eingesetzt.

*NtCPIP1* cDNA wurde mit dem 5'Primer D172 (5'-GGA TCC TCA CGC AAT GCG AGT GAA GAA G-3') und dem 3'Primer D154 (5'-GTC GAC ATT CAT TAC CAG TCT ACC ACA GC-3'), der interne Standard Actin wurde mit dem Primerpaar D202 (5'-ATG GCA GAC GGT GAG GAT ATT CA-3') und D203 (5'-GCC TTT GCA ATC CAC ATC TGT TG-3') amplifiziert (wie AC 1 und AC2, Romeis *et al.* 2001, *EMBO J* 20: 5556). Die PCR-Ansätze (Gesamtvolumen 100 µl) setzten sich wie folgt zusammen: 70 µl H₂O, 5 µl 5'Primer (5 µM), 5 µl 3'Primer (5 µM), 8 µl dNTPs (2,5 mM), 10 µl 10x Reaktions-Puffer, 1 µl cDNA und 5 U r*Taq* DNA Polymerase (Takara Shouzo, Japan). Vor Beginn der Amplifikations-zyklen wurden die Ansätze für 5 min auf 95°C erhitzt. Die Polymerisierungsschritte wurden in einem automatischen T3-Thermocycler (Biometra) nach folgendem Programm durchgeführt: Denaturierung 95°C (1 min), Anlagerung der Primer bei 55°C (45 Sekunden), Polymerase-Reaktion bei 72°C (2 min).Nach 25, 30, und 45 Zyklen wurden jeweils 10 µl des PCR-Ansatzes auf ein Gel aufgetragen.

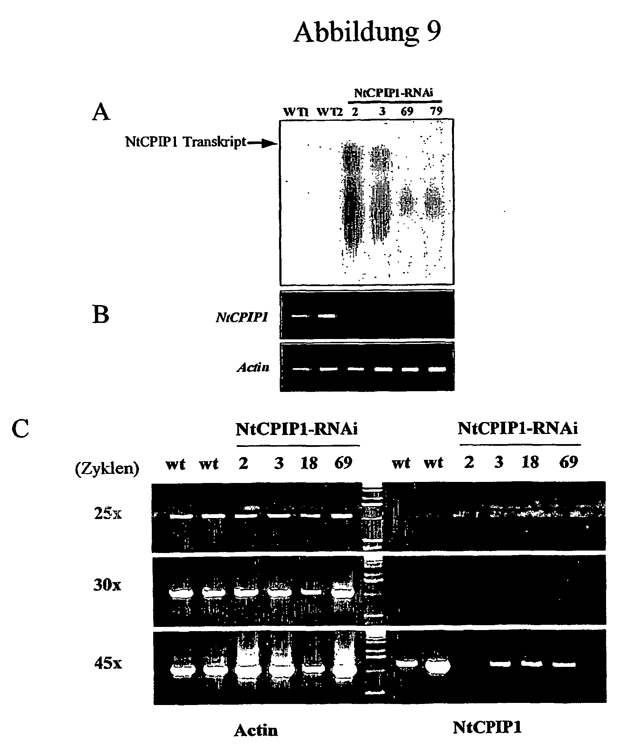

Das Ergebnis zeigt im nicht-gesättigten PCR-Bereich (30 Zyklen) bei Verwendung der *NtCPIP1*-spezifischen Primer nur die Amplifzierung von Produkten in den Wildtyp-Kontrollen und nicht in den 4 transgenen Linien (Abbildung 9c). Erst im ungesättigten Bereich (45 Zyklen) wird eine deutliche Akkumulation in den Linien 3, 28 sowie 69 erkennbar, während bei der Linie 2 kaum amplifiziertes DNA-Fragment zu detektieren ist, was auf sehr effizientes "Silencing" schließen lässt. Die PCR mit den Actin spezifischen Primern hingegen zeigt im ungesättigten Bereich bei 25 Zyklen gleichmäßige DNA-Banden, was den Einsatz vergleichbarer Mengen an eingesetzter Matrize belegt (Abbildung 9c).

Eine weitere transgene Linie (79) zeigte im ungesättigten Bereich (30 Zyklen) ebenfalls keine Amplifikation von Produkt (Abbildung 9b).

Vier der genannten Linien (NtCPIP1-RNAi-2, 3, 69 und 79) wurden ebenfalls im Northern Blot auf Expression von NtCPIP 1 hin untersucht. Wie Abb. 9a entnommen werden kann, zeigten die transgenen Linien keine Expression dieses pflanzlichen DnaJ-ähnlichen Proteins.

Alle untersuchten transgenen Linien waren phänotypisch nicht unterscheidbar von nicht-transformierten Wildtyppflanzen (WT). Sie waren ebenfalls nicht von transgenen Kanamycin-resistenten Kontrollpflanzen unterscheidbar, die das β-Glucuronidasegen (GUS) unter der Kontrolle eines "source leaf'-spezifischen Promotors exprimierten (Linie ME-4, Ebneth, M. (1996) Doktorarbeit, Freie Universität Berlin, Deutschland; Daten nicht gezeigt).

### PVY-Infektion transgener Tabakpflanzen

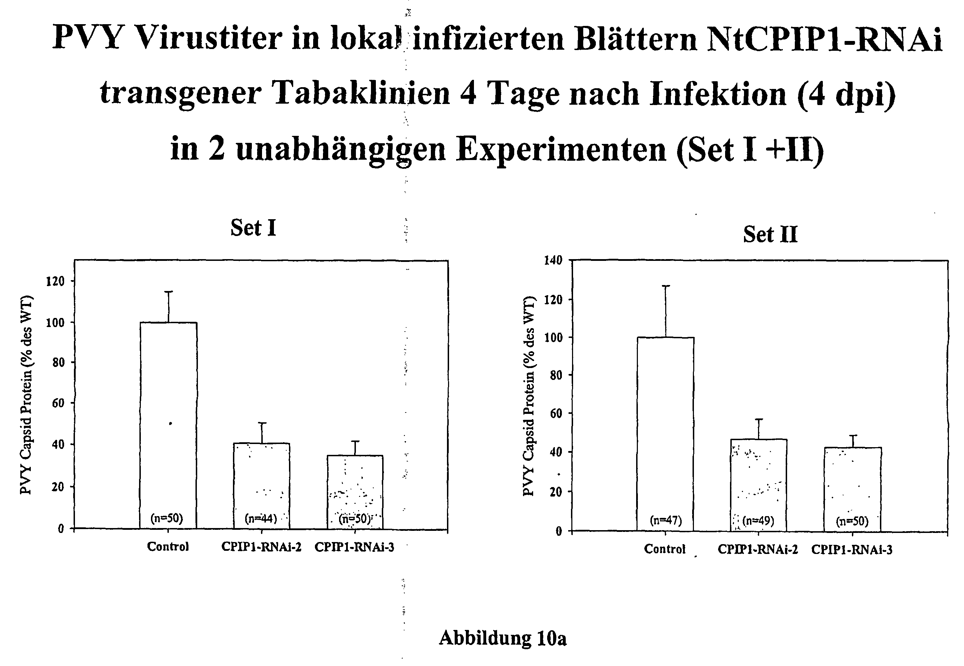

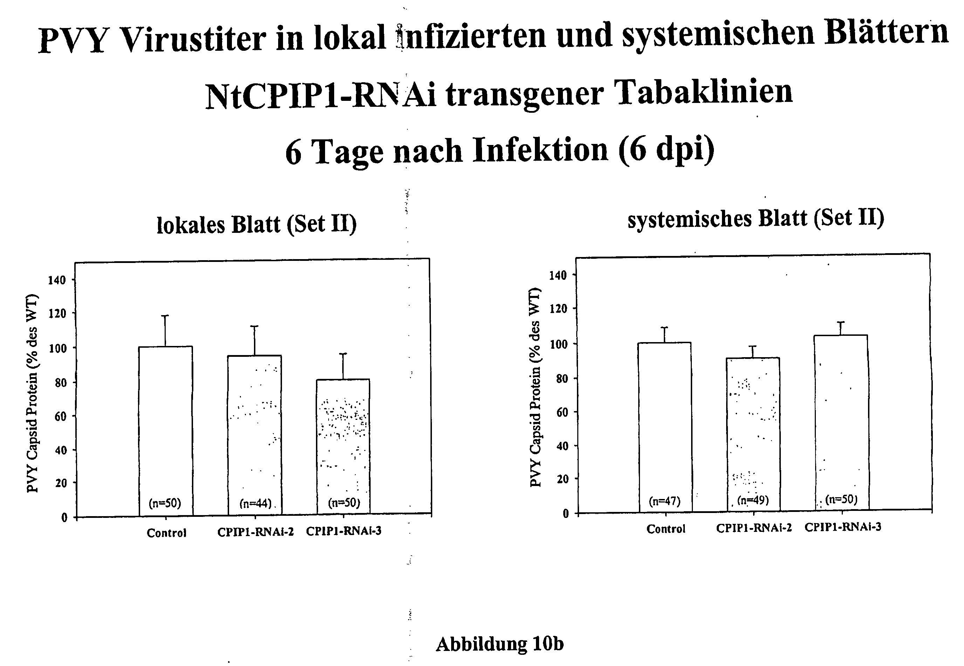

Die Infektion von NtCPIP1-RNAi transgenen Linien sowie nicht-transformierten Kontrollen (WT) mit PVY^{N} erfolgte nach der in Herbers et al. 1996 (vide supra) beschriebenen Methode. Die Ergebnisse sind in den Abbildungen 10a und 10b dargestellt. Es zeigte sich, dass sich in den transgenen Linien NECPIR-RNAi-2 und -3 im lokal infizierten Blatt vier Tage nach der Infektion eine reproduzierbar signifikant erhöhte Virusresistenz einstellte (Abb. 10a). Es handelte sich dabei um eine transiente Resistenz, da sechs Tage nach der Infektion eine Reduktion des Virustiters nicht mehr zu detektieren war (Abb. 10b).

Um die Effekte der reduzierten Expression von endogenem NtCPIP1 auf die PVY Akkumulation und systemische Ausbreitung zu untersuchen, wurden die Kanamycinresisten T1-Nachkommen der selektionierten transgenen Pflanzen mit PVY^{N} infiziert und mit Kontrolllinien (WT, ME-4) verglichen. Um Positionseffekte im Gewächshaus, wie sie wegen der hohen Anzahl an Pflanzen, die benutzt wurden, auftreten könnten, auszuschließen, wurden zwei unabhängige Infektionsexperimente parallel durchgeführt. Die transgenen Linien NtCPIP1-RNAi-2 (n = 49) und NtCPIP1-RNAi-3 (n = 50) wurden mit SNN-Wildtyppflanzen (n = 47) verglichen, wohingegen NtCPIP1-RNAi-69 (n = 25) und NtCPIP1-RNAi-79 (n =25) im Vergleich mit der transgenen Kontrolle ME-4 (n = 23) analysiert wurden.

Innerhalb der unterschiedlichen Pflanzensätze wurden die transgenen Linien und die jeweiligen Kontrollen nach dem Zufallsprinzip vermischt und PVY CP Proteinlevel vier Tage nach der mechanischen Infektion in lokalen (beimpften) Blättern überprüft. Die Analyse wurde ebenso sechs Tage nach der Infektion in lokalen und systemischen Blättern durchgeführt.

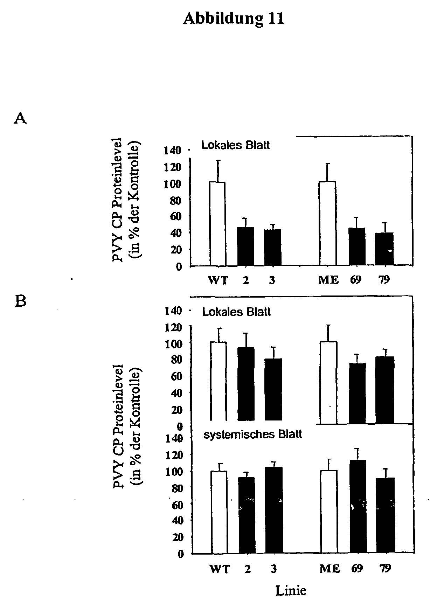

Die Ergebnisse sind in den Abbildungen 10a und 10b sowie 11a und 11b dargestellt, wobei die Werte für die WT-Pflanzen und NtCPIP1-RNAi-2 und 3-Linien in Abb. 11a und b denen der Abbildungen 10a und b entsprechen.

Wie Abbildungen 10a, 10b, 11a und 11b entnommen werden kann, wiesen die transgenen Linien NtCPIP1-RNAi-2 und -3 im lokal infizierten Blatt nach der Infektion eine reproduzierbar signifikant erhöhte Virusresistenz auf. Dies äußerte sich darin, dass vier Tage nach der PVY-Infektion die Virusvervielfältigung signifikant um 53,4% und 57,5% in beimpften Blättern von NtCPIP1-RNAi-2 und NtCPIP1-RNAi-3 reduziert war (im Vergleich zu WT-Pflanzen). In den lokalen Blättern der transgenen Pflanzen NtCPIP1-RNAi-69 und NtCPIP1-RNAi-79 war die Multiplikation des Virus konsistent um 56,0% und 60,1% im Vergleich zur transgenen Kontrolle ME-4 reduziert.

Es handelte sich dabei um eine transiente Resistenz, da sechs Tage nach der Infektion die Reduktion des Virustiters nicht mehr so ausgeprägt war. Eine vergleichbar starke Reduktion des Viruslevels um 59,3% und 65,2% wurde für die transgenen Linien NtCPIP1-RNAi-2 (n = 44) und NtCPIP1-RNAi-3 (n = 50) im Vergleich zu der transgenen Kontrolle ME-4 (n = 50) nachgewiesen (Daten nicht gezeigt).

Diese Ergebnisse zeigen deutlich, dass die Interaktion des pflanzlichen DnaJ-ähnlichen Proteins NtCPIP1 mit dem PVY Capsid Protein eine Rolle bei der Etablierung der Infektion *in planta* spielt und bestätigen die Two - Hybrid Daten.

Die Ergebnisse zeigen zudem, dass durch das Silencing von pflanzlichen DnaJ-ähnlichen Proteinen Pflanzen mit einer erhöhten Virusresistenz hergestellt werden können.

### Herstellung transgener Tabakpflanzen mit mehrfach gesilencten DnaJ-ähnlichen Proteinen

Im oben beschrieben Fall kann das Auftreten einer transienten Virusresistenz damit erklärt werden, dass in Tabak zwei weitere Isoformen von NtCPIP1 vorkommen. Es handelt sich um die ebenfalls im Rahmen der vorliegenden Erfindung identifizierten NtCPIP2a und NtCIP2b. Diese Proteine werden möglicherweise durch das verwendete RNAi-Konstrukt nicht reprimiert und können womöglich das RNA-Silencing von NtCPIP1 kompensieren, da sie nachweislich in der Lage sind, mit PVY CP zu interagieren (siehe oben).

Im Folgenden wurden daher analog zur oben beschriebenen Vorgehensweise Konstrukte hergestellt, die das Silencen von NtCPIP2a und NtCPIP2b erlauben. Mit diesen Konstrukten wurden entsprechende transgene Pflanzen hergestellt, die ebenfalls für NtCPIP1 gesilenct waren. Die Pflanzen wurden durch semi-quantitative RT-PCR hinsichtlich der NtCPIP 1, NtCPIP2a und NtCPIP2b Expression untersucht. Alle drei Proteine waren signifikant gesilenct. Die transgenen Pflanzen wurden daraufhin mit PVY^{N} wie oben beschrieben infiziert und analysiert. Es zeigte sich, dass die transgenen Pflanzen eine nachhaltige und andauerende Virusresistenz aufweisen.

### Beispiel 5: Herstellung von virusresistenten Tabakpflanzen durch Expression dominant-negativer Mutanten von NtCPIP1

Die lokale und transiente Reduktion der PVY-Mengen in NtCPIP1-RNAi-transgenen Pflanzen kann mit mehreren Gründen erklären werden. Der wahrscheinlichste Grund dürfte der sein, dass die Suppression von NtCPIP1 durch Isoformen kompensiert werden kann, die ebenfalls in der Lage sind, mit PVY CP zu interagieren. Wie bereits oben dargestellt wurde, interagieren sowohl NtCPIP2a als auch NtCPIP2b im Hefe "Zwei-Hybrid"-System mit PVY CP und TEV CP.

Um permanent virusresistente Pflanzen herzustellen, wurden daher verkürzte dominant-negative Mutanten der CP-interagierenden pflanzlichen DnaJ-ähnlichen Proteine in transgenen Pflanzen exprimiert.

### Herstellung von transgenen Pflanzen, die dominant-negative Mutanten von NtCPIP1 und NtCPIP2a exprimieren

Eine Serie von N-terminalen Deletionsmutanten von NtCPIP1 und NtCPIP2a wurden durch PCR-Amplifikation unter Verwendung entsprechender Oligonukleotide erhalten. Fragmente, die an ihren 5'- bzw. 3'-Enden die Sequenzen für EcoRI- bzw. SalI-Restriktionsschnittstellen aufwiesen und denen die Aminosäurereste 1- 65 (Δ65N, nt 246 - 978), 1-90 (Δ90N, nt 331 - 987), 1 - 115 (Δ115N, nt 406 - 987) von NtCPIP 1 und die Aminosäurereste 1- 66 (Δ66N, nt 199 - 918), 1 - 94 (Δ94N, nt 283 - 918) und 1-119 (Δ119N, nt 357 - 918) von NtCPIP2a fehlten, wurden in den pCR-Blunt subkloniert und schließlich in den pAD-GAL4 "activation domain" Vektor (Stratagene) eingeführt.

Für die ektopische Expression von dominant-negativen Mutanten von NtCPIP1 und NtCPIP2a wurden trunkierte cDNA-Fragmente, die nicht für die Aminosäurereste 1 - 65 (NtCPIP 1 Δ65N) und 1 - 66 (NtCPIP2a Δ66N) kodierten, durch PCR amplifiziert. Dazu wurden die genspezifischen Primer D249 (5'-GGATCCTATATACGGCGATGAGGCGTTGAAATC-3') und D251 (5'-GTCGACTTAGTCAACAGTCCTGCCCAGCAC-3') für NtCPIP1Δ65N sowie D250 (5'-GGATCCTGACGTGTACGGTGATGATGCATTG-3') und D210 (5'-GTCGACTTAGTCAGCGCTCCTGCACAGTAC-3') für NtCPIP2aΔ66N benutzt. Nach Subklonierung in den pCR-Blunt wurden die Fragmente in den pBinAR zwischen den CaMV 35S Promotor und den ocs Terminator inseriert. Um die Translationseffizienz zu verbessem, wurde die 5'-untranslatierte "overdrive" Sequenz (Ω) des TMV Virus U1 (Gallie et al. (1987) Nucl. Acids Res., 15, 8693 - 8711) zwischen den Promotor und die NtCPIP 1Δ65N oder NtCPIP2aΔ66N kodierenden Sequenzen inseriert. Dadurch wurde ebenfalls ein ATG-Startkodon in einer optimierten Pflanzen-Konsensussequenz innerhalb einer NcoI Klonierungsstelle generiert.

Zur Transformation von Tabakpflanzen (*Nicotiana tabacum* L. cv. Samsun NN) wurden die binären Konstrukte in den *Agrobacterium tumefaciens* Stamm C58C1:pGV2260 eingeführt. Anschließend wurde ein *Agrobacterium*-vermittelter Gentransfer wie oben und in Rosahl et al. (1987, EMBO J., 6,1155 - 1159) beschrieben durchgeführt.

### Analyse von transgenen Pflanzen, die dominant-negative Mutanten von NtCPIP1 und 2a exprimieren

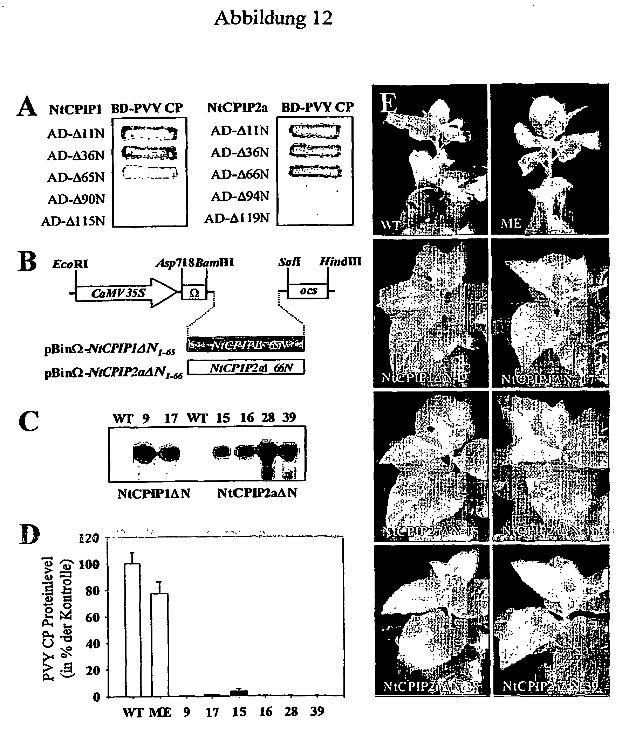

Die oben genannten N-terminalen Deletionsmutanten von NtCPIP1 und NtCPIP2a verfügten jeweils nicht mehr über die J-Domäne. Zunächst sollte daher im Hefe "Zwei-Hybrid" System untersucht werden, ob die Deletion des Hauptanteils der J-Domäne die Interaktion von NtCPIP 1 und NtCPIP2a mit PVY CP beeinflusst. Die N-terminale Deletion der Aminosäuren 1 - 65 und 1 - 66 von NtCPIP 1 bzw. 2a führte jedoch nicht zu einem Verlust der *lac*Z Reportergenaktivität, so dass angenommen werden kann, dass die J-Domäne nicht für die Bindung an PVY CP notwendig ist. Umfassten die N-terminalen Deletionen jedoch mehr als die ersten 90 Aminosäuren, wurde ein kompletter Verlust der Bindung der NtCPIP Isoformen an PVY CP beobachtet (siehe Abb. 12a).

Es wurden daher binäre Konstrukte hergestellt (pBinΩ-NtCPIP1 ΔN₁₋₆₅ und pBinΩ-NtCPIP2a ΔN₁₋₆₆, siehe Abb. 12b), die für NtCPIP Varianten kodierten, die zwar keine J-Domäne aufwiesen, aber in der Lage sind, mit PVY CP zu interagieren. Aufgrund des Fehlens einer J-Domäne sollten diese pflanzlichen DnaJ-ähnlichen Proteine, die üblicherweise als Co-Chaperone und Regulatoren der HSP70-Proteine fungieren (siehe oben), nicht mehr in der Lage sein, mit ihren endogenen zellulären Interaktionspartnem zu wechselwirken.

Nach der *Agrobacterium-*vermittelten Transformation wurden 26 primäre Transformanten für jedes Konstrukt im Gewächshaus angezogen und hinsichtlich der Expression des Transgens durch Northern Blotting untersucht. Die Northern Blots wurden dabei wie oben beschrieben hergestellt. Auf diese Weise konnten einige Pflanzen identifiziert werden, die signifikante Mengen der jeweiligen Transkripte exprimierten. Zwei Pflanzen, die das Konstrukt pBinΩ-NtCPIP1 ΔN₁₋₆₅ (als NtCPIP1 ΔN-9 und -17 bezeichnet) und vier transgene Pflanzen, die das pBinΩ-NtCPIP2a ΔN₁₋₆₆ (als NtCPIP1 ΔN-15, -16, -28 und -39 bezeichnet) exprimierten, wurden weiter analysiert (siehe Abb. 12c).

Pflanzen, die von diesen Zelllinien abgeleitet wurden, waren phänotypisch von Wildtyppflanzen und transgenen Kontrollpflanzen nicht unterscheidbar.

Um zu überprüfen, ob die Expression der N-terminal deletierten NtCPIP1- und 2a-Mutanten die Multiplikation und systemische Ausbreitung des PVY Virus behindert, wurden Kanamycin-resistente T1 Nachkommen der ausgewählten transgenen Pflanzen mit PVY infiziert und mit entsprechenden Wildtyppflanzen oder transgenen Kontrollpflanzen verglichen (WT, ME1-10). 22 - 25 Pflanzen, die die N-terminal deletierten Fragmente von NtCPIP1 und -2a exprimierten, sowie eine vergleichbare Anzahl an WT-Pflanzen (n = 22) und transgenen Kontrollpflanzen (ME1-10, n = 24) wurden mechanisch im Siebenblattstadium beimpft und die Entwicklung einer Virusinfektion durch visuelle Inspektion der Symptome und immunologische Bestimmung des CP Proteinlevels bestimmt.

Alle beimpften transgenen (ME1-10) und nichttransgenen (WT) Kontrollpflanzen wiesen typische Krankheitssymptome beginnend mit 5 - 6 Tagen nach der Beimpfung auf. Die verschiedenen transgenen Linien von NtCPIP1 und 2a zeigten dagegen keine sichtbaren Virus-induzierten Symptome zu diesem Zeitpunkt (Daten nicht gezeigt). Damit übereinstimmend war der Virustiter in den systemischen Blättern der transgenen Linien stark reduziert. Teilweise wurde eine komplette Resistenz beobachtet (siehe Abb. 12d). Die Virusresistenz konnte auch 13 Tage nach der Infektion in den transgenen NtCPIP1 und 2a-Linien bestätigt werden. Alle transgenen Zelllinien wiesen signifikant reduzierte virale Symptome im Vergleich zu den Kontrollzelllinien auf. Die viralen Symptome waren zum Teil gar nicht zu detektieren (siehe Abb. 12e).

### Abbildungslegenden

**Abb.1: a)** Alignment der Proteine NtCPIP1, NtCPIP2a, NtCPIP2b und NtDnaJ_M541, das mit dem TSWV Movement Protein (NSm) interagiert (Soellick et al., 2000). **b)** Homologie der Proteinsequenzen in Prozent identischer Aminosäuren bestimmt nach Clustal-Methode im DNAStar Paket, DNASTAR Inc., Madison, Wisc. **c)** Konsensus-Sequenz der DnaJ-Domäne. Nter und Cter stehen für N-Terminus und C-Terminus und geben die Orientierung der Sequenz an.

**Abb. 2a:** *NtCPIP1* cDNA (SEQ ID NO: 1) und abgeleitete Aminosäuresequenz (SEQ ID NO:4) aus *Nicotiana tabacum* Die im Hefe-2-Hybrid-System identifizierten, mit PVY CP interagierenden Aminsoäuren sind hervorgehoben. Die namensgebende J-Domäne ist unterstrichen. Die Lys-reichen Domänen sind grau unterlegt.

**Abb. 2b:** NtCPIP2a cDNA (SEQ ID NO: 2) mit abgeleiteter Aminosäuresequenz (SEQ ID NO: 5).

**Abb. 2c:** NtCPIP2b cDNA (SEQ ID NO: 3) mit abgeleiteter Aminosäuresequenz (SEQ ID NO: 6).

**Abb. 3:** PVY CP cDNA mit abgeleiteter Aminosäuresequenz

**Abb. 4:** Spezifische Interaktion zwischen dem *Potato virus Y* Capsid Protein (PVY CP) und dem DnaJ-ähnlichen Protein NtCPIP1 aus Nicotana tabacum im Hefe 2-Hybrid-System. Das mit der Gal4-Bindungsdomäne (BD) fusionierte PVY CP (pGBT9-PVY CP) zeigt nur bei Coexpression mit dem Gal4-Aktivierungsdomäne fusionierten NtCPIP 1 (pAD-CPIP1) *lacZ*-Aktivität, während in Kontrollreaktionen NtCPIP1 gegenüber der Bindungsdomäne alleine (pGBT9) oder dem nicht verwandten Protein SNF1 (pBD-SNF1) keine Aktivität hervorruft. Entsprechend zeigt auch die Coexpression von PVY CP und SNF4 (pAD-SNF4) - dem Bindungspartner von SNF1- keine Aktivität, während SNF1 und SNF4 in der Posistivkontrolle deutliche *lacZ*-Aktivität induzieren.

**Abb. 5:** LacZ-Filterlift Assay zur Analyse der Protein-Protein-Interaktionen von NtCPIP1, NtCPIP2a, NtCPIP2b sowie NtDnaJ-M541 (Soellick et al., 2000, *vide supra*) mit potyviralen CPs (PVY, TEV, TVMV, TuMV) und dem TSWV NSm Movement Protein (Soellick et al., *vide supra*) im Hefe-2-Hybrid System.

**Abb. 6:** PVY CP Deletions- und verschiedene Punktmutanten wurden jeweils mit NtCPIP1 in Y190 Zellen ko-transformiert und qualitativ hinsichtlich β-Galactosidase Aktivität analysiert.

**Abb. 7:** Schematische Darstellung des pUC-RNAi-Vektors.

**Abb. 8:** Schematische Darstellung des pBinNtCPIPI-RNAiVektors.

**Abb. 9: a)** Northern Blot Analyse von NtCPIP1-spezifschen Transkripten. Gesamt-RNA wurde entweder aus WT-Pflanzen oder transgenen Pflanzen isoliert und mit NtCPIP1 cDNA hybridisiert. **b)** und **c)** Analyse der NtCPIP1 Suppression durch RT-PCR. In B) sind die PCR Produkte für NtCPIP1 und Actin der PCR Zyklen 30 bzw. 25 gezeigt.

**Abb. 10: a)** PVY Virustiter in lokal infizierten Blättern von NtCPIP1-RNAi transgenen Linien. PVY Capsid Protein Level wurden 4 Tage nach Infektion in den lokal infizierten Blattern der Linien NtCPIP1-RNAi-2 und-3 sowie in Kontrollpflanzen, wie in Herbers et al. 1996 beschrieben, bestimmt. Dargestellt sind die Ergebnisse von zwei unabhängigen Experimente jeweils als % des Wildtyp (Kontroll)-Levels. Die Werte repräsentieren den Mittelwert der angegeben Anzahl von Pflanzen (n) ± Standardfehler. **b)** PVY Virustiter in lokal infizierten und systemischen Blättern von NtCPIP1-RNAi transgenernLinien. PVY Capsid Protein Level wurden 6 Tage nach Infektion in den lokal und systemisch infizierten Blattern der Linien NtCPIP1-RNAi-2 und-3 sowie in Kontrollpflanzen, wie in Herbers et al. 1996 beschrieben, bestimmt. Dargestellt sind die Ergebnisse als % des Wildtyp (Kontroll)-Levels. Die Werte repräsentieren den Mittelwert der angegeben Anzahl von Pflanzen (n) ± Standardfehler.

**Abb. 11:** Effekt von PVY Infektionen in NtCPIP1-gesilencten trans genen Pflanzen. **a)** Der Virus Titer wurde vier Tage nach der Infektion in lokalen (beimpften) Bättem der Linien NtCPIP1-RNAi-2, -3, -69 bzw. -79 und WT-Pflanzen bzw. transgenen Kontrollpflanzen ME-4 bestimmt. **b)** Der Virus Titer wurde vier Tage nach der Infektion in lokalen (beimpften) oder systemischen Bättem (4-5 Blätter oberhalb der lokalen (beimpften) Blättern) der Linien NtCPIP1-RNAi-2, -3, -69 bzw. -79 und WT-Pflanzen bzw. transgenen Kontrollpflanzen ME-4 bestimmt. Die Pflanzen hatten vor der Infektion sechs Blätter entwickelt

**Abb. 12:** Expression von dominant negativen Mutanten von NtCPIP1 und 2a in Tabak-Pflanzen. **a)** Interaktion von N-terminal deletierten Fragmenten von NtCPIP1 und 2a mit PVY CP im Hefe "Zwei-Hybrid"-System. **b)** Schematische Darstellung der zur Expression verwandten binären Konstrukte. **c)** Northern Blot Analyse der NtCPIP1Δ65N- und NtCPIP2aΔ66N-spezifischen Transkripte. Es wurde Gesamt-RNA aus Kontroll- oder transgenen Pflanzen isoliert und mit NtCPIP1Δ65N- bzw. NtCPIP2aΔ66N-cDNA hybridisiert. **d)** PDY Titer in systemischen Blättern (5-6 Blätter oberhalb der beimpften Blätter) 6 Tage nach Infektion. **e)** Entwicklung von Virus-induzierten Symptomen in Kontroll und transgenen Pflanzen 13 Tage nach Infektion.

**Abb. 13:** Eine weitere DNA-Sequenz für ein DnaJ-ähnliches Protein aus *Nicotiana tabacum* (SEQ ID NO:10).

## Patentansprüche

1. Verfahren zur Herstellung von transgenen Pflanzen mit erhöhter Virusresistenz, **dadurch gekennzeichnet,**
**dass** die Expression von pflanzlichen DnaJ-ähnlichen Proteinen, die mit viralen Komponenten interagieren, im Wesentlichen unterbunden wird.

2. Verfahren nach Anspruch 1,
**dadurch gekennzeichnet, dass** die Expression von pflanzlichen DnaJ-ähnlichen Proteinen durch Übertragung von Nukleinsäuremolekülen umfassend Sequenzen, die identisch, homolog oder komplementär zu den Sequenzen sind, die für pflanzliche DnaJ-ähnliche Proteine oder Teile davon kodieren auf Pflanzenzellen, auf Pflanzenzellen im Wesentlichen unterbunden wird.

3. Verfahren nach Anspruch 1 oder 2,
**dadurch gekennzeichnet, dass** die übertragenen Nukleinsäuremoleküle Sequenzen umfassen, die identisch, homolog oder komplementär zu den Sequenzen sind, die die J-Domäne von pflanzlichen DnaJ-ähnlichen Proteine oder Teile davon kodieren.

4. Verfahren nach Anspruch 1 oder 2,
**dadurch gekennzeichnet, dass** die übertragenen Nukleinsäuremoleküle Sequenzen umfassen, die identisch, homolog oder komplementär zu Sequenzen sind, die die Protein-spezifischen Bereiche von pflanzlichen DnaJ-ähnlichen Proteinen, die nicht mit der J-Domäne zusammenfallen, oder Teile davon kodieren.

5. Verfahren nach einem der vorgehenden Ansprüche,
**dadurch gekennzeichnet, dass** der Teil der übertragenen Nukleinsäuresequenzen, der identisch, homolog oder komplementär zu den für pflanzliche DnaJ-ähnliche Proteine oder Teilen davon kodierenden Sequenzen ist, 20 bis 1000 Nukleotide, 20 bis 750 Nukleotide, bevorzugt 20 bis 500 Nukleotide, ebenfalls bevorzugt 20 bis 250 Nukleotide, besonders bevorzugt 20 bis 150 Nukleotide, insbesondere bevorzugt 20 bis 100 Nukleotide und am meisten bevorzugt um die 20-50 Nukleotide umfasst.

6. Verfahren nach einem der vorgehenden Ansprüche,
**dadurch gekennzeichnet, dass** ein Teil der übertragenen Nukleinsäuresequenzen mindestens zu 50%, bevorzugt mindestens zu 60%, ebenfalls bevorzugt mindestens zu 70%, besonders bevorzugt mindestens zu 80%, insbesondere bevorzugt mindestens zu 90% und am meisten bevorzugt mindestens zu 95% homolog zu den für pflanzliche DnaJ-ähnliche Proteine oder Teilen davon kodierenden Sequenzen ist.

7. Verfahren nach einem der vorgehenden Ansprüche,
**dadurch gekennzeichnet, dass** ein Teil der übertragenen Nukleinsäuresequenzen mindestens zu 50%, bevorzugt mindestens zu 60%, ebenfalls bevorzugt mindestens zu 70%, besonders bevorzugt mindestens zu 80%, insbesondere bevorzugt mindestens zu 90% und am meisten bevorzugt mindestens zu 95% komplementär zu den für pflanzliche DnaJ-ähnliche Proteine oder Teilen davon kodierenden Sequenzen ist.

8. Verfahren nach einem der vorgehenden Ansprüche,
**dadurch gekennzeichnet, dass** es die folgenden Schritte umfasst:
a) Herstellen eines Vektors, der die folgenden Sequenzbestandteile in 5'-3'-Orientierung umfasst:
- ein in Pflanzen funktionsfähiger Promoter
- operativ daran gebunden die identische oder homologe Antisense-Sequenz der für das pflanzliche DnaJ-ähnliche Protein oder Teilen davon kodierenden Sequenz, wobei die Sequenz an ihrem 3'-Ende vom Spleißosom erkennbare 3'-Exon-Sequenzen aufweist
- ein Intron
- die identische oder homologe *Sense*-Sequenz der für das pflanzliche DnaJ-ähnliche Protein oder Teilen davon kodierenden Sequenz, wobei die Sequenz an ihrem 5'-Ende vom Spleißosom erkennbare 5'-Exon-Sequenzen aufweist
- eine Terminationssequenz
b) Übertragen des Vektors aus a) auf Pflanzenzellen und gegebenenfalls Integration ins pflanzliche Genom

9. Verfahren nach einem der Ansprüche 1 bis 7,
**dadurch gekennzeichnet, dass** es die folgenden Schritte umfasst:
a) Herstellen eines Vektors, der die folgenden Sequenzbestandteile in 5'-3'-Orientierung umfasst:
- ein in Pflanzen funktionsfähiger Promoter
- operativ daran gebunden die identische oder homologe Antisense-Sequenz der für ein pflanzliches DnaJ-ähnliches Protein oder Teilen davon kodierenden Sequenz
- eine Terminationssequenz
b) Übertragen des Vektors aus a) auf Pflanzenzellen und gegebenenfalls Integration ins pflanzliche Genom

10. Verfahren nach einem der Ansprüche 1 bis 7,
**dadurch gekennzeichnet, dass** es die folgenden Schritte umfasst:
a) Herstellen eines Vektors, der die folgenden Sequenzbestandteile in 5'-3'-Orientierung umfasst:
- ein in Pflanzen funktionsfähiger Promoter
- operativ daran gebunden die identische oder homologe Sense-Sequenz der für ein pflanzliches DnaJ-ähnliches Protein oder Teilen davon kodierenden Sequenz, wobei die Sequenz über selbst-komplementäre Bereiche verfügt
- eine Terminationssequenz
b) Übertragen des Vektors aus a) auf Pflanzenzellen und gegebenenfalls Integration ins pflanzliche Genom

11. Verfahren nach einem der Ansprüche 1 bis 7,
**dadurch gekennzeichnet, dass** es die folgenden Schritte umfasst:
a) Herstellen eines Vektors, der die folgenden Sequenzbestandteile in 5'-3'-Orientierung umfasst:
- ein in Pflanzen funktionsfähiger Promoter
- eine operativ daran gebundene DNA-Sequenz, die zu der für die mRNA des pflanzlichen DnaJ-ähnlichen Proteins oder Teilen davon kodierenden Sequenz komplementär ist
- eine DNA-Sequenz, die für Ribonuklease P kodiert
- eine Terminationssequenz
b) Übertragen des Vektors aus a) auf Pflanzenzellen und gegebenenfalls Integration ins pflanzliche Genom

12. Verfahren nach einem der Ansprüche 1 bis 7,
**dadurch gekennzeichnet, dass** es die folgenden Schritte umfasst:
a) Herstellen eines Vektors, der die folgenden Sequenzbestandteile in 5'-3'-Orientierung umfasst:
- einen in Pflanzen funktionsfähigen Promoter
- eine DNA-Sequenz, die identisch oder homolog zu der für das 5'-Ende des pflanzlichen DnaJ-ähnlichen Proteins kodierenden Sequenz ist
- eine DNA-Sequenz, die für ein Resistenzgen kodiert
- eine DNA-Sequenz, die identisch oder homolog zu der für das 3'-Ende des pflanzlichen DnaJ-ähnlichen Proteins kodierenden Sequenz ist
- eine Terminationssequenz
b) Übertragen des Vektors aus a) auf Pflanzenzellen und Integration ins pflanzliche Genom

13. Verfahren nach einem der Ansprüche 1 bis 7, umfassend die folgenden Schritte:
a) Herstellen eines Vektors, der die folgenden Sequenzbestandteile in 5'-3'-Orientierung umfasst:
- ein in Pflanzen funktionsfähiger Promoter
- operativ daran gebunden eine DNA-Sequenz, die für ein Ribozym kodiert, das spezifisch die mRNA von pflanzlichen DnaJ-ähnlichen Proteinen erkennt
- eine Terminationssequenz
b) Übertragen des Vektors aus a) auf Pflanzenzellen und gegebenenfalls Integration ins pflanzliche Genom

14. Verfahren zur Herstellung von transgenen Pflanzen mit erhöhter Virusresistenz, umfassend die folgenden Schritte
a) Herstellung eines Vektors, der die folgenden Bestandteile in 5'-3'-Orientierung umfasst:
- ein in Pflanzen funktionsfähiger Promoter
- operativ daran gebunden eine DNA-Sequenz, die für eine dominant-negative Mutante eines pflanzlichen DnaJ-ähnlichen Proteins, das mit viralen Komponenten interagiert, oder einen rekombinanten Antikörper, der spezifische für pflanzliche DnaJ-ähnliche Proteine ist, kodiert
- ein Terminationssignal
b) Übertragen des Vektors aus a) auf die Pflanzenzellen und gegebenenfalls Integration ins pflanzliche Genom.

15. Verfahren nach einem der Ansprüche 7 bis 14,
**dadurch gekennzeichnet, dass** der Vektor zusätzlich zu Promotoren weitere regulatorische und funktionelle Sequenzen enthält.

16. Verfahren nach dem Anspruch 15,
**dadurch gekennzeichnet, dass** es sich bei den regulatorischen Sequenzen um Enhancer, um Replikationssignale, Selektionsmarker und/oder um Sequenzen handelt, die eine Propagation der Vektoren in Bakterien und/oder eine Replikation in Pflanzenzellen ermöglichen.

17. Verfahren nach einem der Ansprüche 7 bis 16,
**dadurch gekennzeichnet, dass** es sich bei den Vektoren um Plasmide, Cosmide und/oder rekombinante Viren handelt.

18. Verfahren nach Anspruch 17,
**dadurch gekennzeichnet, dass** es sich bei den Vektoren um pBR322, um pUC-Vektoren, um M13mp-Vektoren oder um Vektoren handelt, die vom Ti- oder Ri-Plasmid von Agrobacterien abgeleitet sind.

19. Verfahren nach einem der Ansprüche 7 bis 18,
**dadurch gekennzeichnet, dass** es sich bei dem Promoter um konstitutive Promotoren, bevorzugt um den 35S- , den Actin- oder den Ubiquitin-Promotor, um gewebespezifische Promotoren, bevorzugt um den Phosphoenolpyruvat-Carboxylase- oder den Fructose-1,6-bisphosphatase-Promoter, um entwicklungsspezifische, licht- , verwundungs-, oder pathogeninduzierte Promotoren, bevorzugt um virusinduzierte Promotoren handelt.

20. Verfahren nach einem der Ansprüche 7 bis 19,
**dadurch gekennzeichnet, dass** der Vektor durch Transformation, Transfektion, Injektion, biolistische Methoden und/oder Elektroporation auf die Pflanzen übertragen wird.

21. Verfahren nach einem der Ansprüche 1 bis 20,
**dadurch gekennzeichnet, dass** die pflanzlichen DnaJ-ähnlichen Proteine eine J-Domäne enthalten, die mindestens zu 40%, bevorzugt mindestens zu 50%, besonders bevorzugt mindestens zu 60%, insbesondere bevorzugt mindestens zu 70%, ebenfalls insbesondere bevorzugt mindestens zu 80% und am meisten bevorzugt bis zu 90% homolog zu der Aminosäuresequenz Seq ID No. 7 ist.

22. Verfahren nach einem der Ansprüche 1 bis 20,
**dadurch gekennzeichnet, dass** die pflanzlichen DnaJ-ähnlichen Proteine durch die Sequenzen Seq ID Nos 1, 2, oder 3 kodiert werden.

23. Verfahren nach einem der Ansprüche 1 bis 20,
**dadurch gekennzeichnet, dass** die pflanzlichen DnaJ-ähnlichen Proteine mindestens zu 40%, bevorzugt mindestens zu 50%, besonders bevorzugt mindestens zu 60%, insbesondere bevorzugt mindestens zu 70%, ebenfalls insbesondere bevorzugt mindestens zu 80% und am meisten bevorzugt mindestens zu 90% homolog zu der Aminosäuresequenz Seq ID Nos. 4, 5 oder 6 sind.

24. Verfahren nach einem der Ansprüche 1 bis 23,
**dadurch gekennzeichnet, dass** die transgenen Pflanzen eine erhöhte Resistenz gegen Poty-, Carla-, Hordei-, Potex-, Bunyai-, Clostero- , Cucumo-, Diantho-, Tobamo-, Gemini-, Lutero-, Rymo-, Tobra-, Furo-, Como-, Nepo-, Bromo- oder Tospoviren zeigen.

25. Verfahren nach Anspruch 24,
**dadurch gekennzeichnet, dass** die Pflanzen eine erhöhte Resistenz gegen PVY, gegen TEV und/oder gegen TSWV. zeigen.

26. Verfahren nach einem der Ansprüche 1 bis 25,
**dadurch gekennzeichnet, dass** es sich bei den transgenen Pflanzen um monokotyle Pflanzen, bevorzugt um Pflanzen, die zu den Gattungen Avena (Hafer), Triticum (Weizen), Secale (Roggen), Hordeum (Gerste), Oryza (Reis), Panicum, Pennisetum, Setaria, Sorghum (Hirse), Zea (Mais) und dergleichen gehören, handelt.

27. Verfahren nach einem der Ansprüche 1 bis 25,
**dadurch gekennzeichnet, dass** es sich bei den transgenen Pflanzen um dikotyle Pflanzen, bevorzugt um Baumwolle, Leguminosen wie Hülsenfrüchte und insbesondere Alfalfa, Sojabohne, Raps, Tomate, Zuckerrübe, Kartoffel, Zierpflanzen, Tabak sowie Bäume handelt.

28. Transgene Pflanzenzelle oder Pflanze mit erhöhter Virusresistenz, hergestellt durch ein Verfahren nach einem der Ansprüche 1 bis 27.

29. Transgene Pflanzenzelle oder Pflanze mit erhöhter Virusresistenz,
**dadurch gekennzeichnet, dass** die Expression von pflanzlichen DnaJ-ähnlichen Proteinen, die mit Viruskomponenten interagieren, im Wesentlichen unterbunden ist.

30. Transgene Pflanzenzelle oder Pflanze nach Anspruch 29
**dadurch gekennzeichnet, dass** die pflanzlichen DnaJ-ähnlichen Proteinen durch die Seq ID Nos. 1, 2 oder 3 kodiert werden.

31. Transgene Pflanzenzelle oder Pflanze nach Anspruch 29
**dadurch gekennzeichnet, dass** die pflanzlichen DnaJ-ähnlichen Proteine mindestens zu 30%, bevorzugt mindestens zu 40%, besonders bevorzugt mindestens zu 50%, ebenfalls besonders bevorzugt mindestens zu 60%, insbesondere bevorzugt mindestens zu 70%, ebenfalls insbesondere bevorzugt mindestens zu 80% und am meisten bevorzugt mindestens zu 90% homolog zu der Aminosäuresequenz Seq ID Nos. 4, 5, oder 6 sind.

32. Transgene Pflanzenzelle oder Pflanze mit erhöhter Virusresistenz,
**dadurch gekennzeichnet, dass** dominant-negative Mutanten von pflanzlichen DnaJ-ähnlichen Proteinen, die mit Viruskomponenten interagieren, oder für DnaJ-ähnliche Proteine spezifische Antikörper in den Pflanzellen bzw. der Pflanze exprimiert werden.

33. Transgene Pflanzenzelle oder Pflanze mit erhöhter Virusresistenz,
**dadurch gekennzeichnet, dass** dominant-negative Mutanten von DnaJ-ähnlichen Proteinen und bevorzugt von pflanzlichen DnaJ-ähnlichen Proteinen, die mit Viruskomponenten interagieren, in den Pflanzellen bzw. der Pflanze exprimiert werden.

34. Transgene Pflanzenzelle oder Pflanze nach Anspruch 32 oder 33,
**dadurch gekennzeichnet, dass** die pflanzlichen DnaJ-ähnlichen Proteinen durch die Seq ID No 1, 2 oder 3 kodiert werden.

35. Transgene Pflanzenzelle oder Pflanze nach Anspruch 32 oder 33,
**dadurch gekennzeichnet, dass** die pflanzlichen DnaJ-ähnlichen Proteine mindestens zu 40%, bevorzugt mindestens zu 50%, besonders bevorzugt mindestens zu 60%, insbesondere bevorzugt mindestens zu 70%, ebenfalls insbesondere bevorzugt mindestens zu 80% und am meisten bevorzugt mindestens zu 90% homolog zu der Aminosäuresequenz Seq ID Nos. 4, 5 oder 6 sind.

36. Transgene Pflanzenzelle oder Pflanze nach einem der Ansprüche 32-34,
**dadurch gekennzeichnet, dass** als dominant-negative Mutanten der DnaJ-ähnlichen Proteine und bevorzugt der pflanzlichen DnaJ-ähnlichen Proteine Proteine exprimiert werden, bei denen die J-Domäne deletiert ist.

37. Transgene Pflanzenzelle oder Pflanze nach einem der Ansprüche 32-36,
**dadurch gekennzeichnet, dass** als dominant-negative Mutanten N-terminale Deletionsmutanten der pflanzlichen DnaJ-ähnlichen Proteine, die durch die Seq ID Nos 1, 2 oder 3 kodiert werden, exprimiert werden, bei denen die J-Domäne deletiert ist.

38. Transgene Pflanzenzelle oder Pflanze mit erhöhter Virusresistenz,
**dadurch gekennzeichnet, dass** die J-Domäne von pflanzlichen DnaJ-ähnlichen Proteinen, die mit viralen Komponenten interagieren, überexprimiert wird.

39. Virusresistente Pflanzenzelle oder Pflanze,
**dadurch gekennzeichnet, dass** sie in den kodierenden und/oder regulatorischen Sequenzen von Genen für pflanzliche DnaJ-ähnliche Proteine Mutationen tragen, die eine Modulation der Expression dieser Proteine und/oder deren Bindungsverhalten gegenüber viralen Proteinen und/oder endogenen zellulären Bindungspartnern bewirken.

40. Virusresistente Pflanzenzelle oder Pflanze nach Anspruch 39.
**dadurch gekennzeichnet, dass** eine dominant-negative Mutante von pflanzlichen DnaJ-ähnlichen Proteinen exprimiert wird, die nicht in der Lage ist, mit viralen Komponenten und/oder den zellulären Interaktionspartnem von pflanzlichen DnaJ-ähnlichen Proteinen zu interagieren.

41. Virusresistente Pflanzenzelle oder Pflanze nach Anspruch 39 oder 40,
**dadurch gekennzeichnet, dass** sie durch das "TILLING"-Verfahren herstellbar sind.

## Revendications

1. Procédé de production de plantes transgéniques à résistance élevée aux virus,
**caractérisé en ce que** l'expression de protéines végétales similaires à l'AdnJ interagissant avec des composants viraux est essentiellement inhibée.

2. Procédé selon la revendication 1,
**caractérisé en ce que** l'expression de protéines végétales similaires à l'AdnJ est essentiellement inhibée par transfert de molécules d'acide nucléique comprenant des séquences qui sont identiques, homologues ou complémentaires aux séquences codant pour des protéines végétales similaires à l'AdnJ ou des parties de celles-ci sur des cellules végétales.

3. Procédé selon la revendication 1 ou 2,
**caractérisé en ce que** les molécules d'acide nucléique transférées comprennent des séquences qui sont identiques, homologues ou complémentaires aux séquences codant pour la domaine J des protéines végétales similaires à l' AdnJ ou des parties de celle-ci.

4. Procédé selon la revendication 1 ou 2,
**caractérisé en ce que** les molécules d'acide nucléique transférées comprennent des séquences qui sont identiques, homologues ou complémentaires à des séquences codant pour les régions de protéine spécifiques de protéines végétales similaires à l'AdnJ, ne coïncidant pas avec la domaine J, ou des parties de celles-ci.

5. Procédé selon l'une des revendications précédentes,
**caractérisé en ce que** la partie des séquences d'acide nucléique transférées qui est identique, homologue ou complémentaire aux séquences codant pour des protéines végétales similaires à l'AdnJ ou des parties de celles-ci comprend de 20 à 1000 nucléotides, de 20 à 750 nucléotides, de préférence de 20 à 500 nucléotides, également de préférence de 20 à 250 nucléotides, de manière particulièrement préférentielle de 20 à 150 nucléotides, de manière tout particulièrement préférentielle de 20 à 100 nucléotides et de la manière la plus préférentielle d'environ 20 à environ 50 nucléotides.

6. Procédé selon l'une des revendications précédentes,
**caractérisé en ce qu'**une partie des séquences d'acide nucléique transférées est homologue pour au moins 50 %, de préférence au moins 60 %, également de préférence au moins 70 %, de manière particulièrement préférentielle au moins 80 %, de manière tout particulièrement préférentielle au moins 90 % et de la manière la plus préférentielle au moins 95 % aux séquences codant pour des protéines végétales similaires à l'AdnJ ou des parties de celles-ci.

7. Procédé selon l'une des revendications précédentes,
**caractérisé en ce qu'**une partie des séquences d'acide nucléique transférées est complémentaire pour au moins 50 %, de préférence au moins 60 %, également de préférence au moins 70 %, de manière particulièrement préférentielle au moins 80 %, de manière tout particulièrement préférentielle au moins 90 % et de la manière la plus préférentielle au moins 95 % aux séquences codant pour des protéines végétales similaires à l'AdnJ ou des parties de celles-ci.

8. Procédé selon l'une des revendications précédentes,
**caractérisé en ce qu'**il comprend les étapes suivantes :
a) production d'un vecteur qui comprend, dans l'orientation 5'-3', les parties de séquence suivantes :
- un promoteur fonctionnel dans des plantes,
- en liaison opérationnelle avec celui-ci, la séquence *antisense* identique ou homologue de la séquence codant pour la protéine végétale similaire à l'AdnJ ou des parties de celle-ci, la séquence présentant, à son extrémité 3', des séquences 3' d'exon reconnaissables par le splicéosome,
- un intron,
- la séquence *sense* identique ou homologue de la séquence codant pour la protéine végétale similaire à l'AdnJ ou des parties de celle-ci, la séquence présentant, à son extrémité 5', des séquences 5' d'exon reconnaissables par le splicéosome,
- une séquence de terminaison ;
b) transfert du vecteur issu de a) sur des cellules végétales et, le cas échéant, intégration dans le génome végétal.

9. Procédé selon l'une des revendications 1 à 7,
**caractérisé en ce qu'**il comprend les étapes suivantes :
a) production d'un vecteur qui comprend, dans l'orientation 5'-3', les parties de séquence suivantes :
- un promoteur fonctionnel dans des plantes,
- en liaison opérationnelle avec celui-ci, la séquence *antisense* identique ou homologue de la séquence codant pour une protéine végétale similaire à l'AdnJ ou des parties de celle-ci,
- une séquence de terminaison ;
b) transfert du vecteur issu de a) sur des cellules végétales et, le cas échéant, intégration dans le génome végétal.

10. Procédé selon l'une des revendications 1 à 7,
**caractérisé en ce qu'**il comprend les étapes suivantes :
a) production d'un vecteur qui comprend, dans l'orientation 5'-3', les parties de séquence suivantes :
- un promoteur fonctionnel dans des plantes,
- en liaison opérationnelle avec celui-ci, la séquence *sense* identique ou homologue de la séquence codant pour une protéine végétale similaire à l'AdnJ ou des parties de celle-ci, la séquence disposant de régions auto-complémentaires,
- une séquence de terminaison ;
b) transfert du vecteur issu de a) sur des cellules végétales et, le cas échéant, intégration dans le génome végétal.

11. Procédé selon l'une des revendications 1 à 7,
**caractérisé en ce qu'**il comprend les étapes suivantes :
a) production d'un vecteur qui comprend, dans l'orientation 5'-3', les parties de séquence suivantes :
- un promoteur fonctionnel dans des plantes,
- une séquence ADN en liaison opérationnelle avec celui-ci, qui est complémentaire à la séquence codant pour l'ARNm de la protéine végétale similaire à l'AdnJ ou des parties de celle-ci,
- une séquence ADN codant pour la ribonucléase P,
- une séquence de terminaison ;
b) transfert du vecteur issu de a) sur des cellules végétales, et le cas échéant, intégration dans le génome végétal.

12. Procédé selon l'une des revendications 1 à 7,
**caractérisé en ce qu'**il comprend les étapes suivantes :
a) production d'un vecteur qui comprend, dans l'orientation 5'-3', les parties de séquence suivantes :
- un promoteur fonctionnel dans des plantes,
- une séquence ADN qui est identique ou homologue à la séquence codant pour l'extrémité 5' de la protéine végétale similaire à l'AdnJ,
- une séquence ADN codant pour un gène de résistance,
- une séquence ADN qui est identique ou homologue à la séquence codant pour l'extrémité 3' de la protéine végétale similaire à l'AdnJ,
- une séquence de terminaison ;
b) transfert du vecteur issu de a) sur des cellules végétales et intégration dans le génome végétal.

13. Procédé selon l'une des revendications 1 à 7, comprenant les étapes suivantes :
a) production d'un vecteur qui comprend, dans l'orientation 5'-3', les parties de séquence suivantes :
- un promoteur fonctionnel dans des plantes,
- en liaison opérationnelle avec celui-ci, une séquence ADN codant pour un ribozyme qui reconnaît spécifiquement l'ARNm des protéines végétales similaires à l'AdnJ,
- une séquence de terminaison ;
b) transfert du vecteur issu de a) sur des cellules végétales et, le cas échéant, intégration dans le génome végétal.

14. Procédé de production de plantes transgéniques à résistance élevée aux virus, comprenant les étapes suivantes :
a) production d'un vecteur qui comprend, dans l'orientation 5'-3', les parties suivantes :
- un promoteur fonctionnel dans des plantes,
- en liaison opérationnelle avec celui-ci, une séquence ADN codant pour un mutant dominant négatif d'une protéine végétale similaire à l'AdnJ qui interagit avec des composants viraux, ou un anticorps recombinant qui est spécifique pour des protéines végétales similaires à l'AdnJ
- un signal de terminaison ;
b) transfert du vecteur issu de a) sur les cellules végétales et, le cas échéant, intégration dans le génome végétal.

15. Procédé selon l'une des revendications 7 à 14,
**caractérisé en ce que** le vecteur contient, en plus des promoteurs, d'autres séquences régulatrices et fonctionnelles.

16. Procédé selon la revendication 15,
**caractérisé en ce qu'**il s'agit, en ce qui concerne les séquences régulatrices, d'enhancers, de signaux de réplication, de marqueurs de sélection et/ou de séquences qui permettent une propagation des vecteurs dans des bactéries et/ou une réplication dans des cellules végétales.

17. Procédé selon l'une des revendications 7 à 16,
**caractérisé en ce qu'**il s'agit, en ce qui concerne les vecteurs, de plasmides, de cosmides et/ou de virus recombinants.

18. Procédé selon la revendication 17,
**caractérisé en ce qu'**il s'agit, en ce qui concerne les vecteurs, de pBR322, de vecteurs pUC, de vecteurs M13mp ou de vecteurs qui sont dérivés du plasmide Ti ou Ri d'agrobactéries.

19. Procédé selon l'une des revendications 7 à 18,
**caractérisé en ce qu'**il s'agit, en ce qui concerne le promoteur, de promoteurs constitutifs, de préférence du promoteur 35S, du promoteur d'actine ou d'ubiquitine, de promoteurs spécifiques aux tissus, de préférence du promoteur phosphoénolpyruvate-carboxylase ou fructose-1,6-bisphosphatase, de promoteurs spécifiques au développement, de promoteurs induits par la lumière, par des lésions ou par un pathogène, de préférence de promoteurs induits par un virus.

20. Procédé selon l'une des revendications 7 à 19,
**caractérisé en ce que** le vecteur est transféré à la plante par transformation, transfection, injection, méthodes biolistiques et/ou électroporation.

21. Procédé selon l'une des revendications 1 à 20,
**caractérisé en ce que** les protéines végétales similaires à l'AdnJ contiennent un domaine J qui est homologue pour au moins 40 %, de préférence au moins 50 %, de manière particulièrement préférentielle au moins 60 %, de manière tout particulièrement préférentielle au moins 70 %, également de manière tout particulièrement préférentielle au moins 80 % et de la manière la plus préférentielle jusqu'à 90 % à la séquence d'acides aminés Seq ID No. 7.

22. Procédé selon l'une des revendications 1 à 20,
**caractérisé en ce que** les protéines végétales similaires à l'AdnJ sont codées par les séquences Seq ID Nos. 1, 2 ou 3.

23. Procédé selon l'une des revendications 1 à 20,
**caractérisé en ce que** les protéines végétales similaires à l'AdnJ sont homologues pour au moins 40 %, de préférence au moins 50 %, de manière particulièrement préférentielle au moins 60 %, de manière tout particulièrement préférentielle au moins 70 %, également de manière tout particulièrement préférentielle au moins 80 % et de la manière la plus préférentielle au moins 90 % à la séquence d'acides aminés Seq ID No. 4, 5 ou 6.

24. Procédé selon l'une des revendications 1 à 23,
**caractérisé en ce que** les plantes transgéniques présentent une résistance élevée aux virus poty, carla, hordei, potex, bunyai, clostero, cucumo, diantho, tobamo, gemini, lutero, rymo, tobra, furo, como, nepo, bromo ou tospo.

25. Procédé selon la revendication 24,
**caractérisé en ce que** les plantes présentent une résistance élevée au PVY, au TEV et/ou au TSWV.

26. Procédé selon l'une des revendications 1 à 25,
**caractérisé en ce qu'**il s'agit, en ce qui concerne les plantes transgéniques, de plantes monocotyles, de préférence de plantes qui appartiennent aux genres Avena (avoine), Triticum (blé), Secale (seigle), Hordeum (orge), Oryza (riz), Panicum, Pennisetum, Setaria, Sorghum (millet), Zea (maïs) et similaires.

27. Procédé selon l'une des revendications 1 à 25,
**caractérisé en ce qu'**il s'agit, en ce qui concerne les plantes transgéniques, de plantes dicotyles, de préférence de coton, de légumineuses comme des légumes secs et notamment la luzerne, le soja, le colza, la tomate, la betterave à sucre, la pomme de terre, les plantes d'ornement, le tabac ainsi que les arbres.

28. Cellule végétale ou plante transgénique à résistance élevée aux virus, produite par un procédé selon l'une des revendications 1 à 27.

29. Cellule végétale ou plante transgénique à résistance élevée aux virus,
**caractérisé en ce que** l'expression de protéines végétales similaires à l'AdnJ interagissant avec des composants viraux est essentiellement inhibée.

30. Cellule végétale ou plante transgénique selon la revendication 29,
**caractérisé en ce que** les protéines végétales similaires à l'AdnJ sont codées par les Seq ID Nos. 1, 2 ou 3.

31. Cellule végétale ou plante transgénique selon la revendication 29,
**caractérisé en ce que** les protéines végétales similaires à l'AdnJ sont homologues pour au moins 30 %, de préférence au moins 40 %, de manière particulièrement préférentielle au moins 50 %, également de manière particulièrement préférentielle au moins 60 %, de manière tout particulièrement préférentielle au moins 70 %, également de manière tout particulièrement préférentielle au moins 80 % et de manière la plus préférentielle au moins 90 % à la séquence d'acides aminés Seq ID Nos. 4, 5 ou 6.

32. Cellule végétale ou plante transgénique à résistance élevée aux virus,
**caractérisé en ce que** des mutants dominants négatifs de protéines végétales similaires à l'AdnJ interagissant avec des composants viraux ou d'anticorps spécifiques pour des protéines similaires à l'AdnJ sont exprimés dans les cellules végétales ou la plante.

33. Cellule végétale ou plante transgénique à résistance élevée aux virus,
**caractérisé en ce que** les mutants dominants négatifs des protéines similaires à l'AdnJ et de préférence des protéines végétales similaires à l'AdnJ interagissant avec les composants viraux sont exprimés dans les cellules végétales ou la plante.

34. Cellule végétale ou plante transgénique selon la revendication 32 ou 33,
**caractérisé en ce que** les protéines végétales similaires à l'AdnJ sont codées par la Seq ID No. 1, 2 ou 3.

35. Cellule végétale ou plante transgénique selon la revendication 32 ou 33,
**caractérisé en ce que** les protéines végétales similaires à l'AdnJ sont homologues pour au moins 40 %, de préférence au moins 50 %, de manière particulièrement préférentielle au moins 60 %, de manière tout particulièrement préférentielle au moins 70 %, également de manière tout particulièrement préférentielle au moins 80 % et de la manière la plus préférentielle au moins 90 % à la séquence d'acides aminés Seq ID Nos. 4, 5 ou 6.

36. Cellule végétale ou plante transgénique selon l'une des revendications 32 - 34,
**caractérisé en ce que** des protéines, dans lesquelles le domaine J est éliminé, sont exprimées comme mutants dominants négatifs des protéines similaires à l'AdnJ et de préférence des protéines végétales similaires à l'AdnJ.

37. Cellule végétale ou plante transgénique selon l'une des revendications 32 - 36,
**caractérisé en ce que** des mutants de délétion N-terminale des protéines végétales similaires à l'AdnJ, qui sont codées par les Seq ID Nos. 1, 2 ou 3, sont exprimés comme mutants dominants négatifs dans lesquelles le domaine J est éliminé.

38. Cellule végétale ou plante transgénique à résistance élevée aux virus,
**caractérisé en ce que** le domaine J des protéines végétales similaires à l'AdnJ interagissant avec des composants viraux est surexprimé.

39. Cellule végétale ou plante résistant aux virus,
**caractérisé en ce qu'**elles présentent des mutations dans les séquences codantes et/ou régulatrices des gènes pour des protéines végétales similaires à l'AdnJ, lesquelles mutations provoquent une modulation de l'expression de ces protéines et/ou de leurs caractéristiques de liaison vis-à-vis des protéines virales et/ou des partenaires endogènes cellulaires de liaison.

40. Cellule végétale ou plante résistant aux virus selon la revendication 39,
**caractérisé en ce qu'**un mutant dominant négatif de protéines végétales similaires à l'AdnJ est exprimé, qui n'est pas en mesure d'interagir avec des composants viraux et/ou les partenaires cellulaires d'interaction des protéines végétales similaires à l'AdnJ.

41. Cellule végétale ou plante résistant aux virus selon la revendication 39 ou 40,
**caractérisé en ce qu'**elles peuvent être produites par le procédé de « TILLING ».

## Claims

1. A method for the production of transgenic plants with increased virus resistance,
**characterized in that**
the expression of plant DnaJ-like proteins, which interact with viral components, is substantially prevented.

2. The method according to claim 1,
**characterized in that** the expression of plant DnaJ-like proteins is substantially prevented by means of transfer of nucleic acid molecules comprising sequences, which are identical, homologous or complementary to the sequences encoding plant DnaJ-like proteins or fragments thereof, to plant cells.

3. The method according to claim 1 or 2,
**characterized in that** the transferred nucleic acid molecules comprise sequences, which are identical, homologous or complementary to the sequences encoding the J-domain of plant DnaJ-like proteins or fragments thereof.

4. The method according to claim 1 or 2,
**characterized in that** the transferred nucleic acid molecules comprise sequences, which are identical, homologous or complementary to the sequences encoding the protein specific regions of plant DnaJ-like proteins, which do not coincide with the J-domain, or fragments thereof.

5. The method according to one of the preceding claims,
**characterized in that** the fragment of the transferred nucleic acid sequences, which is identical, homologous or complementary to the sequences encoding plant DnaJ-like proteins or fragments thereof, comprises 20 to 1000 nucleotides, 20 to 750 nucleotides, preferably 20 to 500 nucleotides, also preferably 20 to 250 nucleotides, especially preferably 20 to 150 nucleotides, particularly preferably 20 to 100 nucleotides, and most preferably about 20 - 50 nucleotides.

6. The method according to one of the preceding claims,
**characterized in that** a fragment of the transferred nucleic acid sequences is at least 50% homologous to the sequences encoding plant DnaJ-like proteins or fragments thereof, preferably at least 60%, also preferably at least 70%, especially preferably at least 80%, particularly preferably at least 90%, and most preferably at least 95%.

7. The method according to one of the preceding claims,
**characterized in that** a fragment of the transferred nucleic acid sequences is at least 50% complementary to the sequences encoding plant DnaJ-like proteins or fragments thereof, preferably at least 60%, also preferably at least 70%, especially preferably at least 80%, particularly preferably at least 90%, and most preferably at least 95%.

8. The method according to one of the preceding claims,
**characterized in that** it comprises the following steps:
a) producing a vector which comprises the following sequence elements in 5'-3' orientation:
- a promoter being functional in plants
- operatively linked thereto the identical or homologous antisense sequence of the sequence encoding the plant DnaJ-like protein or fragments thereof, wherein the sequence exhibits 3' exon sequences at its 3'end, being recognizable by the splicosome
- an intron
- the identical or homologous *sense* sequence of the sequence encoding the plant DnaJ-like protein or fragments thereof, wherein the sequence exhibits 5'exon sequences at its 5'end, being recognizable by the splicosome
- a termination sequence
b) transferring the vector from a) to plant cells and, optionally, integration into the plant genome.

9. The method according to one of the claims 1 to 7,
**characterized in that** it comprises the following steps:
a) producing a vector which comprises the following sequence elements in 5'-3' orientation:
- a promoter being functional in plants
- operatively linked thereto the identical or homologous antisense sequence of the sequence encoding a plant DnaJ-like protein or fragments thereof
- a termination sequence
b) transferring the vector from a) to plant cells and, optionally, integration into the plant genome.

10. The method according to one of the claims 1 to 7,
**characterized in that** it comprises the following steps:
a) producing a vector which comprises the following sequence elements in 5'-3' orientation:
- a promoter being functional in plants
- operatively linked thereto the identical or homologous sense sequence of the sequence encoding a plant DnaJ-like protein or fragments thereof, wherein the sequence has self-complementary regions
- a termination sequence
b) transferring the vector from a) to plant cells and, optionally, integration into the plant genome.

11. The method according to one of the claims 1 to 7,
**characterized in that** it comprises the following steps:
a) producing a vector which comprises the following sequence elements in 5'-3' orientation:
- a promoter being functional in plants
- operatively linked thereto a DNA sequence, which is complementary to the sequence encoding the mRNA of the plant DnaJ-like protein or fragments thereof
- a DNA sequence encoding ribonuclease P
- a termination sequence
b) transferring the vector from a) to plant cells and, optionally, integration into the plant genome.

12. The method according to one of the claims 1 to 7,
**characterized in that** it comprises the following steps:
a) producing a vector which comprises the following sequence elements in 5'-3' orientation:
- a promoter being functional in plants
- a DNA sequence which is identical or homologous to the sequence encoding the 5'end of the plant DnaJ-like protein
- a DNA sequence encoding a resistance gene
- a DNA sequence which is identical or homologous to the sequence encoding the 3'end of the plant DnaJ-like protein
- a termination sequence
b) transferring the vector from a) to plant cells and integration into the plant genome.

13. The method according to one of the claims 1 to 7, comprising the following steps:
a) producing a vector which comprises the following sequence elements in 5'-3' orientation:
- a promoter being functional in plants
- operatively linked thereto a DNA sequence encoding a ribozyme which specifically recognizes the mRNA of plant DnaJ-like proteins
- a termination sequence
b) transferring the vector from a) to plant cells and, optionally, integration into the plant genome.

14. A method for the production of transgenic plants with increased virus resistance, comprising the following steps
a) producing a vector which comprises the following elements in 5'-3' orientation:
- a promoter being functional in plants
- operatively linked thereto a DNA sequence encoding a dominant-negative mutant of a plant DnaJ-like protein, which interacts with viral components, or a recombinant antibody, which is specific for plant DnaJ-like proteins
- a termination signal
b) transferring the vector from a) to plant cells and, optionally, integration into the plant genome.

15. The method according to one of the claims 7 to 14,
**characterized in that** the vector contains further regulatory and functional sequences in addition to promoters.

16. The method according to claim 15,
**characterized in that** the regulatory sequences are enhancers, replication signals, selection markers and/or sequences allowing for a propagation of the vectors in bacteria and/or a replication in plant cells.

17. The method according to one of the claims 7 to 16,
**characterized in that** the vectors are plasmids, cosmids and/or recombinant viruses.

18. The method according to claim 17,
**characterized in that** the vectors are pBR322, pUC vectors, M13mp vectors or vectors derived from the Agrobacterium Ti or Ri plasmid.

19. The method according to one of the claims 7 to 18,
**characterized in that** the promoters are constitutive promoters, preferably the 35S, the actin or the ubiquitin promoter, tissue-specific promoters, preferably the phosphoenolpyruvate carboxylase or the fructose-1,6-bisphosphatase promoter, developmental-specific, light-, wound- or pathogen-induced promoters, preferably virus-induced promoters.

20. The method according to one of the claims 7 to 19,
**characterized in that** the vector is transferred to the plants by means of transformation, transfection, injection, biolistic methods and/or electroporation.

21. The method according to one of the claims 1 to 20,
**characterized in that** the plant DnaJ-like proteins contain a J-domain, which is at least 40% homologous to the amino acid sequence SEQ ID NO. 7, preferably at least 50%, especially preferably at least 60%, particularly preferably at least 70%, also particularly preferably at least 80%, and most preferably up to 90%.

22. The method according to one of the claims 1 to 20,
**characterized in that** the plant DnaJ-like proteins are encoded by the sequences SEQ ID NO. 1,2 or 3.

23. The method according to one of the claims 1 to 20,
**characterized in that** the plant DnaJ-like proteins are at least 40% homologous to the amino acid sequence SEQ ID NO. 4, 5 or 6, preferably at least 50%, especially preferably at least 60%, particularly preferably at least 70%, also particularly preferably at least 80%, and most preferably at least 90%.

24. The method according to one of the claims 1 to 23,
**characterized in that** the transgenic plants exhibit increased resistance to Poty, Carla, Hordei, Potex, Bunyai, Clostero, Cucumo, Diantho, Tobamo, Gemini, Lutero, Rymo, Tobra, Furo, Como, Nepo, Bromo or Tospo viruses.

25. The method according to claim 24,
**characterized in that** the plants exhibit increased resistance to PVY, to TEV and/or to TSWV.

26. The method according to one of the claims 1 to 25,
**characterized in that** the transgenic plants are monocotyledonous plants, preferably plants belonging to the genera Avena (oat), Triticum (wheat), Secale (rye), Hordeum (barley), Oryza (rice), Panicum, Pennisetum, Setaria, Sorghum (millet), Zea (maize) and the like.

27. The method according to one of the claims 1 to 25,
**characterized in that** the transgenic plants are dicotyledonous plants, preferably cotton, leguminous plants such as legumes and especially alfalfa, soy bean, rape, tomato, sugar beet, potato, ornamental plants, tobacco as well as trees.

28. Transgenic plant cell or plant with increased virus resistance, produced by a method according to one of the claims 1 to 27.

29. Transgenic plant cell or plant with increased virus resistance,
**characterized in that** the expression of plant DnaJ-like proteins, which interact with virus components, is substantially prevented.

30. Transgenic plant cell or plant according to claim 29,
**characterized in that** the plant DnaJ-like proteins are encoded by SEQ ID NO. 1, 2 or 3.

31. Transgenic plant cell or plant according to claim 29,
**characterized in that** the plant DnaJ-like proteins are at least 30% homologous to the amino acid sequence SEQ ID NO. 4, 5 or 6, preferably at least 40%, especially preferably at least 50%, also especially preferably at least 60%, particularly preferably at least 70%, also particularly preferably at least 80%, and most preferably at least 90%.

32. Transgenic plant cell or plant with increased virus resistance,
**characterized in that** dominant-negative mutants of plant DnaJ-like proteins, which interact with virus components, or antibodies being specific for DnaJ-like proteins are expressed in the plant cells or the plant.

33. Transgenic plant cell or plant with increased virus resistance,
**characterized in that** dominant-negative mutants of DnaJ-like proteins and preferably of plant DnaJ-like proteins, which interact with virus components, are expressed in the plant cells or the plant.

34. Transgenic plant cell or plant according to claim 32 or 33,
**characterized in that** the plant DnaJ-like proteins are encoded by SEQ ID NO. 1, 2 or 3.

35. Transgenic plant cell or plant according to claim 32 or 33,
**characterized in that** the plant DnaJ-like proteins are at least 40% homologous to the amino acid sequence SEQ ID NO. 4, 5 or 6, preferably at least 50%, especially preferably at least 60%, particularly preferably at least 70%, also particularly preferably at least 80%, and most preferably at least 90%.

36. Transgenic plant cell or plant according to one of the claims 32-34,
**characterized in that** proteins are expressed as dominant-negative mutants of the DnaJ-like proteins and preferably of the plant DnaJ-like proteins, in which the J-domain is deleted.

37. Transgenic plant cell or plant according to one of the claims 32-36,
**characterized in that** N-terminal deletion mutants of the plant DnaJ-like proteins, which are encoded by SEQ ID NO. 1, 2 or 3, are expressed as dominant-negative mutants, in which the J-domain is deleted.

38. Transgenic plant cell or plant with increased virus resistance **characterized in that** the J-domain of plant DnaJ-like proteins which interact with viral components is overexpressed.

39. Virus resistant plant cell or plant,
**characterized in that** they contain mutations in the coding and/or regulatory sequences of the genes for plant DnaJ-like proteins, which cause a modulation of the expression of these proteins and/or their binding behavior to viral proteins and/or endogenous cellular binding partners.

40. Virus resistant plant cell or plant according to claim 39,
**characterized in that** a dominant-negative mutant of plant DnaJ-like proteins is expressed, which is not able to interact with viral components and/or the cellular interaction partners of plant DnaJ-like proteins.

41. Virus resistant plant cell or plant according to claim 39 or 40,
**characterized in that** they can be produced by the "TILLING" method.


### SEQUENZPROTOKOLL

<110> Institut für Pflanzengenetik und Kulturpflanzenfor
<120> Verfahren zur Herstellung von transgenen Pflanzen mit erhöhter Virusresistenz durch "Silencing" pflanzlicher DnaJ-ähnlicher Proteine
<130> I7404
<140>
   <141>
<160> 10
<170> PatentIn Ver. 2.1
<210> 1
   <211> 1371
   <212> DNA
   <213> Nicotiana tabacum
<220>
   <221> gene
   <222> (61)..(978)
   <223> NtCPIP1
<400> 1
   gcaaaagagc gagagagaga gagagagaga gagagagaga gagagagaga gagagagaaa 60
   atgggcgttg attactacaa aatactcaaa gtgtcacgca atgcgagtga agaagatttg 120
   aaaaaatcgt acaagcgatt ggcgatgaaa tggcatccgg ataagaacag tgagaaagaa 180
   gcagaagcga aattcaagca gatatcggag gcctatgatg tgctaagtga tccacagaag 240
   cgtcagatct atgatatata cggcgatgag gcgttgaaat cgggtcaatt cgatccctcg 300
   tcacctatga atggtaatgg gagaggattt aagttcgatt cgcgtgatgc ggaagatatt 360
   tttgcggaat tttttggtgg atcggatggg tatagtagga gtcctactgg tggtactgta 420
   cggattagga agccggcgcc ggtggagaac aagttgccat gtagcttgga ggaattgtac 480
   aagggttcta agaggaagat gaagatttca aggattgttc ttgatgtcac tggtaagcct 540
   acaacaattg aagaggtctt ggcaatacac attaaacctg gttggaagaa aggcacaaaa 600
   atcacttttc cagagaaagg gaaccatgaa cctggagctg cacctggtga tcttattttt 660
   gtaatcgatg aaaagccaca tgatgtcttc aagagagatg gaaatgatct agtgatcaat 720
   cagaaaatct cattagtaga tgctctctct gggaagatta tcaacttggc tactttggat 780
   ggaagggaac tcacgatacc aatcacagat gttgttaagc caggacatga gcagataatc 840
   gcagatgaag gaatgccaat atcaaaagaa cccgggaaga aaggaaattt gaggatcaag 900
   tttgaggtta agttcccgtc aaggcttagt tcagatcaga aattggatat cagaagagtg 960
   ctgggcagga ctgttgacta atccagtgct taacttaagg tatcactaac actgactgat 1020
   tgtggaagtg caatggaatg taaatacttg gaaagtttat gctagacagg agaattgtgc 1080
   atagtaggat tattgttttg acgatgcctc tcaaagttta gttttattta gcgccataaa 1140
   aggagcactc tagcagctat gccagctgcc tatgtaattc tcatgttgga tattaaacac 1200
   cttaggtagt ttactaagtt ttaatgtttg atttggaggt agatgctata cttcaagtaa 1260
   ttggagtcgg gaatagcttg ctatcttgtt ctggttgtga atttcaactg tgggctgtgg 1320
   tagactggta atgaataaaa aaaaaaaaaa aaaaaaaaaa aaaaaaaaaa a          1371
<210> 2
   <211> 918
   <212> DNA
   <213> Nicotiana tabacum
<220>
   <221> gene
   <222> (1)..(918)
   <223> NtCPIP2a
<400> 2
   atgggacttg attactataa cgtactaaaa gtatctcgga atgcaagtga agaagattta 60
   aagagatcgt acaagcgatt agctatgaaa tggcatccag ataagaacag tcagaacaaa 120
   aaagaagcag aagcaaaatt caagcagatt tctgaagcgt atgatgtgct tagtgatcct 180
   aagaagcgtc agatctatga cgtgtacggt gatgatgcat tgaaatccgg tcaatttgcc 240
   tcggcgtcgc cgactagtgc tggtagtaac gccagagggt ttaggttcaa tacgcgtgat 300
   gcggaggcta tttttgctga gtttttcggt ggatcgggta gtaattccgg tgccggagtc 360
   ggtcggaagg cggcgccggt ggagaataaa ctgccgtgta gcttggagga gctttacaaa 420
   ggttctagaa gaaaaatgaa gatctcacgg attcttctgg atgactctgg taagcctaca 480
   actgttgaag aggtcttagc gatacacatc aagcctggtt ggaagaaagg cacaaaaatc 540
   actttcccag agaaaggaaa ctatgagcct ggagctactc ctggtgatct tatttttgtg 600
   atagatgaaa agccgcatgc tgtcttcaag agggatggaa atgatctgga gatcaatcag 660
   aaaatttctt tactagatgc tcttactggg aaaactataa gcttgatcac tttggatgga 720
   cgggaactca caataccaat cacagatatt gttaaaccag gacatgagca tataatccca 780
   aatgaaggaa tgccaatatc aaaggaacgt ggcaagaaag gaaatttgaa gatcaagttt 840
   gacattaaat tcccatcaag gctaagtgca gatcagaaat ctgatatcag gagggtactg 900
   tgcaggagcg ctgactaa                                                918
<210> 3
   <211> 918
   <212> DNA
   <213> Nicotiana tabacum
<220>
   <221> gene
   <222> (1) .. (918)
   <223> NtCPIP2b
<400> 3
   atgggacttg attactatga cgtactgaaa gtatctcgga atgcaagtga agaagattta 60
   aagagatcgt ataagcgatt agcgatgaaa tggcatccag ataagaacag tcagaacaaa 120
   aaggaagctg aagcgaaatt caagcagatt tctgaagcgt atgatgtgct tagtgatcct 180
   cagaagcgtc agatctatga cgtgtacggt gatgatgcat tgaaatccgg tcaatttgct 240
   tcggcgtcgc cgactagtgc tggtagtaac ggcagaggat ttaggttcaa tacgcgtgac 300
   gcggaggcta ttttcgctga gtttttcggt ggatcggata gtaattccgc tgccggagta 360
   ggtcggaagg cggcaccggt ggagaataaa ctgccgtgta gcttggagga gctttacaaa 420
   ggatctagaa ggaaaatgaa gatctcacgg attcttcttg atgactctgg taagcctaca 480
   actgttgaag aggtcctagc gatacacatc aaaccaggtt ggaagaaagg cacaaaaatc 540
   actttcccag agaaagggaa ctatgaacct ggagctactc ctggtgatct tatttttgtg 600
   atagatgaaa agccacatgc tgtcttcaag agggatggaa atgatctagt gatcaatcag 660
   aaaatatctt tactagatgc tcttactggg aaaactataa gcttgatcac tttggatgga 720
   cgggaactca caataccaat cacagatgtt gttaaaccag gacatgagca tataatccca 780
   aatgaaggaa tgccaatatc aaaggaacgt ggcaagaaag gaaatttgaa gatcaagttt 840
   gacattaaat tcccatctag gctaagtgca gatcagaaat ctgatatcag gagggtactg 900
   tgcaggaact ctgactaa                                                918
<210> 4
   <211> 306
   <212> PRT
   <213> Nicotiana tabacum
<220>
   <221> PEPTIDE
   <222> (1) .. (306)
   <223> NtCPIP1
<400> 4
   Met Gly Val Asp Tyr Tyr Lys Ile Leu Lys Val Ser Arg Asn Ala Ser
   1 5 10 15
   Glu Glu Asp Leu Lys Lys Ser Tyr Lys Arg Leu Ala Met Lys Trp His
                   20                  25                  30
   Pro Asp Lys Asn Ser Glu Lys Glu Ala Glu Ala Lys Phe Lys Gln Ile
               35                  40                  45
   Ser Glu Ala Tyr Asp Val Leu Ser Asp Pro Gln Lys Arg Gln Ile Tyr
           50                  55                  60
   Asp Ile Tyr Gly Asp Glu Ala Leu Lys Ser Gly Gln Phe Asp Pro Ser
   65                  70                  75                  80
   Ser Pro Met Asn Gly Asn Gly Arg Gly Phe Lys Phe Asp Ser Arg Asp
                   85                  90                  95
   Ala Glu Asp Ile Phe Ala Glu Phe Phe Gly Gly Ser Asp Gly Tyr Ser
               100                 105                 110
   Arg Ser Pro Thr Gly Gly Thr Val Arg Ile Arg Lys Pro Ala Pro Val
           115                 120                 125
   Glu Asn Lys Leu Pro Cys Ser Leu Glu Glu Leu Tyr Lys Gly Ser Lys
       130                 135                 140
   Arg Lys Met Lys Ile Ser Arg Ile Val Leu Asp Val Thr Gly Lys Pro
   145                 150                 155                 160
   Thr Thr Ile Glu Glu Val Leu Ala Ile His Ile Lys Pro Gly Trp Lys
                   165                 170                 175
   Lys Gly Thr Lys Ile Thr Phe Pro Glu Lys Gly Asn His Glu Pro Gly
               180                 185                 190
   Ala Ala Pro Gly Asp Leu Ile Phe Val Ile Asp Glu Lys Pro His Asp
           195                 200                 205
   Val Phe Lys Arg Asp Gly Asn Asp Leu Val Ile Asn Gln Lys Ile Ser
       210                 215                 220
   Leu Val Asp Ala Leu Ser Gly Lys Ile Ile Asn Leu Ala Thr Leu Asp
   225                 230                 235                 240
   Gly Arg Glu Leu Thr Ile Pro Ile Thr Asp Val Val Lys Pro Gly His
                   245                 250                 255
   Glu Gln Ile Ile Ala Asp Glu Gly Met Pro Ile Ser Lys Glu Pro Gly
               260                 265                 270
   Lys Lys Gly Asn Leu Arg Ile Lys Phe Glu Val Lys Phe Pro Ser Arg
           275                 280                 285
   Leu Ser Ser Asp Gln Lys Leu Asp Ile Arg Arg Val Leu Gly Arg Thr
       290                 295                 300
   Val Asp
   305
<210> 5
   <211> 305
   <212> PRT
   <213> Nicotiana tabacum
<220>
   <221> PEPTIDE
   <222> (1)..(305)
   <223> NtCPIP2a
<400> 5
   Met Gly Leu Asp Tyr Tyr Asn Val Leu Lys Val Ser Arg Asn Ala Ser
    1               5                  10                  15
   Glu Glu Asp Leu Lys Arg Ser Tyr Lys Arg Leu Ala Met Lys Trp His
                20                  25                  30
   Pro Asp Lys Asn Ser Gln Asn Lys Lys Glu Ala Glu Ala Lys Phe Lys
            35                  40                  45
   Gln Ile Ser Glu Ala Tyr Asp Val Leu Ser Asp Pro Gln Lys Arg Gln
        50                  55                  60
   Ile Tyr Asp Val Tyr Gly Asp Asp Ala Leu Lys Ser Gly Gln Phe Ala
   65                  70                  75                  80
   Ser Ala Ser Pro Thr Ser Ala Gly Ser Asn Ala Arg Gly Phe Arg Phe
                   85                  90                  95
   Asn Thr Arg Asp Ala Glu Ala Ile Phe Ala Glu Phe Phe Gly Gly Ser
               100                 105                 110
   Gly Ser Asn Ser Gly Ala Gly Val Gly Arg Lys Ala Ala Pro Val Glu
           115                 120                 125
   Asn Lys Leu Pro Cys Ser Leu Glu Glu Leu Tyr Lys Gly Ser Arg Arg
       130                 135                 140
   Lys Met Lys Ile Ser Arg Ile Leu Leu Asp Asp Ser Gly Lys Pro Thr
   145                 150                 155                 160
   Thr Val Glu Glu Val Leu Ala Ile His Ile Lys Pro Gly Trp Lys Lys
                   165                 170                 175
   Gly Thr Lys Ile Thr Phe Pro Glu Lys Gly Asn Tyr Glu Pro Gly Ala
               180                 185                 190
   Thr Pro Gly Asp Leu Ile Phe Val Ile Asp Glu Lys Pro His Ala Val
           195                 200                 205
   Phe Lys Arg Asp Gly Asn Asp Leu Glu Ile Asn Gln Lys Ile Ser Leu
       210                 215                 220
   Leu Asp Ala Leu Thr Gly Lys Thr Ile Ser Leu Ile Thr Leu Asp Gly
   225                 230                 235                 240
   Arg Glu Leu Thr Ile Pro Ile Thr Asp Ile Val Lys Pro Gly His Glu
                   245                 250                 255
   His Ile Ile Pro Asn Glu Gly Met Pro Ile Ser Lys Glu Arg Gly Lys
               260                 265                 270
   Lys Gly Asn Leu Lys Ile Lys Phe Asp Ile Lys Phe Pro Ser Arg Leu
           275                 280                 285
   Ser Ala Asp Gln Lys Ser Asp Ile Arg Arg Val Leu Cys Arg Ser Ala
       290                 295                 300
   Asp
   305
<210> 6
   <211> 305
   <212> PRT
   <213> Nicotiana tabacum
<220>
   <221> PEPTIDE
   <222> (1) .. (305)
   <223> NtCPIP2b
<400> 6
   Met Gly Leu Asp Tyr Tyr Asp Val Leu Lys Val Ser Arg Asn Ala Ser
   1 5 10 15
   Glu Glu Asp Leu Lys Arg Ser Tyr Lys Arg Leu Ala Met Lys Trp His
   20 25 30
   Pro Asp Lys Asn Ser Gln Asn Lys Lys Glu Ala Glu Ala Lys Phe Lys
   35 40 45
   Gln Ile Ser Glu Ala Tyr Asp Val Leu Ser Asp Pro Gln Lys Arg Gln
   50 55 60
   Ile Tyr Asp Val Tyr Gly Asp Asp Ala Leu Lys Ser Gly Gln Phe Ala
   65 70 75 80
   Ser Ala Ser Pro Thr Ser Ala Gly Ser Asn Gly Arg Gly Phe Arg Phe
   85 90 95
   Asn Thr Arg Asp Ala Glu Ala Ile Phe Ala Glu Phe Phe Gly Gly Ser
   100 105 110
   Asp Ser Asn Ser Ala Ala Gly Val Gly Arg Lys Ala Ala Pro Val Glu
   115 120 125
   Asn Lys Leu Pro Cys Ser Leu Glu Glu Leu Tyr Lys Gly Ser Arg Arg
   130 135 140
   Lys Met Lys Ile Ser Arg Ile Leu Leu Asp Asp Ser Gly Lys Pro Thr
   145 150 155 160
   Thr Val Glu Glu Val Leu Ala Ile His Ile Lys Pro Gly Trp Lys Lys
   165 170 175
   Gly Thr Lys Ile Thr Phe Pro Glu Lys Gly Asn Tyr Glu Pro Gly Ala
   180 185 190
   Thr Pro Gly Asp Leu Ile Phe Val Ile Asp Glu Lys Pro His Ala Val
   195 200 205
   Phe Lys Arg Asp Gly Asn Asp Leu Val Ile Asn Gln Lys Ile Ser Leu
   210 215 220
   Leu Asp Ala Leu Thr Gly Lys Thr Ile Ser Leu Ile Thr Leu Asp Gly
   225 230 235 240
   Arg Glu Leu Thr Ile Pro Ile Thr Asp Val Val Lys Pro Gly His Glu
   245 250 255
   His Ile Ile Pro Asn Glu Gly Met Pro Ile Ser Lys Glu Arg Gly Lys
               260                 265                 270
   Lys Gly Asn Leu Lys Ile Lys Phe Asp Ile Lys Phe Pro Ser Arg Leu
           275                 280                 285
   Ser Ala Asp Gln Lys Ser Asp Ile Arg Arg Val Leu Cys Arg Asn Ser
       290                 295                 300
   Asp
   305
<210> 7
   <211> 66
   <212> PRT
   <213> Nicotiana tabacum
<220>
   <221> DOMAIN
   <222> (1) .. (66)
   <223> consensus-Sequenz der DnaJ-ähnlichen Domäne
<400> 7
   Asp Tyr Tyr Glu Ile Leu Gly Val Pro Arg Asp Ala Ser Asp Glu Glu
   1               5                   10                  15
   Ile Lys Lys Ala Tyr Arg Lys Leu Ala Leu Lys Tyr His Pro Asp Lys
               20                  25                  30
   Asn Pro Gly Asp Pro Glu Ala Glu Glu Lys Phe Lys Glu Ile Asn Glu
           35                  40                  45
   Ala Tyr Glu Val Leu Ser Asp Pro Glu Lys Arg Ala Ile Tyr Asp Gln
       50                  55                  60
   Tyr Gly
   65
<210> 8
   <211> 801
   <212> DNA
   <213> CP cDNA aus potato virus Y (PVY)
<400> 8
   gcaaatgaca caattgatgc aggaggaagc tctaagaaag atgcaaacca agagcaaagt 60
   agcattcaac caaatcccaa caagagaaag gaaaaggacg tgaatgttgg aacatctgga 120
   actcacactg tgccacgaat taaagctatc acgtccaaaa tgagaatgcc caagagtaag 180
   ggtgcaactg tactaaattt agaacattta ctcgagtatg ctccacagca aattgacatc 240
   tcaaatactc gagcaactca atcacagttt gatacatggt atgaagcagt acaacttgca 300
   tacgacatag gagaaactga aatgccaact gtgatgaatg ggcttatggt ttggtgcatt 360
   gaaaatggaa cctcgccaaa tatcaatgga gtttgggtta tgatggatgg agatgaacaa 420
   atcgaatacc cactaaaacc aatcgttgag aatgcaaaac caacacttag gcaaatcatg 480
   gcacatttct cagatgttgc agaagcgtac atagaaatgc gcaacaaaaa ggaaccatac 540
   atgccacgat atggtttagt tcgtaatctg cgcgatggaa gtttggctcg ctatgctttt 600
   gacttttatg aagtcacatc acgaacacca gtgagggcta gagaggcaca cattcaaatg 660
   aaggccgcag ctttaaaatc agctcaatct cgacttttcg gattggatgg tggcatcagt 720
   acacaagagg aaaacacaga gaggcacacc accgaggatg tttctccaag tatgcatact 780
   ctacttggag tcaagaacat g                                            801
<210> 9
   <211> 267
   <212> PRT
   <213> CP aus potato virus Y (PVY)
<400> 9
   Ala Asn Asp Thr Ile Asp Ala Gly Gly Ser Ser Lys Lys Asp Ala Asn
   1               5                   10                  15
   Gln Glu Gln Ser Ser Ile Gln Pro Asn Pro Asn Lys Arg Lys Glu Lys
               20                  25                  30
   Asp Val Asn Val Gly Thr Ser Gly Thr His Thr Val Pro Arg Ile Lys
           35                  40                  45
   Ala Ile Thr Ser Lys Met Arg Met Pro Lys Ser Lys Gly Ala Thr Val
       50                  55                  60
   Leu Asn Leu Glu His Leu Leu Glu Tyr Ala Pro Gln Gln Ile Asp Ile
   65                  70                  75                  80
   Ser Asn Thr Arg Ala Thr Gln Ser Gln Phe Asp Thr Trp Tyr Glu Ala
                   85                  90                  95
   Val Gln Leu Ala Tyr Asp Ile Gly Glu Thr Glu Met Pro Thr Val Met
               100                 105                 110
   Asn Gly Leu Met Val Trp Cys Ile Glu Asn Gly Thr Ser Pro Asn Ile
           115                 120                 125
   Asn Gly Val Trp Val Met Met Asp Gly Asp Glu Gln Ile Glu Tyr Pro
       130                 135                 140
   Leu Lys Pro Ile Val Glu Asn Ala Lys Pro Thr Leu Arg Gln Ile Met
   145                 150                 155                 160
   Ala His Phe Ser Asp Val Ala Glu Ala Tyr Ile Glu Met Arg Asn Lys
                   165                 170                 175
   Lys Glu Pro Tyr Met Pro Arg Tyr Gly Leu Val Arg Asn Leu Arg Asp
               180                 185                 190
   Gly Ser Leu Ala Arg Tyr Ala Phe Asp Phe Tyr Glu Val Thr Ser Arg
           195                 200                 205
   Thr Pro Val Arg Ala Arg Glu Ala His Ile Gln Met Lys Ala Ala Ala
       210                 215                 220
   Leu Lys Ser Ala Gln Ser Arg Leu Phe Gly Leu Asp Gly Gly Ile Ser
   225                 230                 235                 240
   Thr Gln Glu Glu Asn Thr Glu Arg His Thr Thr Glu Asp Val Ser Pro
                   245                 250                 255
   Ser Met His Thr Leu Leu Gly Val Lys Asn Met
               260                 265
<210> 10
   <211> 918
   <212> DNA
   <213> Nicotiana tabacum
<400> 10
   atgggacttg attactataa cgtactaaaa gtatctcgga atgcaagtga agaagattta 60
   aagagatcgt acaagcgatt agctatgaaa tggcatccag ataagaacag tcagaacaaa 120
   aaagaagcag aagcaaaatt caagcagatt tctgaagcgt atgatgtgct tagtgatcct 180
   cagaagcgtc agatctatga cgtgtacggt gatgatgcat tgaaatccgg tcaatttgcc 240
   tcggcgtcgc cgactagtgc tggtagtaac gccagagggt ttaggttcaa tacgcgtgat 300
   gcggaggcta tttttgctga gtttttcggt ggatcgggta gtaattccgg tgccggagtc 360
   ggtcggaagg cggcgccggt ggagaataaa ctgccgtgta gcttggagga gctttacaaa 420
   ggttctagaa gaaaaatgaa gatctcacgg attcttctgg atgactctgg taagcctaca 480
   actgttgaag aggtcttagc gatacacatc aagcctggtt ggaagaaagg cacaaaaatc 540
   actttcccag agaaaggaaa ctatgagcct ggagctactc ctggtgatct tatttttgtg 600
   atagatgaaa agccgcatgc tgtcttcaag agggatggaa atgatctgga gatcaatcag 660
   aaaatttctt tactagatgc tcttactggg aaaactataa gcttgatcac tttggatgga 720
   cgggaactca caataccaat cacagatatt gttaaaccag gacatgagca tataatccca 780
   aatgaaggaa tgccaatatc aaaggaacgt ggcaagaaag gaaatttgaa gatcaagttt 840
   gacattaaat tcccatcaag gctaagtgca gatcagaaat ctgatatcag gagggtactg 900
   tgcaggagcg ctgactaa                                              918